# EUROPEAN PATENT SPECIFICATION

(11) **EP 1 979 341 B1**
(45) Date of publication and mention of the grant of the patent: **04.04.2012**
(21) Application number: 07707998.6
(22) Date of filing: 30.01.2007
(51) Int. Cl.: C07D 401/12, A61K 31/505, A61P 3/06

(54) **TRISUBSTITUTED AMINE COMPOUND**
TRISUBSTITUIERTE AMINVERBINDUNG
COMPOSÉ AMINE TRISUBSTITUÉ

(30) Priority: 31.01.2006 JP 2006023571; 30.11.2006 JP 2006322854
(43) Date of publication of application: 15.10.2008
(73) Proprietor: Mitsubishi Tanabe Pharma Corporation, Osaka 541-8505 (JP)
(72) Inventor: NAKAMURA, Yoshinori, Osaka-shi Osaka 541-8505 (JP); HAYASHI, Norimitsu, Osaka-shi Osaka 541-8505 (JP); HIGASHIJIMA, Takanori, Osaka-shi Osaka 541-8505 (JP); KUBOTA, Hitoshi, Osaka-shi Osaka 541-8505 (JP); OKA, Kozo, Osaka-shi Osaka 541-8505 (JP)
(74) Representative: HOFFMANN EITLE
(86) International application number: PCT/JP2007/051868
(87) International publication number: WO 2007/088999

(56) References cited:
- WO-A-01/36395
- WO-A-2005/030185
- WO-A-2005/103022
- WO-A-2006/056854
- WO-A-2006/073973
- US-A1- 2005 059 810

## Description

### TECHNICAL FIELD

The present invention relates to a compound having an inhibitory activity against cholesteryl ester transfer protein (CETP) and showing an activity of increasing HDL cholesterol level and an activity of decreasing LDL cholesterol level, thereby being useful for prophylaxis and/or treatment of arteriosclerotic diseases, hyperlipernia or dyslipidemia.

### BACKGROUND ART

Hypercholesterolemia, especially high serum level of low-density lipoprotein (LDL) cholesterol, has been revealed to be a risk factor of arteriosclerotic diseases by a number of epidemiological surveys. Actually, drugs capable of decreasing LDL cholesterol level such as 3-hydroxy-3-methylglutaryl-coenzyme A (HMG-CoA) reductase inhibitors have been used with the aim of preventing coronary artery diseases, and demonstrated to have some benefits in many large scale clinical tests. However, their preventive effect on coronary diseases is limited to some extent, and is not satisfactory enough yet.

Recently, low serum level of high density lipoprotein (HDL) cholesterol has been revealed to be a potent risk factor of arteriosclerotic diseases by a number of epidemiological surveys and large scale clinical tests. HDL is known to have various antiarteriosclerotic effects and attention is focused on the potentiality of drugs increasing HDL cholesterol level as a means for prevention or treatment of arteriosclerotic diseases. However, there are no drugs that can be used in a satisfactory manner for this purpose. Fibrates and HMG-CoA reductase inhibitors have only low activity of increasing HDL cholesterol level; nicotinic acid derivatives can significantly increase HDL cholesterol level, but have serious toleration issues. Accordingly, there has been a demand for a well-tolerated agent which can significantly elevate HDL cholesterol level, thereby preventing or reversing the progression of atherosclerosis.

It is known that many proteins are involved in the regulation mechanism for catabolism of various lipoproteins. Among them, the role of cholesteryl ester transfer protein (CETP) became to draw attention. CETP is a protein responsible for transfer of cholesteryl ester (CE) and triglyceride between lipoproteins, and mediate the transfer of CE from HDL to LDL or to very low density lipoprotein (VLDL). Accordingly, CETP activity affects greatly the lipid composition in lipoprotein particles. For example, it is known that administration of an active neutralizing monoclonal antibody against CETP to rabbit or hamster elevates HDL cholesterol level and lower LDL cholesterol level. Furthermore, human being having decreased or eliminated CETP activity due to gene mutation shows raised blood HDL cholesterol level and lowered blood LDL cholesterol level. On the other hand, it is known that transgenic mice and rats made to express CETP show lowered HDL cholesterol level and raised LDL cholesterol level. Thus, it is considered that CETP greatly contribute to the regulation of serum lipids, and thereby affecting the change of serum lipid profile such as decrease of HDL cholesterol level and increase of LDL cholesterol leveL Accordingly, it is assumed that a high value of CETP activity would induce arteriosclerosis.

In fact, CETP activity varies depending on animal species. It is known that, arteriosclerotic lesions are readily formed by cholesterol loading in animals with high CETP activity such as rabbits, whereas such lesions hardly occur in animals with low CETP activity such as rats. Furthermore, it is confirmed that continuous suppression of CETP activity by administration of antisense oligodeoxynuclotide resulted in effects such as increase of blood HDL cholesterol level and reduction in arteriosclerotic lesions in cholesterol-fed rabbits.

The above findings indicate that CETP activity is in negative correlation with HDL cholesterol, and that inhibition of CETP activity would decrease the degree of risk for arteriosclerotic diseases. It is therefore expected that compounds capable of inhabiting CETP activity can block the transfer of cholesterol from LDL to LDL or VLDL, and thereby increasing HDL cholesterol that tends to prevent arteriosclerosis while lowering LDL cholesterol that tends to promote arteriosclerosis. In this way, such compounds can serve as a useful preventive or therapeutic agent for arteriosclerotic disease, hyperlipemia or dyslipidemia and provide effective medical treatment for the first time.

Examples of compounds having CETP inhibitory activity include tetrahydroquinoline derivatives. See, PCT International Publication WO00/17164, pamphlet, PCT International Publication WO00/17165 pamphlet and PCT International Publication WO00/17166 pamphlet.

However, these compounds have defects. That is, they are poorly soluble in water and cannot be absorbed enough in vivo, a sufficient blood level for taking medicinal effect can hardly be achieved even when administered as an ordinary formulation for oral administration. See, WO03/63868.

Accordingly, it has been demanded to find a novel compound which eliminates the above-mentioned defects and intensive studies have been done on dibenzylamine type compounds, and the like. See, PCT International Publication WO05/100298 pamphlet, PCT International Publication WO04/020393 pamphlet, PCT International Publication WO06/073973 pamphlet and JP 2003-221376 A.

Further compounds having CETP inhibitory activity are disclosed in US 2005/059810 A1 and WO 2005/030185 A and WO 2006/056854.

### DISCLOSURE OF INVENTION

The present invention provides compounds having an excellent inhibitory activity against CETP, thereby useful for prophylaxis and/or treatment of arteriosclerotic diseases, hyperlipemia or dyslipidemia.

The present inventors have intensively studied in order to achieve the above-mentioned objects, and have found a compound having an inhibitory activity against CETP and showing an activity of increasing HDL cholesterol level and an activity of decreasing LDL cholesterol level, and have accomplished the present invention.

### BEST MODES FOR CARRYING OUT THE INVENTION

That is, the present invention provides the following embodiments:
1. A compound of the general formula (1): 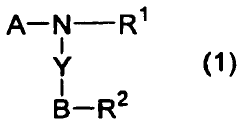 wherein Y is a methylene group;
   A is a group of a formula: -A¹-A²,
   A¹ is a pyrimidinyl group;
   A² is an alkoxy group optionally substituted by carboxyl group, a piperidyl group optionally substituted by carboxyl group, an amino group optionally substituted by 1 to 2 groups selected independently from carboxyalkyl group and akyl group; or an alkyl group substituted by carboxyl group;
   B is a pyrimidinyl, pyridyl, quinolyl, dihydroindolyl, pyrazolyl, isoquinolyl, imidazolyl or imidazopyridyl group; wherein said heterocyclic group may optionally be substituted by 1 to 5 substituents selected independently from the following groups: an oxo group; an alkoxy group; an alkyl group optionally substituted by 1 to 3 halogen atoms; a halogen atom; an alkylsulfanyl group; an alkylsulfinyl group; and an amino group optionally substituted by 1 to 2 substituents selected independently from an alkyl group or an alkyl group substituted by morpholinyl;
   R¹ is 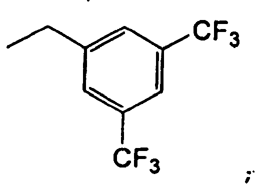 R² is
   (a) an amino group optionally substituted by 1 to 2 groups selected independently from a carboxyalkyl group, an alkoxyalkyl group, a hydroxyalkyl group, mono- or dialkylaminoalkyl group, an alkyl group, a cycloalkylalkyl group and an alkoxycarbonyl group;
   (b) an alkoxy group;
   (c) a phenyl group optionally substituted by 1 to 2 substituents selected independently from an alkyl group and an alkoxy group;
   (d) a pyrimidinyl group optionally substituted by 1 to 2 substituents selected from an alkoxy group and an alkyl group;
   (e) a pyridyl group optionally substituted by 1 to 2 substituents selected from an alkoxy group and an alkyl group; or a pharmaceutically acceptable derivative thereof.
2. A compound selected from the following group: 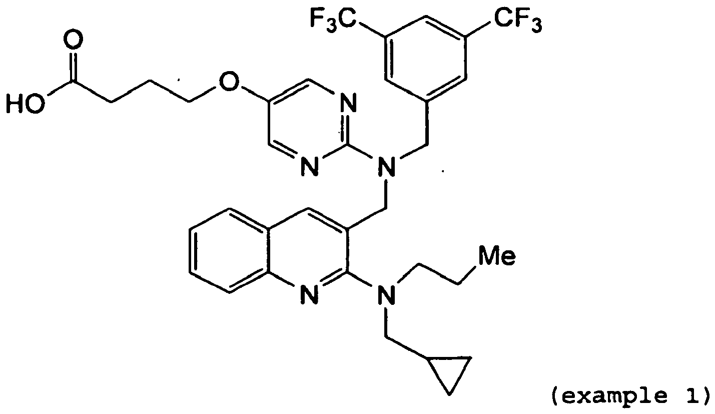 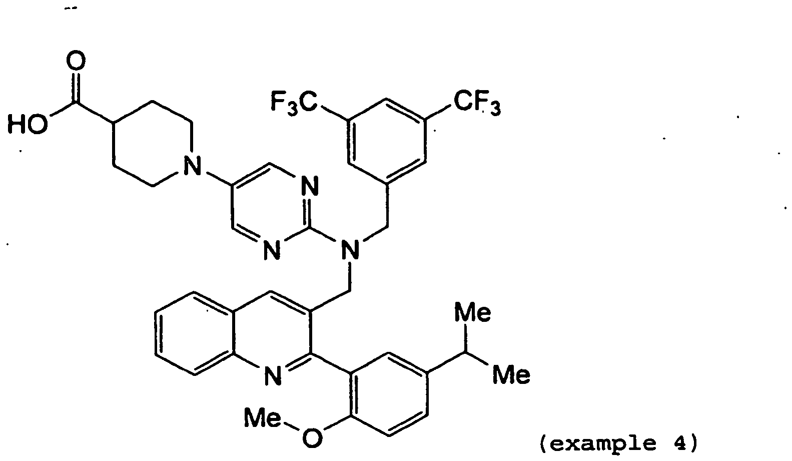 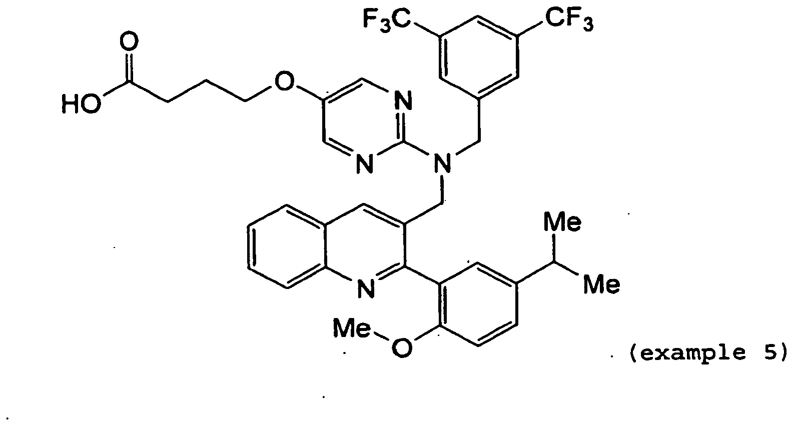 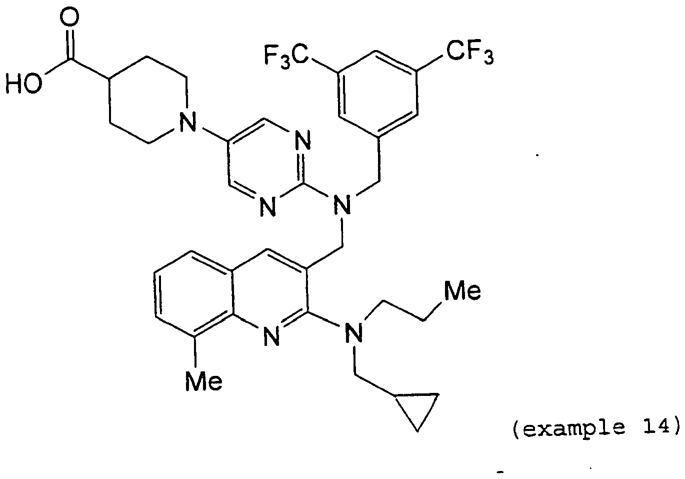 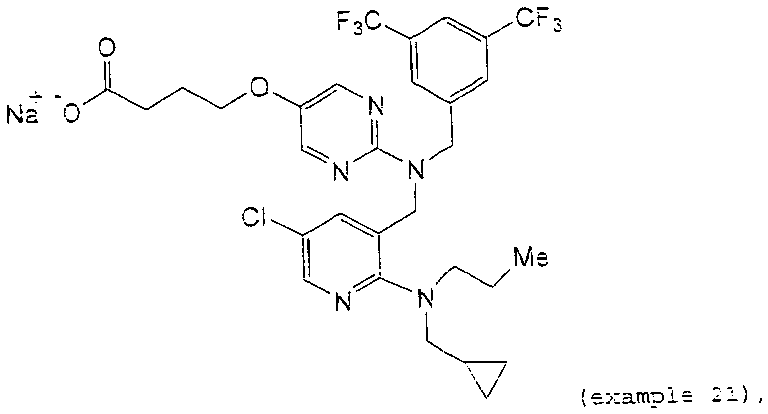 and 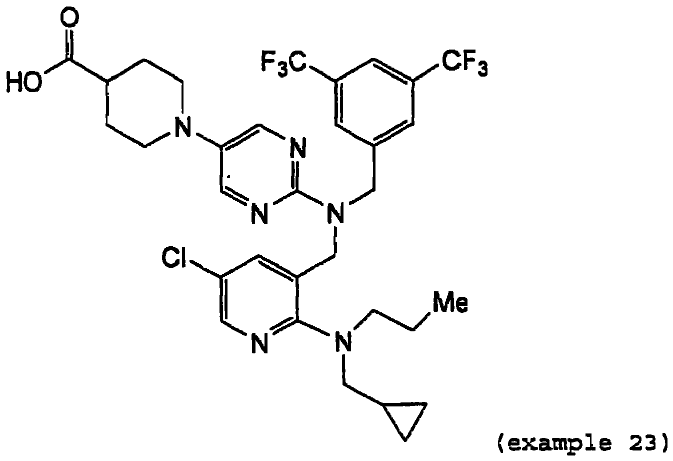 or a pharmaceutically acceptable derivative thereof.
3. A pharmaceutical composition, which comprises as an active ingredient a compound according to the above embodiments 1 or 2 or a pharmaceutically acceptable derivative thereof.
4. A compound according to the above embodiments 1 or 2, or a pharmaceutically acceptable derivative thereof for use in the prophylaxis or treatment of arteriosclerosis such as atherosclerosis, peripheral vascular disease, dyslipidemia, hyperbetalipoproteinemia, hypoalphalipoproteinemia, hypercholesterclemia, hypertriglyceridemia, famillial-hypercholesterolemia, cardiovascular diseases, angina, ischemia, cardiac ischemia, stroke, myocardial infarction, reperfusion, injury, restenosis after angioplasty, hypertension, cerebral infarction, cerebral stroke, diabetes, vascular complication of diabetes, thrombotic diseases, obesity, endotoxemia, metabolic syndrome, cerebrovascular disease, coronary artery disease, ventricular dysfunction, cardiac arrhythmia, pulmonary vascular disease, reno-vascular disease, renal disease, splanchnic vascular disease, vascular hemostatic disease, fatty liver disease, steatohepatitis, inflammatory disease, autoimmune disorders and other systemic disease indications, immune function modulation, pulmonary disease, anti-oxidant disease, sexual dysfunction, cognitive dysfunction, schistosomiasis, cancer, regression of xanthoma or Alzheimer's disease, which comprises administering an effective amount of a compound according to the above embodiments 1 or 2, or a pharmaceutically acceptable derivate thereof, to a patient in need thereof.
5. Use of a compound according to the above embodiments 1 or 2, or a pharmaceutically acceptable derivative thereof, for the manufacture of a medicament in treatment of patients suffering from arteriosclerosis such as atherosclerosis, peripheral vascular disease, dyslipidemia, hyperbetalipoproteinemia, hypoalphalipoproteinemia, hypercholesterolemia, hypertrigylceridemia, familial-hypercholesterolemia, cardiovascular diseases, angina, ischemia, cardiac ischemia, stroke, myocardial infarction, reperfusion injury, restenosis after angioplasty, hypertension, cerebral infarction, cerebral stroke, diabetes, vascular complication of diabetes, thrombotic diseases, obesity, endotoxemia, metabolic syndrome, cerebrovascular disease coronary artery disease, ventricular dysfunction, cardiac arrhythmia, pulmonary vascular disease, reno-vascular disease, renal disease, splanchnic vascular disease, vascular hemostatic disease, fatty liver disease, steatohepatitis, inflammatory disease, autoimmune disorders and other systemic disease indications, immune function modulation, pulmonary disease, anti-oxidant disease, sexual dysfunction, cognitive dysfunction, schistosomiasis, cancer, regression of xanthoma or Alzheimer's disease.

When the compound (1) has one or more asymmetric carbon atoms, the compound (1) of the present invention encompasses racemate, racemic mixture, individual enantiomers or diastereomers. The present compounds also include all such isomers and a mixture thereof.

Also, when the present compound has an alkenyl or alkynyl group, cis (Z)- and trans (E)- forms may occur. In addition, the present compounds include individual stereoisomer of the compound and optionally, individual tautomeric forms thereof and a mixture thereof.

Separation of diastereomer or cis- and trans- isomers can be achieved by the conventional method such as fractional crystallization, chromatography and HPLC method, and the like. Also the drug containing the individual stereoisomer as needed can be prepared from a corresponding optically active intermediate or alternatively by resolving the corresponding racemate by using the suitable chiral support (e.g. HPLC) or by performing a fractional crystallization of diastereomeric salt formed by reacting a corresponding racemate and a suitable optically active acid or base. Alternatively, the resolution of the mixture of enantiomers can be done by forming on a novel covalently bounded species formed by means of reacting it with a suitable chiral compound. For example, at first, the coupling reaction between a racemic carboxylic acid and a chiral amine or a chiral alcolol gives a mixture of diastereoisomers (amide or ester, respectively) and then it is isolated by the conventional technique such as chromatography, HPLC or fractional crystallization, and the like. Thereafter, the resulting one diastereoisomer can be converted into one enantiomer of the desired compound by cleaving the new covalent binding with a suitable chemical reaction such as hydrolysis, and the like.

As used herein, the term "pharmaceutically acceptable derivative" represents a pharmaceutical acceptable salt, solvate or prodrug (e.g. ester) of the present compound, and which can provide (directly or indirectly) the compound of the present invention or an active metabolite or a residue thereof. Such derivatives can be obtained by a person skilled in the art without undue experimentation. See, for example, Burger's Medicinal Chemistry and Drug Discovery 5th ed., vol. 1st, "Principles and Practice". Preferred pharmaceutically acceptable derivative is a salt, a solvate, an ester, a carbaminic ester and a phosphoric ester. Especially preferred pharmaceutically acceptable derivative is a salt, a solvate and an ester. Most preferred pharmaceutically acceptable derivative is a salt and an ester.

A person skilled in the art of organic chemisty knows that many organic compounds can be formed a complex with a solvent of reaction system and which can be precipitated out or crystallized out from the solvent. These complexes are widely known as a "solvate". For example, the complex with water is known as a "hydrate". The solvate of the compound of the present invention falls within the scope of the invention.

As used herein, the "prodrug" represent a compound which convert to the active form having a pharmacological activity by a hydrolysis in vivo (such as in a blood). The example of the pharamaceutically acceptable prodrug is described in the literature: T. Higuchi and V. Stera, Prodrugs as Novel Delivery Systems, "Bioreversible Carriers in Drug Design", Edward B. Roche ed., American Pharmaceutical Association and Pergamon Press, A.C.S. Symposium Series, vol. 14th, (1987); and D. Freisher, S. Roman and H. Barbara, Improved oral drug delivery: solubility limitations overcome by the use of prodrugs, Advanced Drug Delivery Reviews (1996) 19(2): 115-130).

A prodrug is a carrier that releases the compound of the formula (1) which is bound covalently in vivo when administered to a patient. In general, the prodrug is prepared by the conventional method or by modifying a functional group such that the moidified moiety is cleaved in vivo to give a parent compound. Examples of the prodrug include the compound wherein a hydroxy, amine or sulfhydryl group binds to an optional group such that provide a hydroxy amine or sulfhydryl group by cleaving it when administered to a patient. Thus the representative examples of prodrug are the following ones, but not limited thereto; i. e. the derivatives with acetic ester, formic ester and benzoic ester at the functional groups such as alcohol, sulfhydryl or amine of the compound of the formula (1). In addition, when the functional group is a carboxylic acid, esters such as methyl ester, ethyl ester and double ester, and the like can be used. The esters exhibit inherently the activity in a human body and/or are hydrolyzed under in vivo condition to the active sompound. The suitable pharmaceutically acceptable esters capable of hydrolyzing in vivo include those which are decomposed easily in the human body to release the parent acid compound or a salt thereof.

The compound of the present invention may be a pharmacetically acceptable salt form thereof. The suitable salt is outlined in literature (Berge et. al., J. Pharm. Sci., 66: 1-19 (1977)).

In general, the pharmaceutically acceptable salts can be easily prepared with a desirable acid or base as needed. The resulting salts can be recovered by filtering after precipitating it out from the solution or distilling the solvent off.

The suitable additive salts may be formed with an acid forming a nontoxic salt. Examples of the salt include hydrochloride, hydrobromide, hydroiodide, sulfate, bisulfate, nitrate, phosphate, hydrogen phosphate, acetate, maleate, malate, fumarate, lactate, tartrate, citrate, formate, gluconate, succinate, pyruvate, oxalate, oxaloacetate, trifluoroacetate, saccarate, benzoate, methanesulfonate, ethanesulfonate, benzenesulfonate and p-toluenesulfonate.

Examples of the pharmaceutically acceptable salt with a base include alkaline metal salts including ammonium salt, sodium salt and potassium salt; alkaline earth metal salts including calcium salt and magnesium salt as well as salts with organic bases including a primary, secondary and tertiary amine (e.g. isopropylamine, diethylamine, ethanolamine, trimethlamine, dicyclohexylamine and N-methyl-D-glucamine).

The term "halogen" refers to fluorine, chlorine, bromine or iodine.

The term "alkyl group" or "alkyl" means a straight or branched saturated hydrocarbon chain having 1 to 10 carbon atoms and a cyclic saturated hydrocarbon chain having 3 to 10 carbon atoms. As a straight or branched hydrocarbon chain, those having 2 to 10 carbon atoms are preferred and those having 2 to 6 carbons are more preferred. More preferred examples are straight chain alkyl groups having 1 to 6 carbon atoms, especially those having 1 to 4 carbon atoms. Examples of alkyl group include methyl, ethyl, propyl, isopropyl, butyl, sec-butyl, tert-butyl, pentyl, isopentyl, neopentyl, tert-pentyl, 1-methylbutyl, 2-methylbutyl, 3-methylbutyl, hexyl and isohexyl groups, and the like.

The term "alkoxy group" or "alkoxy" means a straight or branched alkyloxy group having 1 to 10 carbon atoms and a cyclic alkyloxy group having 3 to 10 carbon atoms. As a straight or branched hydrocarbon chain, those having 2 to 10 carbon atoms are preferred and those having 2 to 6 carbons are more preferred. More preferred examples are straight chain alkoxy groups having 1 to 6 carbon atoms, especially those having 1 to 4 carbon atoms. Examples of alkoxy group include methoxy, ethoxy, propoxy, isopropoxy, butoxy, isobutoxy, tert-butoxy, pentoxy, isopentoxy, neopentoxy, tert-pentoxy, hexoxy and isohexoxy groups, and the like.

The term "alkylene group" or "alkylene" means a saturated hydrocarbon chain wherein a hydrogen atom is removed from each of the terminal carbons of a straight hydrocarbon chain. Preferred examples include an alkylene group having 1 to 6 carbon atoms, specifically, methylene, ethylene, trimethylene and tetramethylene groups, and the like. When an alkylene group herein used contains 1 to 3 heteroatoms selected independently from nitrogen, sulfur and oxygen atoms, the term "alkylene" includes a group of the formula:-O-(CH₂)ₘ-O-, -S-(CH₂)ₘ-S-, -NH-(CH₂)ₘ-NH-, or -O-(CH₂)ₘ-NH- (wherein m is an integer of 1 to 4), or the like.

The term "alkanoyl group" or "alkanoyl" means a straight or branched alkylcarbonyl group having 1 to 10 carbon atoms, preferably an alkylcarbonyl group having 1 to 6 carbon atoms, more preferably an alkylcarbonyl group having 1 to 4 carbon atoms. Examples of alkanoyl group include acetyl, propionyl, butyryl, valeryl and pivaloyl groups, and the like.

The term "alkenyl group" or "alkenyl" means a straight or branched hydrocarbon chain having 2 to 10 carbon atoms and containing at least one double bond, preferably an alkenyl group having 2 to 6 carbon atoms, more preferably an alkenyl group having 2 to 4 carbon atoms Examples of alkenyl group include vinyl, 1-propenyl, allyl, isopropenyl, butenyl, butadienyl and pentenyl groups, and the like.

As herein used throughout the claims and specification, when the term "mono- or di-alkyl" refers to di-alkyl, the alkyl moieties may be the same or independent from each other.

The cycloalkyl or cycloalkyl group as used herein is C3-10 cyclic hydroncarbon group and includes for example, cyclopropyl, cyclobutyl, cyclopentyl, cycohecyl, cycloheptyl, cyclooctyl, cyclononyl groups, and the like and preferably C3-6 cyclic hydrocarbon group.

The cycloalkoxy and cycloalkoxy group as used herein is oxy group substituted by C3-10 cyclic hydrocarbon and includes, for example cyclopropyloxy; cyclobutoxy, cyclopentoxy, cyclohexoxy, and the like and preferably oxy group substituted by C3-6 cyclic hydrocarbon group.

The heterocycle or heterocycic group as used herein includes 5 to 8 membered heterocyclic group including 1 to 4 heteroatoms selected from oxygen, sulfur and nitrogen atoms and dicyclic or tricyclic fused heterocyclic group fused thereto. Specific examples of the heterocycic group include, for example, a thienyl, furyl, pyrrolyl, pyrrolinyl, oxazolyl, thiazolyl, pyrazolyl, imidazolyl, imidazolinyl, isoxazolyl, isothiazolyl, oxadiazolyl, furazanyl, thiadiazolyl, triazolyl, triazinyl, triazolidinyl, tetrazolyl, pyridyl, imidazopyridyl, pyrimidinyl, thiomorpholinyl, morpholinyl, triazinyl, pyrrolidinyl, piperidyl, pyranyl, tetrahydropyranyl, thiopyranyl, oxadinyl, thiadinyl, piperazinyl, triazinyl, oxotriazinyl, pyridazinyl, pyrazinyl, benzofuryl, benzothiazolyl, benzoxazolyl, tetrazolopyridazinyl, triazolopyridazinyl, benzimidazolyl, quinolyl, isoquinolyl, dihydroisoquinolyl, cinnolinyl, phthalaziny, quinazolinyl, quinoxalinyl, indolizinyl, dihydroindolyl, indolyl, quinolizinyl, naphthyridinyl, purinyl, pteridinyl, dibenzofuranyl, benzofuranyl, carbazolyl, acridinyl, phenanthridinyl, chromanyl, benzoxazinyl, phenazinyl, phenothiazinyl, phenoxazinyl, hexahydroazepinyl, imidazolidinyl, oxazolidinyl, tetrahydrofuranyl, dioxolanyl, oxiranyl, dihydropyrimidinyl, oxazolinyl, dihydrooxazinyl, dihydropyrazolyl, imidazopyridyl, dihydropyrazinyl, tetrahydroquinolyl, benzothienyl, dihydrooxazolyl, oxathiadiazolyl, dihydrooxazolyl or tetrahydroquinolyl group, and the like.

The homocycle or homocyclic group as used herein include, for example, 3 to 7 membered carbocyclic group optionally fused, such as C6-10 aryl group (e.g. phenyl and napthyl group, and the like), C3-10 cycloalkyl group (e.g. cyclopropyl, cyclobutyl, cyclopentyl, cyclohexyl, cyclohepthyl, and the like), C3-10 cycloalkenyl group (e.g., cyclopropenyl, cyclobutenyl, cyclopentenyl, cycohexenyl, cycloheptenyl, and the like).

### EFFECT OF THE INVENTION

The compound (1) of the present invention has an inhibitory activity against CETP and shows the effects on increasing HDL cholesterol level and decreasing LDL cholesterol Thus the compound is useful in a prophylaxis and a treatment of diseases such as arteriosclerosis and hyperlipidemia, and the like.

The present compound (1) can be administered either orally or parenterally, and can be formulated into a suitable pharmaceutical preparations with a conventional pharmaceutically acceptable carriers used therefor.

The pharmaceutically acceptable salts of the compound (1) include, for example, alkali metal salts such as lithium, sodium or potassium salt; alkali earth metal salts such as calcium or magnesium salt; salts with zinc or aluminum; salts with organic bases such as ammonium, choline, diethanolamine, lysine, ethylenediamine, tert-butylamine, tert-octylamine, tris(hydroxymethyl)aminomethane, N-methylglucosamie, triethanolamine or dehydroabiethylamine; salts with inorganic acids such as hydrochloric acid, hydrobromic acid, hydroiodic acid, sulfuric acid, nitric acid or phosphoric acid; salts with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, benzenesulfonic acid or toluenesulfonic acid; or salts derived from acidic amino acids such as aspartic acid or glutamic acid.

Additionally, the pharmaceutically acceptable salts of the compound of the formula (1) include, for example, quaternary salts formed between a compound of the formula (1) and an alkyl halide or a phenylalkyl halide.

Preferred pharmaceutical preparations for oral administration of the present compound include solid formulations such as tablets, granules, capsules or powders; and liquid formulations such as solutions, suspensions or emulsions. Preferred pharmaceutical preparations for parenteral administration include injections or infusions formulated with injectable distilled-water, physiological saline or aqueous glucose solution; suppository; or inhalation preparation, and the like.

These pharmaceutical preparations comprise a compound (1) of the present invention or a pharmaceutically acceptable salt thereof and a pharmaceutically acceptable carrier which is usually used for oral or parenteral administration. The pharmaceutically acceptable carriers for oral administration include, for example, a binder (syrup, gum acacia, gelatin, sorbit, tragacanth, polyvinylpyrrolidone, and the like), an excipient (lactose, sugar, cornstarch, potassium phosphate, sorbit, glycine, and the like), a lubricant (magnesium stearate, talc, polyethylene glycol, silica, and the like), a disintegrant (potato starch, and the like), and a wetting agent (anhydrous sodium lauryl sulfate, and the like).

Also the pharmaceutically acceptable carriers for parenteral administration include, for example, injectable distilled-water, physiological saline and aqueous glucose solution.

The dose of a compound (1) of the present invention or a pharmaceutically acceptable salt thereof varies depending on the administration route, age, body weight, disease, and condition/severity, of the patient It however can usually be in the range of about 0.001 - 1,000 mg/kg/day, preferably in the range of about 0.01 - 100 mg/kg/day, more preferably in the range of about 0.1 - 10 mg/kg/day.

The compounds (1) of the present invention have an inhibitory activity against CETP and show an activity of increasing HDL cholesterol level and an activity of lowering LDL cholesterol level. Accordingly, they are useful in the prophylaxis or treatment of a subject (particularly, mammal including human) suffering from arteriosclerosis such as atherosclerosis, peripheral vascular disease, dyslipidemia, hyperbetalipoproteinemia, hypoalphalipoproteinemia, hypercholesterolemia, hypertriglyceridemia, familial-hypercholesterolemia, cardiovascular diseases, angina, ischemia, cardiac ischemia, stroke, myocardial infarction, reperfusion injury, restenosis after angioplasty, hypertension, cerebral infarction, cerebral stroke, diabetes, vascular complication of diabetes, thrombotic diseases, obesity, endotoxemia, metabolic syndrome, cerebrovascular disease, coronary artery disease, ventricular dysfunction, cardiac arrhythmia, pulmonary vascular disease, reno-vascular disease, renal disease, splanchnic vascular disease, vascular hemostatic disease, fatty liver disease, steatohepatitis, inflammatory disease, autoimmune disorders and other systemic disease indications, immune function modulation, pulmonary disease, anti-oxidant disease, sexual dysfunction, cognitive dysfunction, schistosomiasis, cancer, regression of xanthoma, Alzheimer's disease, or the like.

In addition, the compounds of the present invention may be used in combination with other drugs useful for treatment of these diseases. For example, a compound of the present invention may be used in combination with an inhibitor of cholesterol synthesis such as HMG-CoA reductase inhibitor; an inhibitor of cholesterol absorption such as anion exchange resin; a triglyceride lowering agent such as fibrates, niacin and fish oil; an antihypertensive such as ACE inhibitor, angiotensin receptor blocker, calcium antagonist and beta blocker; an antiobesity agent such as central anorectic, lipase inhibitor and CB1 antagonist; an antidiabetic agent such as insulin sensitizer, D2 agonist, sulfonylurea, biguanide, α-glucosidase inhibitor, SGLT inhibitor and DPPIV inhibitor; or other cholesterol reducer such as ACAT inhibitor.

The compound (1) of the present invention may be prepared by the following methods, which should not be construed to be limited thereto.

In each process for preparing a compound of the formula (1) described above, when protection of a functional group contained in any compound is needed, the protection can be carried out in a conventional manner ad libitum. General statement related to protecting groups and their use is provided by Greene, Protective Groups in Organic Synthesis, John Wiley and Sons, New York, 1991.

Further in each process, the reaction can be carried out by the conventional method and a procedure for isolation and the purification may be selected from the conventional method such as crystallization, recrystallization and chromatography and preparative HPLC, and the like as appropriate, or may be combined with one another.

### [Method 1]

The compound (1) can be prepared by the following method I. 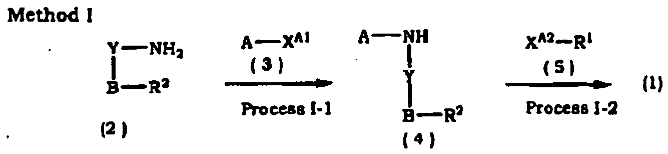 wherein the X^{A1} and X^{A2} are a leaving group and the other symbols have the same meanings as defined above

### (Process 1-1)

The compound (4) can be prepared by reacting the compound (2) with the compound (3) in a solvent in the presence of a base.

Any solvent which dose not disturb the reaction can be preferably used as a solvent used, and include, for example, ethers including diethylether, tetrahydrofuran (THF), dioxane, 1,2-methoxyethane, diglyme; hydrocarbons including benzene, toluene, hexane, xylene; alcohols including methanol, ethanol, isopropyl alcohol, tert-butanol; esters including ethyl acetate, methyl acetate, butyl acetate; polar solvents including acetone, N,N-dimethylformamide, dimethyl sulfoxide, which can be used alone or in a combination thereof. Prefered solvents in this reaction include ethanol, dioxane, toluene and N,N-dimethylformamide.

A conventional base may be used as a base, and includes for example, alkaline metal hydride including sodium hydride and potassium hydride; alkaline metal alkoxide including sodium ethoxide, sodium methoxide, sodium tert-butoxide and potassium tert-butoxide; alkyl lithium including n-butyl lithium and sec-butyl lithium; alkaline metal amide including lithium diisopropylamide, sodium amide and lithium bis(trimethylsilyl)amide; alkaline metal carbonate including sodium carbonate, potassium carbonate, sodium bicarbonate and potassium bicarbonate; alkaline metal hydroxide including lithium hydroxide, sodium hydroxide and potassium hydroxide; alkaline metal phosphate including sodium and phosphate potassium phosphate; organic base including triethylamine, diisopropylethylamine, pyridine and N- methylmorpholine; preferably triethylamine, sodium bicarbonate, sodium tert-butoxide, diisopropylethylamine, sodium hydride and potassium tert-butoxide.

The leaving group includes a halogen atom including chlorine atom, bromine atom, iodine atom, and a substituted sulfonyloxy group including methanesulfonyloxy group, p-toluenesulfonyloxy group and trifluoromethanesulfonyloxy group.

### (Process I-2)

The compound (1) can be prepared by reacting the compound (4) with the compound (5) in a same manner as in process I-1 or I'-1 described below.

### (Process I'-1)

The process (I-1) may be replaced by the following process I'-1.

In the above process I-1, the compound (4) can also be prepared by reacting the compound (2) with the compound (3) in the presence or absence of a base or in the presence of a metal catalyst such as palladium catalyst in a suitable solvent.

A conventional palladium catalyst can be used as a palladium catalyst, and include palladium acetate, tetrakis(triphenylphosphine)-palladium, tris(dibenzylideneacetone)dipalladium, dichlorobis(triphenyl-phosphine)palladium, dichlorobis(tri-o-tolylphosphine)palladium, bis-(triphenylphosphine)palladium acetate, or the like.

As the base, alkaline metal hydroxide including sodium hydroxide, potassium hydroxide; alkaline earth metal hydroxide including barium hydroxide; alkaline metal alkoxide including sodium methoxide, sodium ethoxide, potassium ethoxide, sodium tert-butoxide, potassium tert-butoxide; alkaline metal carbonate including sodium carbonate, potassium carbonate, cesium carbonate; alkaline metal bicarbonate including sodium bicarbonate, potassium bicarbonate; alkaline metal phosphate including potassium phosphate; amines including triethylamine, diisopropylethylamine, methylpiperidine, dicyclohexylmethylamine; and pyridines including pyridine, 4-dimethylaminopyridine can be preferably used.

Additionally, phosphines may be added in the present reaction. As the phosphines, triphenylphosphine, tributylphosphine, tri-tert-butylphosphonium tetrafluoroborate, 1,3-bis(diphenylphosphino)propane, 2,2'-bis(diphenylphosphino)-1,1'-binaphthyl, 1,1'-bis(diphenylphosphino)-ferrocene, 2-(di-tert-butylphosphino)biphenyl, 2-dicyclohexylphosphino-2'-(N,N-dimethylamino)biphenyl, 2-(dicyclohexylphosphino)biphenyl, and the like can be preferably used.

Any solvent which dose not disturb the reaction can be preferably used as a solvent used in the reaction, and include, for example, ethers including diethylether, tetrahydrofuran (THF), dioxane, 1,2-methoxyethane, diglyme; hydrocarbons including benzene, toluene, hexane, xylene; alcohols including methanol, ethanol, isopropyl alcohol, tert-butanol; esters including ethyl acetate, methyl acetate, butyl acetate; polar solvents including acetone, N,N-dimethylformamide, dimethyl sulfoxide, and which can be used alone or in a combination thereof.

### (Process I'-2)

The compound (1) can be prepared by reacting the compound (4) with the compound (5) in a same manner as in process I'-1 or I-1.

### [Method II]

The compound can be prepared by a method II. 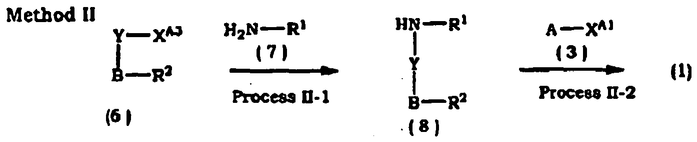 wherein the X^{A3} is a leaving group and the other symbols have the same meanings as defined above

### (Process II-1)

The compound (8) can be prepared by reacting the compound (6) with the compound (7) in a same manner as process I-1.

### (Process II-2)

The compound (1) can be prepared by reacting the compound (8) with the compound (3) in a same manner as in process I-1 or I'-1.

### [Method III]

The compound (1) can be prepared by a method III. 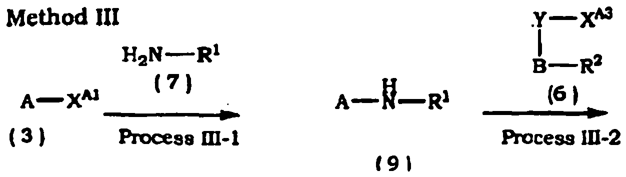 wherein the symbols have the same meanings as defined above

### (Process III-1)

The compound (9) can be prepared by reacting the compound (3) with the compound (7) in a same manner as in process I-1 or process I'-1.

### (Process III-2)

The compound (1) can be prepared by reacting the compound (9) with the compound (6) in a same manner as in process I-1.

### [Method IV]

The compound of the general formula (1-a): 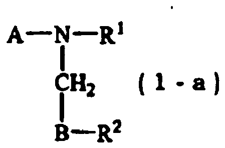 wherein the symbols have the same meanings as defined above , that is, those wherein Y is a methylene group in the compound of the formula (1), can be prepared by a following method IV.

The compound (1-a) can be prepared by reducing the compound of a general formula (6'): 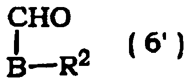 wherein the symbols have the same meanings as defined above, or its corresponding carboxylic acid or its corresponding carboxylic acid ester to provide the compound of a general formula (6"): 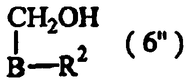 wherein the symbols have the same meanings as defined above, followed by halogenating the resulting compound and then reacting the resulting compound with the above compound (9) in a solvent in the presence of a base.

The reduction can be carried out by treating a starting compound with a reducing agent in a suitable solvent. Boron hydrides (sodium borohydride, and the like) and aluminum hydrides (lithium aluminum hydride, diisobutylaluminum hydride, and the like) can be preferably used as a reducing agent.

The hologenation can be carried out by treating a starting compound with a halogenating agent in a suitable solvent. As the halogenating agent, a conventional halogenating agent including thionyl chloride, phosphorus oxychloride, as well as carbon tetrahalide (e.g., carbon betrachloride, carbon tetrabromide, and the like) and phosphines (e.g., triphenylphosphine, tritolylphosphine, triethylphosphine, and the like) can be preferably used.

A conventional base may be used as a base and include for example, alkaline metal hydride including sodium hydride and potassium hydride; alkaline metal alkoxide including sodium ethoxide, sodium methoxide, sodium tert-butoxide and potassium tert-butoxide; alkyl lithium including n-butyl lithium and sec-butyl lithium; alkaline metal amide including lithium diisopropylamide, sodium amide and lithium bis(trimethylsilyl)amide; alkaline metal carbonate including sodium carbonate, potassium carbonate, sodium bicarbonate and potassium bicarbonate; alkaline metal hydroxide including lithium hydroxide, sodium hydroxide and potassium hydroxide; alkaline metal phosphate including sodium and phosphate potassium phosphate; organic base including triethylamine, diisopropylethylamine, pyridine and N- methylmorpholine; preferably triethylamine, sodium bicarbonate, sodium tert-butoxide, diisopropylethylamine, sodium hydride and potassium tert-butoxide.

Any solvent which dose not disturb the reaction can be preferably used as a solvent used, and include, for example, ethers including diethylether, tetrahydrofuran (THF), dioxane, 1,2-methoxyethane, diglyme; hydrocarbons including benzene, toluene, hexane, xylene; alcohols including methanol, ethanol, isopropyl alcohol, tert-butanol; esters including ethyl acetate, methyl acetate, butyl acetate; polar solvents including acetone, N,N-dimethylformamide, dimethyl sulfoxide, which can be used alone or in a combination thereof. Prefered solvents in this reaction include ethanol, dioxane, toluene and N,N-dimethylformamide.

### [Method IV']

The compound (1-a) can also be prepared by the following method IV'.

The compound (1-a) can also be prepared by halogenating the above compound (6") according to the above method IV, followd by reacting the resulting compound with the above compound (7) in a solvent in the presence of a base to provide the compound of a general formula (8') 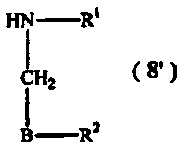 wherein the symbols have the same meanings as defined above, then reacting the resulting compound with the compound (3) acccording to the above process II-2.

### [Method IV"]

In the above method IV', the compound (8') can be prepared by reacting the compound (6') with the compound (7) in a solvent in the presence of a reducing agent.

Any solvent which dose not disturb the reaction can be preferably used as a solvent used in the reactions, and include for example, halogens including 1,2-dichloroethane, dichloromethane, chloroform, ethers including diethylether, tetrahydrofuran (THF), dioxane, 1,2-methoxyethane, diglyme; hydrocarbons including benzene, toluene, hexane, xylene; alcohols including methanol, ethanol, isopropyl alcohol, tert-butanol; esters including ethyl acetate, methyl acetate, butyl acetate; polar solvents including acetone, N,N-dimethylformamide, dimethyl sulfoxide, and which can be used alone or in a combination thereof. Especially preferred solvent in this reaction includes 1,2-dichloroethane, dichloromethane and toluene. The reducing reagent includes sodium borohydrides including sodium triacetoxyborohydride, sodium cyanoborohydride; and aluminum hydrides including lithium aluminium hydride and diisobutylaluminium hydride.

### [Method V]

The compound of a general formula (1-b): 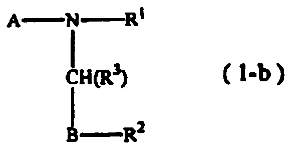 wherein R³ is an alkyl group and the other symbols have the same meanings as defined above, those wherein Y is an alkyl group in the compound (1), can be prepared by undergoing a conventional Grignard reaction of the compound (6') with a reagent of a general formula: R³MgBr wherein the symbol has the same meaning as defined above, to provide the compound of a general formula (6'"): 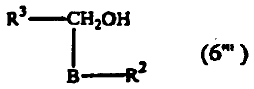 wherein the symbols have the same meanings as defined above, followed by halogenating the resulting compound in a same manner as in the above method IV, then reacting the resulting compound with the above compound (9) in a solvent in the presence of a base.

### [Method V']

The compound (1-b) can also be prepared by the following method V'.

The compound (1-b) can also be prepared by haogenating the above compound (6"') according to the above method V, followed by reacting the resulting compound with the above compound (7) in a solvent in the presence of a base, to provide a compound of a general formula (8"]: 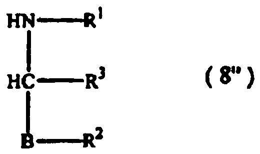 wherein the symbols have the same meanings as defined above, then reacting the resulting compound with the compound (3) according to the above process II-2.

### [Method VI]

The compound of a general formula (1-c): 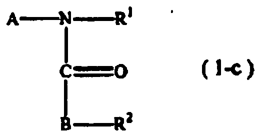 wherein the symbols have the same meanings as defined above, those wherein Y is an methylene group substituted by an oxo group in the compound (1), can be prepared by oxidating the compound (6') to provide the compound of a general formula (6""): 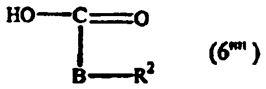 wherein the symbols have the same meanings as defined above, followed by halogenating the resulting compound according to the above method IV, then reacting the resulting compound with the above compound (9) in a solvent in the presence of a base.

### [Method VI]

The compound (1-c) can also be prepared by the following method VI'.

The compound (1-c) can be prepared by halogenating the above compound (6"") according to the above method VI, followed by reacting the resulting compound with the above compound (7) in a solvent in the presence of a base, to provide a compound of a general formula (8"'): 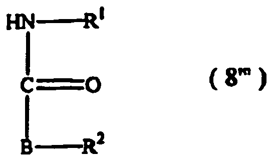 wherein the symbols have the same meanings as defined above, then reacting the resulting compound with the compound (3) according to the above process 11-2.

### (Method VII]

The compound (1-a) can be prepared by the following method VII.

The compound (1-a) can also be prepared by reducing the compound of a general formula (6""): 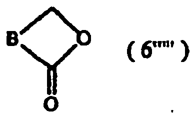 wherein the symbols have the same meanings as defined above, to provide the compound of a general formula (6"""): 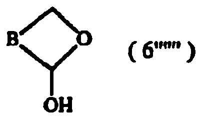 wherein the symbols have the same meanings as defined above, then reacting the resulting compound with the above compound (9) in a same manner as in the method It, to provide the compound of a general formula (I-d): 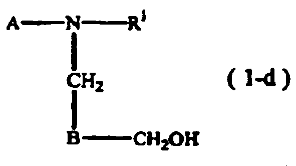 wherein the symbols have the same meanings as defined above, and interconverting on R² moiety.

### [Method VII]

The compound (1-a) can also be prepared by reacing the above compound (6"") with the above compound (7) in a same manner as in the above method IV" to provide the compound of a general formula (8"): 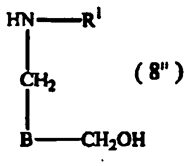 wherein the symbols have the same meanings as defined above, followed by reacting the resulting compound with the compound (3) according to the above process II-2 to provide the compound (1-d), then interconverting on R² moiety.

In addition, substituents on the A, B, R¹ and R² may be further interconverted according to the known method after or before a synthesis of the compound (1).

A² can be interconverted by the following methods (EA) to (EL).

In the following each process, unless otherwise specified, a conventional base can be used as the base, and for example, alkaline metal hydride including sodium hydride, potassium hydride; alkaline metal hydroxide including sodium hydroxide, potassium hydroxide; alkaline earth metal hydroxide including barium hydroxide; alkaline metal alkoxide including sodium methoxide, sodium ethoxide, potassium ethoxide, potassium tert-butoxide; alkaline metal carbonate including sodium carbonate, potassium carbonate, cesium carbonate; alkaline metal bicarbonate including sodium bicarbonate, potassium bicarbonate; amines including triethylamine, diisopropylethylamine, methylpiperidine, dimethylaniline, 1,8-diazabicyclo[5.4.0]undecene, 1,4-diazabicyclo[2.2.2]octane, 1,5-diazabicyclo[4.3.0]nonene; pyridines including pyridine, dimethylaminopyridine can be preferably used.

Also in the following each process, a conventional acid can be used as the acid, and for example, unless otherwise specified, an inoroganic acid (e.g. hydrochloric acid, nitric acid and sulfuric acid; origanic acid represented by sulfonic acids (e.g. methane, sulfonic acid, p-toluene sulfonic acid and trifluoromethane sulfonic acid; and the like) can be preferably used.

Also in the following each process, any solvent which dose not disturb the reaction can be used as the solvent and specifically include hydrocarbons including pentane, hexane; aromatic hydrocarbons including benzene, toluene, nitrobenzene; halogenated hydrocarbons including dichloromethane, chloroform; ethers including diethylether, tetrahydrofuran amides including dimethylformamide, N-methylpyrrolidone, 1,3-dimethylimidazolidin-2-one; sulfoxides including dimethylsulfoxide; alcohols including methanol, ethanol; esters including ethyl acetate, butyl acetate; ketones including acetone, methyl ethyl ketone; nitriles including acetonitrile; water, or a mixed solvent thereof.

Also in the following each process, the leaving group includes a halogen atom including chlorine atom, bromine atom, iodine atom, and a substituted sulfonyloxy group including methanesulfonyloxy group, trifluoromethanesulfonyloxy group and toluenesulfonyloxy group.

(EA) The compound wherein A¹ is a tetrazolyl group and A² is an alkyl group or a substituted alkyl group can be prepared by alkylating a compound wherein A¹ is a tetrazolyl group and A² is a hydrogen atom.

The alkylation can be carried out by reacting with a compound of the formula:

A^{2A}-Z²

wherein A^{2A} is an alkyl group or a substituted alkyl group and Z² is a leaving group, in a suitable solvent in the presence or absence of a base, or by reacting with a compound of the formula:

A^{2A}-OH

wherein the symbol has the same meaning as defined above, in a suitable solvent in the presence of phosphines and azodicarboxylic esters.

N-alkylation of a compound wherein A¹ is a nitrogen-containing heterocyclic group can be carried out in a similar manner as above.

The reaction proceeds more preferably when a catalytic amount of an alkaline metal iodide (e.g., potassium iodide, and the like) is added.

Both phosphines and azodicarboxylic esters which usually employed in Mitsunobu reaction can be preferably used. Phosphines include, for example, triphenylphosphine, tributylphosphine, and the like, and azodicarboxylic esters include diethyl azodicarboxylate, diisopropyl azodiformate, and the like.

(EB) The compound wherein A¹ is 2-oxodihydropyrimidinyl group and A² is an alkyl group or a substituted alkyl group can be prepared by alkylating a compound of the formula (1) wherein A¹ is 2-hydroxypyrimidinyl group and A² is a hydrogen atom, with a compound of the formula:

A^{2A}-Z²

wherein the symbols have the same meaning as defined above.

The reaction can be carried out in the same manner as in the above (EA).

(EC) The compound wherein A² is an optionally substituted amino group or a group of the formula: 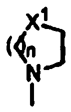 wherein the symbols have the same meaning as defined above, can be prepared by reacting a compound of the formula (1) wherein A² is a halogen atom, with a corresponding amine or a compound of the formula: 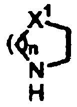 wherein the symbols have the same meanings as defined above.

The reaction can be carried out in the presence or absence of a base, and in the presence or absence of a palladium catalyst in a suitable solvent.

As the palladium catalyst, a conventional palladium catalyst including palladium acetate, tetrakis(triphenylphosphine)palladium, tris(dibenzylideneacetone)dipalladium, dichlorobis(triphenylphosphine)-palladium, dichlorobis(tri-o-tolylphosphine)palladium, bis-(triphenylphosphine)palladium acetate, or the like can be used.

As the base, alkaline metal hydroxide including sodium hydroxide, potassium hydroxide; alkaline earth metal hydroxide including barium hydroxide; alkaline metal alkoxide including sodium methoxide, sodium ethoxide, potassium ethoxide, potassium tert-butoxide; alkaline metal carbonate including sodium carbonate, potassium carbonate, cesium carbonate; alkaline metal bicarbonate including sodium bicarbonate, potassium bicarbonate; alkaline metal phosphate including potassium phosphate; amines including triethylamine, diisopropylethylamine, methylpiperidine, dicyclohexylmethylamine; and pyridines including pyridine, 4-dimethylaminopyridine can be preferably used.

Additionally, phosphines may be added in the present reaction. As the phosphines, triphenylphosphine, tributylphosphine, tri-tert-butylphosphonium tetrafluoroborate, 1,3-bis(diphenylphosphino)propane, 2,2'-bis(diphenylphosphino)-1,1'-binaphthyl, 1,1'-bis(diphenylphosphino)-ferrocene, 2-(di-tert-butylphosphino)biphenyl, 2-dicyclohexylphosphino-2'-(N,N-dimethylamino)biphenyl, 2-(dicyclohexylphosphino)biphenyl, and the like can be preferably used as the phosphines.

(ED) The compound wherein A² is an optionally substituted amino group can be prepared by coupling a compound of the formula (1) wherein A² is a halogen atom with a compound of the formula:

(R²⁰)₃Sn-NR²¹R²²

wherein R²⁰ is an alkyl group and NR²¹R²² is an optionally substituted amino group.

The coupling reaction can be carried out in the presence of a palladium catalyst in the presence or absence of a base in a suitable solvent.

The palladium catalysts, bases, and phosphines described in the above (EC) can be used in the same manner as in the above (EC).

(EE) The compound wherein A² is a cyano group can be prepared by cyanating a compound of the formula (1) wherein A² is a halogen atom.

The cyanation can be carried out by reacting a starting compound with a metal cyanide including sodium cyanide, potassium cyanide, or zinc cyanide in the presence of a palladium catalyst in a suitable solvent.

The same palladium catalyst as that described in the above (EC) can be preferably used.

(EF) The compound wherein A² is an optionally substituted alkoxycarbonyl group can be prepared by reacting a compound of the formula (1) wherein A² is a halogen atom, with a corresponding alkylalcohol under carbon monoxide atmosphere using a palladium catalyst in the presence of a base in a suitable solvent.

The same palladium catalyst and base as those described in the above (EC) can be preferably used.

Additionally, the reaction can be more preferably carried out by adding a ligand, and phosphines described in the above (EC) can be preferably used as the ligand.

(EG) The compound wherein A² is an optionally substituted alkenyl group can be prepared by coupling a compound of the formula (1) wherein A² is a halogen atom with a corresponding alkene.

The coupling reaction can be carried out in the presence of a palladium catalyst in the presence or absence of a base in a suitable solvent.

The same palladium catalyst as that described in the above (EC) can be preferably used.

The same base as that described in the above (EC) can be preferably used and silver carbonate can also be used.

Additionally, the reaction can be more preferably carried out by adding a ligand, and phosphines described in the above (EC) can be preferably used as the ligand.

(EG') The compound wherein A² is an optionally substituted alkenyl group can be prepared by dehydration reaction of a compound having a hydroxyalkyl group in a substituent A². The dehydration reaction can be carried out by treating a starting compound with acid.

As the acid, a conventional acid can be used. For example, an inoroganic acid (e.g. hydrochloric acid, nitric acid and sulfuric acid) or an origanic acid (e.g. methane sulfonic acid, p-toluene sulfonic acid, acetic acid, trifluoroacetic acid, trifluoromethane sulfonic acid, and the like) can be preferably used.

(EH) The compound wherein A² is a boronic acid ester can be prepared by reacting with the compound wherein A² is a leaving group with a trialkoxyborane (trianethoxyborane, triisopropoxyborane, and the like), a dialkoxyborane (pinacolborane, and the like) or a tetraalkoxydiboron (bis(pinacolato)diboron, and the like) in the presence of palladium catalyst. The leaving group includes a halogen atom including chlorine atom, bromine atom, iodine atom, and a substituted sulfonyloxy group including methanesulfonyloxy group, trifluoromethanesulfonyloxy group, and toluenesulfonyloxy group. The reaction can be carried out in the same manner as in the above (EC).

(EH') The compound wherein A2 is a hydroxy group can be prepared by reacting the compound wherein A2 is a boronic acid ester with the peroxide. Aqueous hydrogen peroxide, m-chloroperbenzoic acid and OXONE™ (DuPont Co. Ltd), and the like can be perferably used as the peroxide.

(EI) The compound wherein A² is an alkoxy group or a substituted alkoxy group can be prepared by alkoxylating the compound (1) wherein A² is a halogen atom.

The alkoxylation can be carried out by optionally adding a copper catalyst to react with a corresponding alcohol in a suitable solvent or solvent-free in the presence of a base.

The same base as described in the above (EC), in particular, cesium carbonate can be preferably used.

The copper catalyst including copper iodide, copper bromide, copper chloride, copper acetate, copper trifluoromethanesulfonate, and the like can be preferably used.

Additionally, this reaction proceeds more preferably when 1,10-phenanthroline, 2-aminopyridine, or the like is added.

(EJ) The compound wherein A² is an optionally substituted alkyl group, an optionally substituted heterocyclic group or an optionally substituted aryl group can be prepared by coupling a compound of the formula (1) wherein A² is a leaving group with a corresponding alkyl, aryl or heterocyclic boronic acid or a corresponding alkyl, aryl or heterocyclic boronic acid ester.

The coupling can be carried out in the presence of a palladium catalyst, and in the presence or absence of a base in a suitable solvent.

This reaction can be carried out in the same manner as in the above (EC).

The leaving group is the same as defined above (EH).

(EJ) The compound wherein A² is an optionally substituted alkyl group, an optionally substituted heterocyclic group or an optionally substituted aryl group can be prepared by coupling a compound of the formula (1) wherein A² is a boronic acid or a boronic acid ester with an alkyl, an aryl or a heterocyclic group which have a leaving group.

The coupling can be carried out in the presence of a palladium catalyst, and in the presence or absence of a base in a suitable solvent. This reaction can be carried out in the same manner as in the above (EC). The leaving group is the same as defined above (EH).

(EK) The compound wherein A² is an alkoxycarbonylalkylsulfonyl group can be prepared by reacting a compound of the formula (1) wherein A² is a halogen atom with an alkoxycarbonylalkylsulfinic acid alkaline metal salt.

The alkoxycarbonylalkylsulfunic acid alkaline metal salt can be prepared according to the method described, for example, in Baskin et. al., Tetrahedron Lett., 43, 8479 (2002).

Additionally, this reaction can be carried out in the presence of a copper catalyst in a suitable solvent according to the method described in the said literature.

The same copper catalyst as described in (EI) above can be used, and in particular, copper iodide can be preferably used.

(EL) The compound wherein A² is a group of the formula: 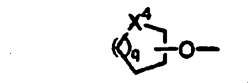 wherein the symbols have the same meaning as defined above, can be prepared by condensing a compound wherein A² is a hydroxy group with a compound of the formula: 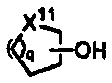 wherein X¹¹ is O SO, SO₂ or NR^{p} (R^{p} is a protecteing group) and q is an integer from 1 to 4, and as needed, removing the protecting group for amino group.

As a protecting group, a conventional protecting group including benzyloxycarbonyl group, tert-butoxycarbonyl group, and the like can be used.

The reaction can be carried out in a suitable solvent in the presence of phosphines and azodicarboxylic esters. The reaction can be carried out in the same manner as in the above (EA).

The removal of a protecting group can be carried out in a conventional manner including catalytic reduction, acid-treatment, and the like, depending on the type of a protecting group.

The same manner as in the above reactions (EA) to (EL) for conversions of A² can also be applied for conversion of the other substituent as needed.

Additionally, a substituent(s) of a compound (1) of the present invention can be converted into different one(s) within the scope of the compound (1) according to the following methods as appropriate.

In the following each process, a conventional base can be used as the base, and unless otherwise specified, the base described in the above (EA) can be preferably used.

Additionally, in the following each process, a conventional acid can be used as the acid, and unless otherwise specified, a mineral acid including hydrochloric acid, nitric acid, sulfuric acid, or an organic acid represented by sulfonic acids (e.g., methanesulfonic acid, p-toluenesulfonic acid, trifluoromethanesulfonic acid) or carboxylic acids (e.g., acetic acid, trifluoroacetic acid) can be preferably used.

Further additionally, in the following each process, any solvent which dose not disturb the reaction can be used, and as such, the solvent described in the above (EA) can be preferably used.

The leaving group includes a halogen atom including chlorine atom, bromine atom, iodine atom, and a substituted sulfonyloxy group including methanesulfonyloxy group, trifluoromethanesulfonyloxy group, and toluenesulfonyloxy group.

(E1) The compound wherein A is a heterocyclic group substituted by an optionally substituted amino group or a group of the formula: 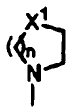 wherein the symbols have the same meanings as defined above, can be prepared by reacting a compound (1) wherein A is a heterocyclic group substituted by an optionally substituted alkylsulfonyloxy group, with a corresponding amine or a compound of the formula: 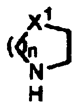 wherein the symbols have the same meaning as defined above in the presence of a palladium catalyst, and in the presence or absence of a base in a suitable solvent or solvent-free.

The reaction can be carried out in the same manner as in the above (EC).

(E2) The compound wherein A is a heterocyclic group substituted by an optionally substituted amino group or a group of the formula: 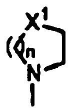 wherein the symbols have the same meanings as defined above, can also be prepared by reacting a compound wherein A is a heterocyclic group substituted by a halogen atom or an optionally substituted alkylsulfonyloxy group, with a corresponding amine or a compound of the formula: 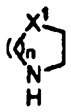 wherein the symbols have the same meanings as defined above.

The reaction can be carried out by optionally adding a copper catalyst in the presence or absence of a base in a suitable solvent

Copper iodide, copper bromide, copper chloride, copper acetate, copper trifluoromethanesulfonate, and the like can be preferably used as the copper catalyst.

The same base as that described in the above (EC) can be preferably used.

Additionally, the reaction proceeds more preferably when N,N'-dimethylethylenediamine, 1,10-phenanthroline, ethylene glycol, phenylphenol, and the like is added.

(E3) The compound wherein A is a heterocyclic group substituted by an optionally substituted alkylsulfanyl group can be prepared by reacting a compound wherein A is a heterocyclic group substituted by a halogen atom or an optionally substituted alkylsulfonyloxy group with a corresponding alkylthiol.

The reaction can be carried out in the same manner as in that described in the above (EI) and more preferably facilitated by adding 1,10-phenanthroline or ethylene glycol.

(E4) The compound wherein A is a heterocyclic group substituted by an optionally substituted heterocyclic group can be prepared by coupling a compound wherein A is a heterocyclic group substituted by a halogen atom or an optionally substituted alkylsulfonyloxy group with a corresponding heterocyclic tin compound or a corresponding heterocyclic boron compound.

The reaction can be carried out in the same manner as in the above (ED) or (EI).

(E5) The compound wherein A is a heterocyclic group substituted by an alkoxy group can be prepared by reacting a compound wherein A is a heterocyclic group substituted by a halogen atom or an alkylsulfonyl group with a corresponding alkaline metal alkoxide in a suitable solvent. The corresponding alkaline metal alkoxide can be obtained by treating a corresponding alkylalcohol with alkaline metal hydride or alkaline metal in the said solvent.

(E6) The compound having an aminoalkyl group as a substituent on A can be prepared by catalytically reducing a compound having a cyano group or a cyanoalkyl group as a substituent on A.

The catalytic reduction can be carried out by using a catalyst under hydrogen atmosphere in a suitable solvent in a conventional manner. The catalyst includes a palladium catalyst including palladium-carbon, a nickel catalyst including Raney nickel, a platinum catalyst including platinum-carbon, and the like.

(E7) The compound having an optionally substituted mono- or di-alkylsulfamoylaminoalkyl group as a substituent on A can be prepared by reacting a compound having an aminoalkyl group as a substituent on A with a corresponding halogenated mono- or di-alkylsulfamoyl.

The reaction can be carried out in a suitable solvent in the presence of a base.

(E8) The compound having an optionally substituted mono-alkylcarbamoylamnoalkyl group as a substituent on A can be prepared by reacting a compound having an aminoalkyl group as a substituent on A with a corresponding alkyl isocyanate in a suitable solvent.

(E9) The compound having a group of the formula: 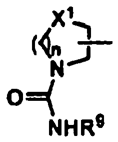 wherein R⁹ is an alkyl group and the other symbols have the same meanings as defined above as a substituent on A can be prepared by reacting a compound having a group of the formula: 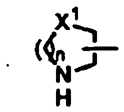 wherein the symbols have the same meanings as defined above as a substituent on A with a corresponding alkyl isocyanate (R⁹NCO). The reaction can be carried out in the same manner as in the above (E8).

(E10) The compound having an optionally substituted mono- or di-alkylcarbamoylaminoalkyl group as a substituent on A can be prepared by condensing a compound having an aminoalkyl group as a substituent on A with an optionally substituted mono- or di-alkylamine using a carbonylating agent in a suitable solvent in the presence or absence of a base.

A conventional carbonylating agent such as carbonyldiimidazole, phosgene, triphosgene, and the like can be used.

(E11) The compound having a morpholinylcarbonylamino group as a substituent on A can be prepared by condensing a compound having an amino group as a substituent on A with morpholine using a carbonylating agent in a suitable solvent. The reaction can be carried out in the same manner as in the above (E10).

(E12) The compound having a group of the formula: 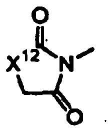 wherein X¹² is O or NH, as a substituent on A can be prepared by treating a compound having a group of the formula:

H-X¹²-CH₂-CONH-

wherein the symbols have the same meanings as defined above as a substituent on A with a carbonylating agent in a suitable solvent.

The reaction can be carried out in the same manner as in the above (E10).

(E12') The compound having a group of the formula: 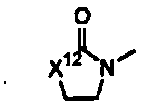 wherein X¹² is O or NH, as a substituent on A can be prepared by treating a compound having a group of the formula:

H-X¹²-CH₂-CH₂-NH-

wherein the symbols have the same meanings as defined above as a substituent on A with a carbonylating agent in a suitable solvent

The reaction can be carried out in the same manner as in the above (E10).

(E13) The compound having an optionally substituted carbamoyl group as a substituent on A can be prepared by condensing a compound having a carboxyl group as a substituent on A with a desirable amine.

The condensation can be carried out using a condensing agent in a suitable solvent A conventional condensing agent such as dicyclohexylcarbodiimide, 1-ethyl-3-(3-dimethylaminopropyl)carbodiimide, carbonyldiimidazole, and the like can be preferably used.

Additionally, the condensation can be more preferably carried out by adding an activating agent including 1-hydroxybenzotriazole, 1-hydroxysuccinimide, and the like.

(E14) The compound having a group of the formula: 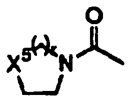 wherein the symbols have the same meanings as defined above as a substituent on A can be prepared by condensing a compound having a carboxyl group as a substituent on A with a compound of the formula: 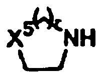 wherein the symbols have the same meanings as defined above.

The reaction can be carried out in the same manner as in the above (E13).

(E15) The compound having a tetrazolyl group as a substituent on A can be prepared by reacting a compound having a cyano group as a substituent on A with an alkaline metal azide in the presence of an acid in a suitable solvent.

The alkaline metal azide includes sodium azide, lithium azide, and the like.

An ammonium salt of a halogenated hydrogen including ammonium chloride can be preferably used as the acid.

(E16) The compound having an optionally substituted alkyl tetrazolyl group as a substituent on A can be prepared by alkylating a compound having a tetrazolyl group as a substituent on A.

The alkylation can be carried out in the same manner as in the above (EA).

(E17) The compound having an optionally substituted amino group or a group of the formula: 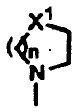 wherein the symbols have the same meanings as defined above as a substituent on A can be prepared by reacting a compound having a halogen atom or an optionally substituted alkylsulfonyloxy group as a substituent on A with a corresponding amine or a compound of the formula: 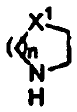 wherein the symbols have the same meanings as defined above. ,

The reaction can be preferably carried out in the presence or absence of a base in a suitable solvent.

(E18) The compound having an optionally substituted alkylamino group or a group of the formula: 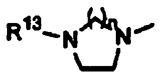 wherein R¹³ is an alkyl group optionally substituted by a hydroxy group, an alkoxycarbonyl group, a morpholinyl group or a phenyl group, and n has the same meaning as defined above, as a substituent on A can be obtained by reacting a compound having an amino group or a group of the formula 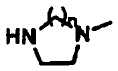 wherein the symbols have the same meanings as defined above as a substituent on A with a corresponding alkyl halide or a corresponding sulfonic alkyl esters.

The sulfonic alkyl esters including methanesulfonic ester, toluenesulfonic ester, trifluoromethanesulfonic ester, and the like can be preferably used.

The reaction can be preferably carried out in the presence or absence of a base in a suitable solvent.

(E19) The compound having a group of the formula: 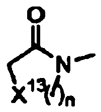 wherein X¹³ is O or NH, and the other symbol has the same meaning as defined above as a substituent on A, can be prepared by ring-closing a compound having a group of the formula:

Z³-(CH₂)ₙ-X¹³-CH₂-CONH-

wherein Z³ is a leaving group and the other symbols have the same meanings as defined above, as a substituent on A.

The reaction can be preferably carried out in the presence or absence of a base in a suitable solvent.

(E20) The compound having a carboxyl group as a substituent on A can be prepared by hydrolyzing a compound having an alkoxycarbonyl group as a substituent on A.

The hydrolysis can be carried out by treating a starting compound with a base or an acid in a suitable solvent according to a conventional manner. An alkaline metal hydroxide can be preferably used as the base.

(E21) The compound containing a carboxyl group as a substituent on A can be prepared by hydrolyzing a compound containing a cyano group as a substituent on A.

The hydrolysis can be carried out by treating a starting compound with an acid or a base in a suitable solvent.

(E22) The compound containing a carbamoyl group as a substituent on A can be prepared by hydrolyzing a compound containing a cyano group as a substituent on A.

The hydrolysis can be carried out by treating a starting compound with an acid or a base in a suitable solvent.

(E23) The compound having a carboxyalkyl group as a substituent on A can also be prepared by catalytically reducing a compound having a carboxyalkenyl group, a benzyloxycarbonylalkenyl group or a benzyloxycarbonylalkyl group as a substituent on A.

The catalytic reduction can be carried out in the same manner as in the above (E6).

(E24) The compound having a hydroxy group as a substituent on A can be prepared by hydrolyzing a compound wherein A has an alkanoyloxy group.

The hydrolysis can be carried out in the same manner as in the above (E20).

(E25) The compound containing sulfoxide (SO) or sulfone (SO2) in a substituent on A can be prepared by oxidizing a compound having S in a substituent on A (e.g., a compound having a thiomorpholinyl group or an alkylsulfanylalkyl group as a substituent on A).

The oxidation can be carried out by treating a starting compound with an oxidizing agent in a suitable solvent.

Peroxides such as hydrogen peroxide, m-chloroperbenzoic acid, acetyl hydroperoxide, and the like can be preferably used as the oxidizing agent.

(E26) The compound containing N-oxide in a substituent on A can be prepared by oxidizing a compound having N in a substituent on A (e.g., a compound having a pyridyl group as a substituent on A).

The oxidation can be carried out in the same manner as in the above (E25).

(E27) The compound having a 1,2-dihydroxyalkyl group as a substituent on A can be prepared by treating a compound having an alkyl group substituted by mono- or di-alkyldioxolanyl group as a substituent on A with an acid in a suitable solvent.

A strongly acidic resin can also be preferably used as the acid, in addition to those previously described.

(E28) The compound having an alkyl group substituted by a hydroxy group and an optionally substituted alkoxy group as substituents on A can be prepared by reacting a compound having an oxylanylalkyl group as a substituent on A with an alkaline metal salt of the corresponding alcohol in a suitable solvent.

The alkaline metal salt of alcohol includes a lithium salt, a sodium salt, a potassium salt, and the like.

(E29) The compound having an alkyl group substituted by a hydroxy group and an amino group, or an alkyl group substituted by a hydroxy group and an optionally substituted mono- or di-alkylamino group as substituents on A can be prepared by reacting a compound having an oxylanylalkyl group as a substituent on A with ammonia or a corresponding mono- or di-alkylamines in a suitable solvent.

(E30) The compound having a hydroxycarbamimidoyl group as a substituent on A can be prepared by reacting a compound having a cyano group as a substituent on A with hydroxylamine or a salt thereof in a suitable solvent Any solvent which does not disturb the reaction can be used, and as such, the solvent described in the above (EA) can be preferably used.

(E31) The compound having an oxodihydrooxadiazolyl group as a substituent on A can be prepared by reacting a compound having a hydroxycarbamimidoyl group as a substituent on A with a carbonylating agent in a suitable solvent in the presence or absence of a base.

The same carbonylating agent as that described in the above (E10) can be used.

(E32) The compound having a sulfo group as a substituent on A can be prepared by hydrolyzing a compound having an alkoxycarbonylalkylsulfonyl group as a substituent on A.

The hydrolysis can be carried out in the same manner as in the above (E20).

(E33) The compound having a sulfamoyl group as a substituent on A can be prepared by condensing a compound having a sulfo group as a substituent on A with a desirable amine.

The condensation can be carried out by treating a compound having a sulfo group as a substituent on A with a halogenating agent in a suitable solvent, followed by reacting the resulting compound with a desirable amine in the presence or absence of a base.

A conventional halogenating agent including thionyl halide, phosphorus oxyhalide, or the like can be used.

(E34) The compound having a hydroxyalkyl group as a substituent on A can be prepared by reducing a compound having a carboxyalkyl group as a substituent on A, or by converting the carboxyl group into an acid anhydride or an ester and then reducing the resulting compound.

A process for conversion into an acid anhydride can be carried out by reacting a starting compound with a halogenated alkyl formate in a suitable solvent in the presence of a base.

A process for conversion into an ester can be carried out by reacting a starting compound with an alcohol in the presence of a condensing agent in a suitable solvent. This process can be carried out in the same manner as in (E33) except that a desirable alcohol is used in place of amine.

The reduction can be carried out by treating the resulting compound with a reducing agent in a suitable solvent

Boron hydrides (e.g. sodium borohydride), aluminum hydrides (lithium aluminum hydride, diisobutylaluminum hydride, and the like) can be preferably used as the reducing agent.

(E35) The compound having an aromatic group substituted by a cyano group as a substituent on R¹, optionally having one to three heteroatoms independently selected from oxygen atom, sulfur atom and nitrogen atom (hereinafter, referred to as "an aromatic group"), can also be prepared by cyanating a compound having an aromatic group substituted by a halogen atom as a substituent on R¹.

The cyanation can be carried out in the same manner as in the above (EE).

(E36) The compound wherein A is a hydrogen atom can be prepared by acid-treatment or reduction of a compound wherein A is a tert-butoxycarbonyl group or a benzyloxycarbonyl group.

The acid-treatment can be carried out in the same manner as in the above (E27) and the reduction can be carried out in the same manner as in the above (E23).

(E37) The compound wherein A is an optionally substituted alkoxycarbonyl group, or an optionally substituted carbamoyl group can be prepared by reacting a compound wherein A is a hydrogen atom with a carbonylating agent, or a desirable alcohol or a desirable amine in a suitable solvents

The reaction can be carried out in the same manner as in the above (E10).

(E38) The compound having an amino group as a substituent on A can be prepared by undergoing a Curtius rearrangement reaction of a compound having a carboxyl group as a substituent on A.

Curtius rearrangement reaction can be carried out using a conventional azidating agent (e.g., diphenylphosphorylazide, and the like) in a suitable solvent in the presence of a base.

The reaction may also be carried out by adding alcohols to provide a compound having an optionally substituted alkoxycarbonylamino group as a substituent on A, followed by removing the alkoxycarbonyl group.

The removal of the alkoxycarbonyl group can be carried out in a conventional manner such as an acid-treatment or a reduction depending on the type of alkoxycarbonyl group to be removed. The acid-treatment can be carried out in the same manner as in the above (E27) and the reduction can be carried out in the same manner as in the above (E23).

(E39) The compound having a hydroxy group as a substituent on A can be prepared by catalytically reducing a compound having a benzyloxy group as a substituent on A. The reduction can be carried out in the same manner as in the above (E23).

(E40) The compound having an oxo group as a substituent on A can be prepared by oxidizing a compound having a hydroxy group as a substituent on A.

The oxidation can be carried out by using an oxidizing agent in a suitable solvent.

A conventional oxidizing agent can be used as the oxidizing agent, such as chromate-pyridine complex, pyridinium chlorochromate, pyridinium dichromate, Dess-Martin reagent (1,1,1-tris(acetoxy)-1,1-dihydro-1,2,benziodoxol-3-(1H)-one), dimethylsulfoxide, and the like.

(E41) The compound containing an optionally substituted alkoxy group as a substituent on A can be prepared by alkylating a compound containing an oxo group or a hydroxy group as a substituent on A.

The alkylation can be carried out by using a corresponding compound in the same manner as in the above (EA).

(E41') The compound containing an optionally substituted heterocyclyloxy group or an optionally substituted aryloxy group as a substituent on A can be prepared by coupling a compound containing a hydroxy group as a substituent on A with a corresponding aryl compound or a heterocyclic compounds which have a leaving group.

The coupling can be carried out in the same manner as in the above (EC).

The leaving group have the same meaning as defined above (EH).

(E42) The compound having an optionally substituted alkanoylamino group as a substituent on A can be prepared by condensing a compound having an amino group as a substituent on A with a corresponding carboxylic acid or a reactive derivative thereof.

The condensation with the corresponding carboxylic acid can be preferably carried out in a suitable solvent in the presence of a condensing agent. The reaction can be carried out in the same manner as in the above (E 13).

Additionally, the condensation with the reactive derivative of the corresponding carboxylic acid can be carried out in a suitable solvent or solvent-free in the presence or absence of a base.

The reactive derivative includes an acid halide, an acid anhydride, an activated ester, an activated amide, and the like.

(E43) The compound having a group of the formula: 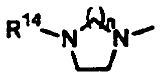 wherein R¹⁴ is an alkanoyl group optionally substituted by a hydroxy group or an alkoxy group, and n has the same meaning as defined above, as a substituent on A can be prepared by condensing a compound of a group of the formula: 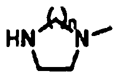 wherein the symbol has the same meaning as defined above, as a substituent on A with a corresponding carboxylic acid or a reactive derivative thereof.

The reaction can be carried out in the same manner as in the above (E42).

(E44) The compound having a maleimide group as a substituent on A can be prepared by reacting a compound having an amino group as a substituent on A with a maleic anhydride. The reaction can be carried out in a suitable solvent

(E45) The compound having an alkyl group substituted by a pyridyl group and a hydroxy group as substituents on A can be prepared by reacting a compound having an alkyl group substituted by a pyridyl group of which nitrogen atom is oxidized as a substituent on A with a trifluoroacetic anhydride. The reaction can be carried out in a suitable solvent.

(E46) The compound having a halogen atom as a substituent on A can be prepared by treating a compound having a hydroxy group as a substituent on A with a halogenating agent

As the halogenating agent, a conventional halogenating agent including thionyl chloride, phosphorus oxychloride, as well as carbon tetrahalide (e.g., carbon tetrachloride, carbon tetrabromide, and the like) and phosphines (e.g., triphenylphosphine, tritolylphosphine, triethylphosphine, and the like) can be preferably used.

(E46') The compound having a halogen atom as a substituent on A can be prepared by treating a compound with a halogenating agent. As the halogenating agent, a conventional halogenating agent such as bromine, N-bromosuccinimide, and the like can be preferably used.

(E47) The compound having a cyanoalkyl group as a substituent on A can be prepared by reducing a compound having a cyanoalkenyl group as a substituent on A.

The reduction can be carried out by treating a starting compound with a reducing agent or by catalytically reducing in a suitable solvent.

Any reducing agent can be used subject that it reduces only a double bond without affecting a cyano group. For example, sodium bis(2-methoxyethoxy)aluminum hydride in the presence of a copper bromide can be preferably used.

The catalytic reduction can be carried out in the same manner as in the above (E23).

(E48) The compound (1) having a hydroxyalkyl group as a substituent on A can be prepared by reducing a compound having a formyl group as a substituent on A.

The reduction can be carried out by treating a starting compound with a reducing agent in a suitable solvent.

The reaction can be carried out in the same manner as in the process for reducing in the above (E34).

(E49) The compound wherein a substituent on B is a hydroxy group can be prepared by demethylating a compound wherein the substituent on B is a methoxy group.

The demethylation can be carried out by treating a starting compound with a demethylating agent in a suitable solvent.

A conventional reagent including trimethylsilyl iodide, hydrogen bromide/acetic acid, boron tribromide, concentrated sulfuric acid, and the like can be used as the demethylating agent.

(E50) The compound wherein a substituent on B is an optionally substituted alkoxy group can be prepared by alkylating a compound wherein the substituent on B is a hydroxy group.

The alkylation can be carried out in the same manner as in the above (EA).

(E51) The compound wherein a substituent on B is an optionally substituted alkylsulfonyloxy group can be prepared by alkylsulfonylating a compound wherein the substituent on B is a hydroxy group.

The alkylsulfonylation can be carried out by reacting a corresponding alkylsulfonyl halide or a corresponding alkylsulfonic anhydride in a suitable solvent in the presence or absence of a base.

(E52) The compound wherein a substituent on B is a cyano group can be prepared by cyanating a compound wherein the substituent on B is an optionally substituted alkylsulfonyloxy group.

The cyanation can be carried out in the same manner as in the above (EE).

(E53) The compound wherein a substituent on B is an aminoalkyl group can be prepared by reducing a compound wherein the substituent on B is a cyano group.

The reduction can be carried out in the same manner as in the above (E6).

(E54) The compound wherein a substituent on B is an alkyl group can be prepared by alkylating a compound wherein the substituent on B is an optionally substituted alkylsulfonyloxy group.

The alkylation can be carried out by reacting alkyl aluminums in the presence of a palladium catalyst, a silver catalyst and a copper catalyst in a suitable solvent

Tetrakis(triphenylphosphine)palladium as the palladium catalyst, silver carbonate as the silver catalyst, copper. **(I)** chloride as the copper catalyst can be preferably used.

(E54') The compound wherein a substituent on B is an optionally substituted alkyl group can be prepared by catalytically reducing a compound wherein a substituent on B is an optionally substituted alkenyl group. The reaction can be carried out in the same manner as in the above (E6).

(E55) The compound having an imidazolinyl group or an oxazolinyl group as a substituent on A can be prepared by (i) reacting a compound containing a cyano group as a substituent on A with a desirable alcohol in the presence of an acid in a suitable solvent or solvent-free to provide a compound containing an alkoxycarbonimidoyl group as a substituent on A, and (ii) reacting the compound containing an alkoxycarbonimidoyl group as a substituent on A with 2-aminoethanol or ethylene diamine in a suitable solvent or solvent-free.

(E56) The compound having a carboxyl group as a substituent on A can be prepared by (i) oxidizing a compound containing a hydroxyalkyl group as a substituent on A in the same manner as in the above (E40) to provide a compound containing an oxo group as a substituent on A, and (ii) oxidizing the compound containing an oxo group as a substituent on A.

The oxidization for the second process can be carried out by using an oxidizing agent in a suitable solvent. Sodium chlorite, Silver(I) oxide, Sodium periodate and the like can be preferably used as the oxidizing agent.

(E57) The compound having a carboxyl group as a substituent on A can be directly prepared by oxidizing a compound containing a hydroxyalkyl group as a substituent on A.

The oxidization can be carried out by using Jones reagent, potassium permanganate, and the like as the oxidizing agent.

(E58) The compound wherein A is hydrogen atom can be prepared by treating a compound wherein A is ethoxycarbonyl group with a silyl halide or a base. Trimethylsilyl iodide can be preferably used as the silyl halide. Sodium hydroxide can be preferably used as the base.

In each process for preparing a compound (1) described above, when protection of a functional group contained in any compound is needed, the protection can be carried out in a conventional manner as appropriate. General statement related to protecting groups and their use is provided by Greene, Protective Groups in Organic Synthesis, John Wiley and Sons, New York, 1991.

When an amino group is protected by a benzyloxycarbonyl group, the protecting group can be removed by a catalytic reduction under hydrogen atmosphere in a suitable solvent.

When a hydroxy group is protected by a benzyl group, the protecting group can also be removed by a catalytic reduction in a similar manner as above.

When an amino group is protected by a tert-butoxycarbonyl group, the protecting group can be removed by treating a starting compound with an acid (e.g., hydrochloric acid, trifluoroacetic acid, toluenesulfonic acid, and the like) in a suitable solvent.

When a hydroxy group is protected by a tetrahydropyranyl group, the protecting group can also be removed by treating a starting compound with an acid in a similar manner as above.

Other substituents of the present compouind (1) can also be converted in the same manner as in the reactions (E1) to (E58) for conversion of the above groups.

### [Preparation of a compound (6')]

The compound (6') can be prepared according to the following method (a) or (b).
(a) The compound (6') can be prepared by reacting the compound of a general formula (10): 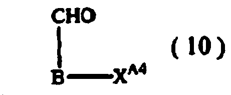 wherein X^{A4} is a leaving group and the other symbols have the same meanings as defined above, with the compound of a general formula (11):

   H-R² (11)

   wherein the symbol has the same meaning defined above, in the same manner as in the above process I-1 or process I'-1.
(b) The compound (6') can be prepared by reacting the compound of a general formula (12): 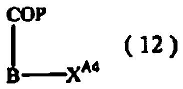
wherein P is a protecting group for a carboxyl group and the other symbols have the same meanings as defined above, with the compound (11) in the same manner as in the above process I-1 or process I'-1, followed by reducing the resulting compound to provide the compound (6") and further oxidizing the resulting compound.

The reduction and oxidation in the above methods can be carried out by the conventional method.

### [Preparation of a compound (2)]

The compound (2) can be prepared by oximating the compound (6') to provide the compound of a general formula (13): 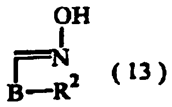 wherein the symbols have the same meanings as defined above, followed by reducing the resulting compound.

The oximation can be carried out by the conventional oximation methods, for example, by treating the compound (6') with a salt of hydroxylamine in the presence of an acid or a base such as alkaline metal hydroxide, sodium acetate or pyridine in an alcohol, acetic acid or pyridine. Also any acidic material can be used as the agent for preparing a salt of hydroxyl amine, for example, a mineral acid (e.g. sulfuric acid, phosphoric acid, hydrogen bromide and hydrogen iodide), and organic acid (e.g. acetic cid, oxalic acid, trichloroacetic acid, methanesulfonic acid, p-toluenesulfonic acid, 1,5-naphthalenedisulfonic acid).

The subsequent reduction reaction can be carried out in a conventional manner.

The compound (2) can also be prepared from the compound (6'') using the method of Gabriel synthesis, described in detail in Mitsunobu, O. Comp. Org. Syn. 1991, 6, 79-85.

### [Preparation of a compound (6'"')]

The compound (6"'") can be prepared from the compound of a general formula (14): 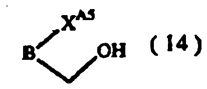 wherein X^{A5} is a leaving group and the other symbols have the same meanings as defined above, by a conventional insertion reaction of carbon monoxide aith a transition metal catalyst

The reaction can be carried out in an aprotic solvent such as tetrathydrofuran or DMF, and the like. Preferred transition metal includes, for example, a salt form of palladium such as palladium (II)-acetate, and the like or a palladium (0) compound such as tetrakis(triphenylphosphine)palladium, and the like. This kind of an insertion reaction of carbon monoxide can be carried out by the method described in detail in J. Org. Chem. 1992, 57, 5979 or "Organometallic compound-Synthesis and Application-(Tokyo Kagaku Dozin Co., Ltd.), Metal-catalyzed Cross-coupling Reaction (WILLY-VCH), Handbook of Palladium-Catalyzed Organic Reactions (Academic Press)", and the like.

The compound (1-a) wherein A¹ is tetrazolyl group and A² is a hydrogen atom can be prepared by cyanidating the above compound (8') to provide the compound of a general formula (8""): 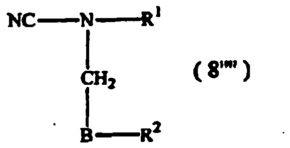 wherein the symbols have the same meanings as defined above, folowed by reacting with an alkaline metal azide.

The cyanidation can be carried out by reacting a starting compound with cyanogen halide in the presence of a base in a suitable solvent.

The cyanogen halide is preferably cyanogen bromide.

The conventional base can be preferably used as a base, such as alkaline metal carbonate (e.g. potassium carbonate) or an alkaline metal bicarbonate (e.g. sodium bicarbonate).

Any solvent which dose not disturb the reaction can be used as a solvent and the solvent illustrated in the above method I can be preferably used.

The conversion of a cyano group into a tetrazolyl group can be achieved by reacting the compound having a cyano group with an alkaline metal azide in the presence of an acid in a suitable solvent.

The alkaline metal azide includes sodium azide and lithium azide, and the like.

The ammonium salt of halogenated hydrogen such as ammonium chloride can be preferably used as an acid.

In addition, for performing the above methods, there can be referred to PCT International Publication WO04/020393 pamphlet, WO05/100298 pamphlet and JP. 2003-221376 A.

The methods for preparation of the compound (1) are applicable to preparation of the corresponding compounds of formula (I-A), (I-B) and (1-2).

Many of starting materials and reagents for preparation of the aforementioned compound of the formula 1 are either commercially available or disclosed in literatures, or can be readily prepared by a method that is disclosed in literatures or used generally in the organic synthesis.

### Experiment

The inhibitory activity of the compounds of the present invention against CETP was tested in this experiment.

### Preparation of Acceptor Microemulsion

A solution of 1-Palmitoyl-2-oleoyl-sn-glycero-3-phosphocholine (3.5 mg), cholesteryl oleate (3mg) and triolein (0.7 mg) in chloroform was mixed and lipid was air-dried under nitrogen gas to remove solvent. 1,4-Dioxane (0.25 ml) was then added and the mixture was stirred for dissolution. The resultant lipid solution (0.2 ml) was slowly injected under the surface of Tris-saline-EDTA(TSE) buffer solution [10mM Tris/HCl (pH 7.4), 0.15M NaCl, 2mM EDTA] (10 ml) with Hamilton syringe, while sonicating in ice-bath. After 1-hour-sonication in ice-bath, the solution was stored at 4 °C.

### Preparation of Donor Microemulsion

A solution of egg PC (phosphatidylcholine) (0.33 mg) and BODIPY-CE (0.62 mg) in chloroform was mixed. After removing solvent by air-drying lipid under nitrogen gas, TSE buffer solution (3 ml) was added and the solution was sonicated in ice-bath. This solution was filtered to sterilize through 0.22 µm filter and stored at 4 °C.

### Inhibitory Activity against CETP in vitro

A test solution was prepared using dimethyl sulfoxide as a solvent. Plasma from a healthy volunteer was diluted to 0.64 % with TSE buffer, and to the resultant plasma solution (187 µl) was added a test solution (3 µl) or the solvent alone followed by incubation at 37 °C for 24 hours. After addition of TSE buffer solution (10 µl) containing 5 % donor microemulsion and 5 % acceptor microemulsion, the mixture was incubated at 37 °C for 3 hours. Before and after the incubation, the fluorescence intensity was measured at Ex.550nm/Em.600nm. CETP activity was defined as the difference between the measurements obtained before incubation and after incubation. The decreasing rate of the difference in the sample was defined as the inhibition rate of CETP activity. IC₅₀ for each sample was calculated from the inhibition rate of CETP activity. Using this protocol, compounds given in Examples were shown to exhibit a CETP inhibitory activity with an IC₅₀ of less than or equal to 50 µM.

### EXAMPLES

The present invention is illustrated in more detail by Examples and Reference Examples, but the present invention should not be construed to be limited thereto.

### Example 1

(1) 2-Chloroquinoline-3-carboxaldehyde (650mg) and cyclopropylmethyl-propyl-amine (576mg) are dissolved in toluene (5ml), and thereto is added potassium carbonate (1.41g) and the mixture is stirred at 120°C overnight. The reaction solution is cooled to room temperature, and thereto are added ethyl acetate and water, and the mixture is separated, and the organic layer is washed with a saturated brine, dried over magnesium sulfate and concentrated under reduced pressure. The residue is dissolved in ethanol (5ml), and thereto is added sodium borohydride (128mg) at room temperature and the mixture is stirred for 1 hour. To the reaction solution are added saturated aqueous ammonium chloride solution and ethyl acetate, and the mixture is separated, and the organic layer is washed with a saturated brine, dried over magnesium sulfate and concentrated under reduced pressure. The resulting residue is purified by silica gel column chromatography (hexane : ethyl acetate = 1:0→3:1) to give-[2-(cyclopropylmethyl-propyl-amino)-quinolin-3-yl]-methanol (350mg). MS (m/z): 271 [M+H]⁺
(2) [2-(Cyclopropylmethyl-propyl-amino)-quinolin-3-yl]-methanol (100mg) is dissolved in methylene chloride (1ml) and thereto is added thionyl chloride (30µl) under ice-cooling, and the mixture is stirred at the same temperature for 10 minutes. To the reaction solution are added a saturated aqueous sodium bicarbonate solution and ethyl acetate, and the mixture is separated, and the organic layer is washed with a saturated brine, dried over magnesium sulfate, and concentrated under reduced pressure. Ethyl 4-[2-(3,5-bis-trifluoromethyl-benzylamino)-pyrimidin-5-yloxy]-butyrate (167mg) is dissolved in N,N-dimethylformamide (0.5ml) and thereto is added sodium hydride (60%) (14.8mg) under ice-cooling, and the mixture is stirred for 15 minutes. Thereto is added a solution of a residue obtained above in N,N-dimethylformamide (0.5ml) and the mixture is stirred at room temperature for 1 hour. To the reaction solution are added ethyl acetate and water, and the mixture is separated, and the organic layer is washed with a saturated brine, dried over magnesium sulfate and concentrated under reduced pressure. The resulting residue is purified by silica gel column chromatography (hexane : ethyl acetate = 49:1→9:1) to give ethyl 4-(2-{(3,5-bis-trifluoromethyl-benzyl)-[2-(cyclopropylmethyl-propyl-amino)-quinolin-3-ylmethyl]-amino}-pyrimidin-5-yloxy)-butyrate (35mg). MS (m/z): 704 [M+H]⁺
(3) Ethyl 4-(2-{(3,5-bis-trifluoromethyl-benzyl)-[2-(cyclopropylmethyl-propyl-amino)-quinolin-3-ylmethyl]-amino}-pyrimidin-5-yloxy)-butyrate (34mg) is dissolved in ethanol (0.5ml) and thereto is added a 2N-aqueous sodium hydroxide solution (72µl) and the mixture is stirred at room temperature overnight. The reaction solution is concentrated under reduced pressure and to the residue are added ethyl acetate and a dilute hydrochloric acid, and the mixture is separated and the organic layer is washed with a saturated brine, dried over magnesium sulfate and concentrated under reduced pressure to give 4-(2-{(3,5-bis-trifluoromethylbenzyl)-[2-(cyclopropylmethyl-propyl-amino)-quinolin-3-ylmethyl]-amino}-pyrimidin-5-yloxy)-butyric acid (30mg). MS (m/z): 676 [M+H]⁺

### Example 2

The corresponding starting compound is treated in a similar manner to Example 1 to give the compound as listed in Table 1.

### Example 3

(1) 2-Chloroquinoline-3-carboxaldehyde (330mg) is dissolved in 1,2-dimethoxy-ethane (7.5m1), and thereto are added a 2M-aqueous sodium carbonate solution (1.72ml), water (2.5ml) and ethanol (2.5ml), and the mixture is degassed under reduced pressure and flushed with nitrogen gas. Thereto are added 5-isopropyl-2-methoxyphenylboronic acid (417mg) and tetrakis(triphenylphosphine)palladium (199mg), and the mixture is flushed with nitrogen gas again, and the mixture is set in the microwave instrument, heated to 150°C by irradiating microwave of 200W and stirred for 15 minutes. The reaction solution is cooled to room temperature, and thereto are added diethyl ether and water and the mixture is separated, and the organic layer is washed with a saturated brine, dried over magnesium sulfate and concentrated under reduced pressure. The residue is purified by silica gel column chromatography (hexane : ethyl acetate = 19:1→3:1) to give 2-(5-isopropyl-2-methoxy-phenyl)-quinoline-3-carboxaldehyde (493mg). MS (m/z): 306 [M+H]⁺
(2) 2-(5-Isopropyl-2-methoxy-phenyl)-quinoline-3-carboxaldehyde (470mg) is dissolved in ethanol (5ml), and thereto is added sodium borohydride (58mg) at room temperature and the mixture is stirred overnight. To the reaction solution are added a saturated aqueous ammonium chloride solution and diethylether, and the mixture is separated, and the organic layer is washed with a saturated brine, dried over magnesium sulfate and concentrated under reduced pressure. The residue is purified by silica gel column chromatography (hexane : ethyl acetate = 4:1→1:1) to give [2-(5-isopropyl-2-methoxy-phenyl)-quinolin-3-yl]-methanol (415mg). MS (m/z): 308 [M+H]⁺
(3) [2-(5-Isopropyl-2-methoxy-phenyl)-quinolin-3-yl]-methanol (409mg) is dissolved in methylene chloride (25ml) and thereto is added thionyl chloride (107µl) under ice-cooling, and the mixture is stirred at room temperature for 15 minutes and concentrated under reduced pressure. The residue and (3,5-bis-trifluoromethyl-benzyl)-(5-bromopyrimidin-2-yl)-amine (532mg) are dissolved in N,N-dimethylformamide (5ml) and thereto is added sodium hydride (62%) (103mg) under ice-cooling and the mixture is stirred at room temperature overnight. To the reaction solution are added diethyl ether and water and the mixture is separated, and the organic layer is washed with a saturated brine, dried over magnesium sulfate and concentrated under reduced pressure. The resulting residue is purified by silica gel column chromatography (hexane : ethyl acetate = 19:1→4:1) to give (3,5-bis-trifluoromethyl-benzyl)-(5-bromo-pyrimidin-2-yl)-[2-(5-isopropyl-2-methoxy-phenyl)-quinolin-3-ylmethyl]amine (450mg). MS (m/z): 689/691 [M+H]⁺

### Example 4

(1) (3,5-Bis-trifluoromethyl-benzyl)-(5-bromo-pyrimidin-2-yl)-[2-(5-isopropyl-2-methoxy-phenyl)-quinolin-3-ylmethyl]-amine (200mg) is dissolved in toluene (3ml), and thereto are added tris(dibenzylideneacetone)dipalladium (26.6mg), sodium tert-butoxide (41.8mg), 2-(di-tert-butylphosphino)biphenyl (34.6mg) and ethyl piperidine-4-carboxylate (65µl) and the mixture is stirred at room temperature under nitrogen flow overnight. To the reaction solution is added a saturated brine, and the mixture is extracted with diethyl ether. The organic layer is washed with a saturated brine, dried over magnesium sulfate and concentrated under reduced pressure. The resulting residue is purified by silica gel column chromatography (hexane : ethyl acetate = 43:7→73:27) to give ethyl 1-(2-{(3,5-bis-trifluoromethyl-benzyl)-[2-(5-isopropyl-2-methoxyphenyl)-quinolin-3-ylmethyl]-amino}-pyrimidin-5-yl)-piperidine-4-carboxylate (85mg), MS (m/z): 766 [M+H]⁺
(2) Ethyl 1-(2-{(3,5-bis-trifluoromethyl-benzyl)-[2-(5-isopropyl-2-methoxy-phenyl)-quinolin-3-ylmethyl]-amino}-pyrimidin-5-yl)-piperidine-4-carboxylate (80mg) is dissolved in ethanol (3ml), and thereto is added a 2N-aqueous sodium hydroxide solution (157µl) and the mixture is stirred at room temperature overnight. To the reaction solution are added diethyl ether and water and the pH of aqueous layer is made pH 4 with a 1N-hydrochloric acid, and the mixture is separated, and the organic layer is washed with a saturated brine, dried over magnesium sulfate and concentrated under reduced pressure. The resulting residue is purified by silica gel column chromatography (chloroform : methanol = 1:0→93:7) to give 1-(2-{(3,5-bis-trifluoromethyl-benzyl)-[2-(5-isopropyl-2-methoxyphenyl)-quinolin-3-ylmethyl]-amino}-pyrimidin-5-yl)-piperidine-4-carboxylic acid (75mg). MS (m/z): 738 [M+H]⁺

### Example 5

(1) (3,5-Bis-trifluoromethyl-benzyl)-(5-bromo-pyrimidin-2-yl)-[2-(5-isopropyl-2-methoxy-phenyl)-quinolin-3-ylmethyl]-amine (165mg), [1,1'-bis(diphenylphosphino)ferrocene]dichoropalladium methylene chloride complex (39mg), potassium acetate (70mg) and bis(pinacolate)diboron (122mg) are dissolved in dimethylsulfoxide (1.6ml), and the mixture is heated to 80°C under nitrogen atmosphere and stirred for 1 hour. The reaction solution is cooled to room temperature and thereto are added water and diethyl ether, and the mixture is separated, and the organic layer is washed with a saturated brine, dried over magnesium sulfate and concentrated under reduced pressure. The resulting residue is dissolved in tetrahydrofuran (5ml) and thereto is added dropwise a 30 % aqueous hydrogen peroxide solution (0.5ml) under ice-cooling. One hour thereafter, thereto is added a saturated aqueous sodium thiosulfate solution under ice-cooling to consume the excess hydrogen peroxide, and thereto are added water and etyl ether and the mixture is separated. The organic layer is washed with a saturated brine, dried over magnesium sulfate and concentrated under reduced pressure. The resulting residue is purified by silica gel column chromatography (hexane : ethyl acetate = 4:1→67:33) to give 2-{(3,5-bis-trifluoromethyl-benzyl)-[2-(5-isopropyl-2-methoxy-phenyl)-quinolin-3-ylmethyl]-amino}-pyrimidin-5-ol (116mg). MS (m/z): 627 [M+H]⁺
(2) 2-{(3,5-Bis-trifluoromethyl-benzyl)-[2-(5-isopropyl-2-methoxyphenyl)-quinolin-3-ylmethyl]-amino}pyrimidin-5-ol (110mg) and ethyl 4-bromobutyrate (28µl) are dissolved in N,N-dimethylformamide (2ml) and thereto is added potassium carbonate (29mg), and the mixture is stirred at room temperature overnight. Thereto are added diethyl ether and a saturated brine, and the mixture is separated, and the organic layer is washed with a saturated brine, dried over magnesium sulfate and concentrated under reduced pressure. The resulting residue is purified by silica gel column chromatography (hexane : ethyl acetate = 41:9→7:3) to give ethyl 4-(2-{(3,5-bis-trifluoromethyl-benzyl)-[2-(5-isopropyl-2-methoxyphenyl)-quinolin-3-ylmethyl]-amino}-pyrimidin-5-yloxy)-butyrate (71mg). MS (m/z): 741 [M+H]⁺
(3) Ethyl 4-(2-{(3,5-bis-trifluoromethyl-benzyl)-[2-(5-isopropyl-2-methoxy-phenyl)-quinolin-3-ylmethyl]-amino}-pyrimidin-5-yloxy)-butyrate (65mg) is dissolved in ethanol (3ml), and thereto is added a 2N-aqueous sodium hydroxide solution (132µl) and the mixture is stirred at room temperature overnight. Thereto are added diethyl ether and water, and the pH of aqueous layer is made pH 4 with a 1N-hydrochloric, and the mixture is separated, and the organic layer is washed with a saturated brine, dried over magnesium sulfate and concentrated under reduced pressure. The resulting residue is purified by silica gel column chromatography (chloroform : methanol = 1:0→47:3) to give 4-(2-{(3,5-bis-trifluoromethylbenzyl)-[2-(5-isopropyl-2-methoxy-phenyl)-quinolin-3-ylmethyl]-amino}-pyrimidin-5-yloxy)-butyric acid (51mg). MS (m/z): 713 [M+H]⁺

### Example 6

(1) To toluene (50ml) are added 2-chloroquinoline-3-carboxaldehyde (8.97g) and cyclopropylmethyl-propyl-amine (7.95g), followed by an addition of potassium carbonate (19.4g), and the mixture is stirred at 120°C for 8 hours. The reaction solution is cooled to room temperature, and thereto is added water and the mixture is separated, and the organic layer is washed with a saturated brine, dried over magnesium sulfate, and concentrated under reduced pressure. The resulting residue is purified by silica gel column chromatography (hexane : ethyl acetate = 19:1→4:1) to give 2-(cyclopropylmethyl-propyl-amino)-quinoline-3-carboxaldehyde (9.3g). MS (m/z): 269 [M+H]⁺
(2) 2-(Cyclopropylmethyl-propyl-amino)-quinoline-3-carboxaldehyde (750mg), 3,5-bis-trifluoromethyl-benzylamine (883mg) and acetic acid (479µl) are dissolved in 1,2-dichloroethane (5ml), and thereto is added triacetoxy sodium borohydride (1.18g) at room temperature and the mixture is stirred overnight. To the reaction solution are added methylene chloride and a 1N-aqueous sodium hydroxide solution, and the mixture is separated, and the organic layer is washed with a saturated brine, dried over magnesium sulfate, and concentrated under reduced pressure. The resulting residue is purified by silica gel column chromatography (hexane : ethyl acetate = 97:3→17:3) to give {3-[(3,5-bis-trifluoromethyl-benzylamino)-methyl]-quinolin-2-yl}-cyclopropylmethyl-propyl-amine (1.30g). MS (m/z): 496 [M+H]⁺
(3) {3-[(3,5-Bis-trifluoromethyl-benzylamino)-methyl]-quinolin-2-yl}-cyclopropylmethyl-propyl-amine (979mg) is dissolved in ethanol (10ml) and thereto are added sodium bicarbonate (505mg) and cyanogen bromide (230mg), and the mixture is stirred at room temperature overnight. Thereto are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 9:1→3:1) to give (3,5-bis-trifluoromethyl-benzyl)-[2-(cyclopropylmethyl-propyl-amino)-quinolin-3-ylmethyl)-cyanamide (1.02g). MS (m/z): 521 [M+H]⁺
(4) To N,N-dimethylformamide (10ml) are added (3,5-bis-trifluoromethyl-benzyl)-[2-(cyclopropylmethyl-propyl-amino)-quinolin-3-ylmethyl]-cyanamide (950mg), sodium azide (1.186g) and ammonium chloride (976mg), and the mixture is stirred at 100°C overnight. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (chloroform : methanol = 1:0→19:1) to give (3-{[(3,5-bis-trifluoromethylbenzyl)-(2H-tetrazol-5-yl)-amino]-methyl}-quinolin-2-yl)-cyclopropylmethyl-propyl-amine (696mg). MS (m/z): 564 [M+H]⁺

### Example 7

(1) To N,N-dimethylformamide (1ml) are added (3-{[(3,5-bis-trifluoromethyl-benzyl)-(2H-tetrazol-5-yl)-amino]-methyl}-quinolin-2-yl)-cyclopropylmethyl-propyl-amine (250mg), ethyl 2-bromo-2-methyl-propionate (195µl), a catalytic amount of potassium iodide, and triethylamine (1ml), and the mixture is stirred at 70°C overnight. The reaction solution is cooled to room temperature, and thereto are added diethyl ether and water, and the mixture is separated, and the organic layer is washed with a saturated brine, dried over magnesium sulfate, and concentrated under reduced pressure. The resulting residue is purified by NH-silica gel column chromatography (hexane : ethyl acetate = 97:3→22:3) to give ethyl 2-(5-{(3,5-bis-trifluoromethyl-benzyl)-[2-(cyclopropylmethyl-propyl-amino)-quinolin-3-ylmethyl]-amino}-tetrazol-2-yl)-2-methyl-propionate (205mg). MS (m/z): 678 [M+H]⁺
(2) Ethyl 2-(5-{(3,5-bis-trifluoromethyl-benzyl)-[2-(cyclopropylmethyl-propyl-amino)-quinolin-3-ylmethyl]-amino}-tetrazol-2-yl)-2-methyl-propionate (200mg) is dissolved in a mixed solvent of ethanol (6ml) and water (1ml), and thereto is added lithium hydroxide monohydrate (24.8mg) and the mixture is stirred at room temperature overnight. Thereto are added diethyl ether and water, and the pH of aqueous layer is made 4 with a 1N-hydrochloric acid, and the mixture is separated, and the organic layer is washed with a saturated brine, dried over magnesium sulfate, and concentrated under reduced pressure. The resulting residue is purified by silica gel column chromatography (chloroform : methanol = 1:0→9:1) to give 2-(5-{(3,5-bis-trifluoromethyl-benzyl)-[2-(cyclopropylmethyl-propylamino)-quinolin-3-ylmethyl]-amino}-tetrazol-2-yl)-2-methyl-propionic acid (172mg). MS (m/z): 650 [M+H]⁺

### Example 8

(1) (3-{[(3,5-Bis-trifluoromethyl-benzyl)-(2H-tetrazol-5-yl)-amino]-methyl}-quinolin-2-yl)-cyclopropylmethyl-propyl-amine (250mg), methyl 3-hydroxy-2,2-dimethyl-propionate (94µl) and triphenylphosphine (193mg) are dissolved in tetrahydrofuran (5ml), and thereto is added a solution of 40% diethyl azodicarboxylate in toluene (290µl) at room temperature. The reaction solution is stirred at room temperature overnight and concentrated under reduced pressure. The resulting residue is purified by silica gel column chromatography (hexane : ethyl acetate = 19:1→41:9) to give methyl 3-(5-{(3,5-bis-trifluoromethyl-benzyl)-[2-(cyclopropylmethyl-propyl-amino)-quinolin-3-ylmethyl]-amino}-tetrazol-2-yl)-2,2-dimethyl-propionate (174mg). MS (m/z): 678 [M+H]⁺
(2) Methyl 3-(5-{(3,5-bis-trifluoromethyl-benzyl)-[2-(cyclopropylmethyl-propyl-amino)-quinolin-3-ylmethyl]-amino}-tetrazol-2-yl)-2,2-dimethyl-propionate (170mg) is dissolved in a mixed solvent of ethanol (6ml) and water (1ml), and thereto is added lithium hydroxide monohydrate (21mg), and the mixture is stirred at room temperature overnight. The reaction solution is concentrated under reduced pressure, and to the residue is added diethyl ether and water, and the pH of aqueous layer is made pH 4 with a 1N-hydrochloric acid, and the mixture is separated, and the organic layer is washed with a saturated brine, dried over magnesium sulfate, and concentrated under reduced pressure. The resulting residue is purified by silica gel column chromatography (chloroform : methanol = 1:0→97:3) to give 3-(5-{(3,5-bis-trifluoromethyl-benzyl)-[2-(cyclopropylmethyl-propylamino)-quinolin-3-ylmethyl]-amino}-tetrazol-2-yl)-2,2-dimethyl-propionic acid (153mg). MS (m/z): 664 [M+H]⁺

**Table 1**

| | | | |
|---|---|---|---|
| 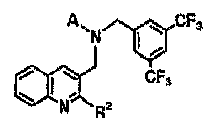 | | | |

| Ex. No. | A- | -R² | Physical properties, etc. |
|---|---|---|---|
| 1 | 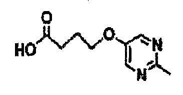 | 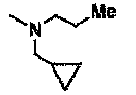 | MS (m/z) : 676 [M + H]₊ |
| 2 | 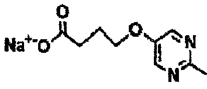 | 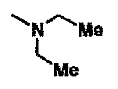 | MS (m/z) : 634 [M - Na ]⁻ |
| 3 | 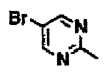 | 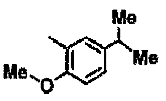 | MS (m/z) : 689/691 [M+H]⁺ |
| 4 | 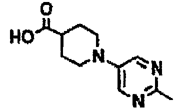 | 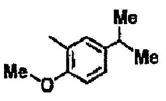 | MS (m/z) : 738 [M+H]⁺ |
| 6 | 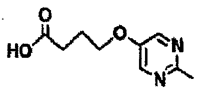 | 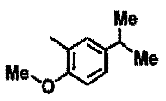 | MS (m/z) : 713 [M+H] ⁺ |
| 6 | 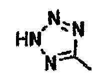 | 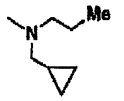 | MS (m/z) : 564 [M + H]⁺ |
| 7 | 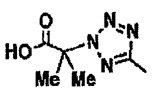 | 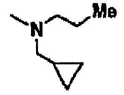 | MS (m/z) : 650 [M+H]⁺ |
| 8 | 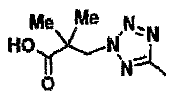 | 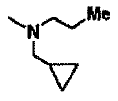 | MS (m/z) : 664 [M+H]⁺ |

### Example 9

(1) 2-Chloro-6-methoxy-quinoline-3-carboxaldehyde (2.0g) and cyclopropylmethyl-propyl-amine (1.53g) are dissolved in toluene (10ml), and thereto is potassium carbonate (3.74g) and the mixture is stirred at 120°C overnight The reaction solution is cooled to room temperature, and thereto is added water and the mixture is separated, and the organic layer is washed with a saturated brine, dried over magnesium sulfate, and concentrated under reduced pressure. The resulting residue is purified by silica gel column chromatography (hexane : ethyl acetate = 99:1→9:1) to give 2-(cyclopropylmethyl-propyl-amino)-6-methoxy-quinoline-3-carboxaldehyde (2.03g). MS (m/z): 299 [M+H]⁺
(2) 2-(Cyclopropylmethyl-propyl-amino)-6-methoxy-quinoline-3-carboxaldehyde (1.62g) is dissolved in ethanol (10ml) and thereto is added sodium borohydride (205mg) at room temperatue, and the mixture is stirred at room temperature for 1 hour. To the reaction solution are added a saturated aqueous ammonium chloride solution and diethyl ether, and the mixture is separated, and the organic layer is washed with a saturated brine, dried over magnesium sulfate, and concentrated under reduced pressure. The resulting residue is purified by silica gel column chromatography (hexane : ethyl acetate = 9:1→7:3) to give [2-(cyclopropylmethyl-propyl-amino)-6-methoxy-quinolin-3-yl]-methanol (1.64g). MS (m/z): 301 [M+H]⁺
(3) [2-(Cyclopropylmethyl-propyl-amino)-6-methoxy-quinolin-3-yl]-methanol (1.54g) is dissolved in methylene chloride (5ml), and thereto is added thionyl chloride (411µl) under ice-cooling, and the mixture is stirred at the same temperature for 15 minutes and concentrated under reduced pressure. The resulting residue and (3,5-bis-trifluoromethyl-benzyl)-(5-bromopyrimidin-2-yl)-amine (2.05g) are dissolved in N,N-dimethylformamide (10ml), and thereto is added sodium hydride (62%) (397mg) under ice-cooling, and the mixture is stirred at room temperature overnight. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 99:1→9:1) to give (3-{[(3,5-bis-trifluoromethyl-benzyl)-(5-bromo-pyrimidin-2-yl)-amino]-methyl}-6-methoxy-quinolin-2-yl)-cyclopropylmethyl-propyl-amine (2.94g). MS (m/z): 682/684 [M+H]⁺

### Example 10

The corresponding starting compound is treated in a similar manner to Example 4 to give the compound as listed in Table 2.

**Table 2**

| | | |
|---|---|---|
| 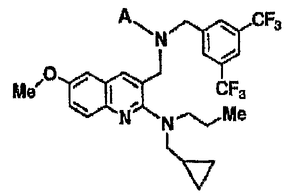 | | |

| Ex. No. | A- | Physical properties, etc. |
|---|---|---|
| 9 | 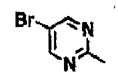 | MS (m/s) : 682/684 [M+H]⁺ |
| 10 | 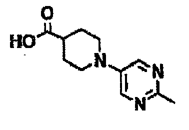 | MS (m/z) : 731 [M+H]⁺ |

### Example 11

The corresponding starting compound is treated in a similar manner to Example 9 to give the compound as listed in Table 3.

### Example 12

The corresponding starting compound is treated in a similar manner to Example 4 to give the compound as listed in Table 3.

**Table 3**

| | | |
|---|---|---|
| 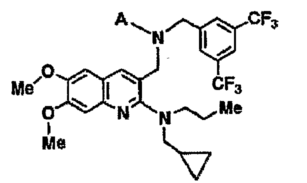 | | |

| Ex. No. | A- | Physical properties, etc. |
|---|---|---|
| 11 | 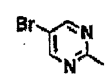 | MS (m/z) : 712/714 [M+H]⁺ |
| 12 | 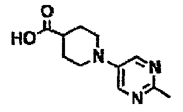 | MS (m/z) : 761 [M+H]⁺ |

### Example 13

The corresponding starting compound is treated in a similar manner to Example 9 to give the compound as listed in Table 4.

### Example 14

The corresponding starting compound is treated in a similar manner to Example 4 to give the compound as listed in Table 4.

**Table 4**

| | | |
|---|---|---|
| 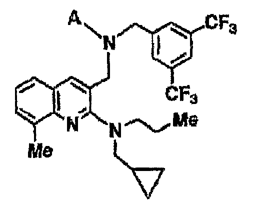 | | |

| Ex. No. | A- | Physical properties, etc. |
|---|---|---|
| 13 | 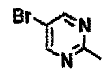 | MS (m/z) : 666/668 [M + H]⁺ |
| 14 | 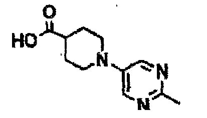 | MS (m/z) : 715 [M+H]⁺ |

### Example 15

The corresponding starting compound is treated in a similar manner to Example 6 to give the compound as listed in Table 5.

### Example 16

The corresponding starting compound is treated in a similar manner to Example 7 to give the compound as listed in Table 5.

### Example 17

The corresponding starting compound is treated in a similar manner to Example 8 to give the compound as listed in Table 5.

### Examples 18

The corresponding starting compound is treated in a similar manner to Example 9 to give the compound as listed in Table 5.

### Example 19

The corresponding starting compound is treated in a similar manner to Example 4 to give the compound as listed in Table 5.

### Example 20

(1) (3,5-Bis-trifluoromethyl-benzyl)-(5-bromo-pyrimidin-2-yl)-[5-(cyclopropylmethyl-propyl-amino)-1-methyl-3-trifluoromethyl-1H-pyrazol-4-ylmethyl]-amine (500mg) is dissolved in 1,4-dioxane (5ml), and thereto are added benzyl acrylate (217mg), tri-tert-butylphosphine tetrafluoroboric acid salt (21.5mg), N-ethyldiisopropylamine (155µl) and tris(dibenzylideneacetone)dipalladium (34mg), and the mixture is stirred at room temperature under nitrogen flow overnight. To the reaction solution are added diethyl ether and water, and the mixture is separated. The organic layer is washed with a saturated brine, dried over magnesium sulfate, and concentrated under reduced pressure. The resulting residue is purified by silica gel column chromatography (hexane : ethyl acetate = 91:9→4:1) to give benzyl 3-(2-{(3,5-bis-trifluoromethyl-benzyl)-[5-(cyclopropylmethyl-propyl-amino)-1-methyl-3-trifluoromethyl-1H-pyrazol-4-ylmethyl]-amino}-pyrimidin-5-yl)-acrylate (447mg). MS (m/z): 755 [M+H]⁺
(2) Benzyl 3-(2-{(3,5-bis-trifluoromethyl-benzyl)-[5-(cyclopropylmethyl-propyl-amino)-1-methyl-3-trifluoromethyl-1H-pyrazol-4-ylmethyl]-amino}-pyrimidin-5-yl)-acrylate (440mg) is dissolved in a mixed solvent of tetrahydrofuran (5ml) and ethanol (1ml), and thereto is added 10% palladium-carbon (50mg) and the mixture is stirred under hydrogen atmosphere at room temperature overnight The catalyst is removed by filtration, and the filtrate is concentrated under reduced pressure. The resulting residue is purified by silica gel column chromatography (chloroform : methanol = 1:0→47:3) to give 3-(2-{(3,5-bis-trifluoromethylbenzyl)-[5-(cyclopropylmethyl-propyl-amino)-1-methyl-3-trifluoromethyl-1H-pyrazol-4-ylmethyl]-amino}-pyrimidin-5-yl)-propionic acid (363mg). MS (m/z): 667 [M+H]⁺

**Table 5**

| | | |
|---|---|---|
| 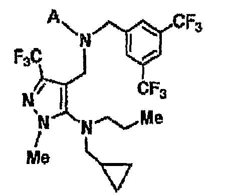 | | |

| Ex. No. | A- | Physical properties, etc. |
|---|---|---|
| 15 | 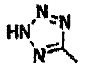 | MS (m/z) : 585 [M+H]⁺ |
| 16 | 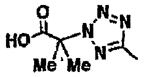 | MS (m/z) : 671 [M+H]⁺ |
| 17 | 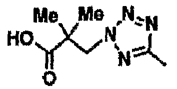 | MS (m/z) : 685 [M+H] ⁺ |
| 18 | 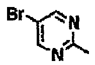 | MS (m/z) : 673/675 [M + H]⁺ |
| 19 | 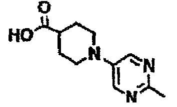 | MS (m/z) : 722 [M+H]⁺ |
| 20 | 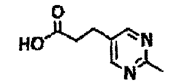 | MS (m/z) : 667 [M+H]⁺ |

### Example 21

(1) 5-Chloro-2-fluoropyridine-3-carboxaldehyde (970mg) and cyclopropylmethyl-propyl-amine (1.3ml) are dissolved in toluene (10ml), and thereto is added potassium carbonate (2.52g) and the mixture is stirred at 120°C for 30 minutes. The reaction solution is cooled to room temperature, and thereto are added ethyl acetate and water, and the mixture is separated, and the organic layer is washed with a saturated brine, dried over magnesium sulfate, and concentrated under reduced pressure. The resulting residue is purified by silica gel column chromatography (hexane : ethyl acetate = 19:1→17:3) to give 5-chloro-2-(cyclopropylmethyl-propyl-amino)-pyridine-3-carboxaldehyde (1.49g). MS (m/z): 253/255 [M+H]⁺
(2) 5-Chloro-2-(cyclopropylmethyl-propyl-amino)-pyridine-3-carboxaldehyde (1.45g) is dissolved in ethanol (10ml) and thereto is added sodium borohydride (217mg) at room temperature and the mixture is stirred for 1 hour. To the reaction solution are added a saturated aqueous ammonium chloride solution and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 24:1→17:3) to give [5-chloro-2-(cyclopropylmethyl-propylamino)-pyridin-3-yl)-methanol (1.35g). MS (m/z): 255/257 [M+H]⁺
(3) [5-Chloro-2-(cyclopropylmethyl-propyl-amino)-pyridin-3-yl]-methanol obtained in the above (2) as the starting compound is treated in similar manners to Examples 1(2) and (3) to give 4-(2-{(3,5-bis-trifluoromethylbenzyl)-[5-chloro-2-(cyclopropylmethyl-propyl-amino)-pyridin-3-ylmethyl]-amino}-pyrimidin-5-yloxy)-butyric acid sodium salt. MS (m/z): 658/660 [M-Na]⁻

### Example 22

[5-Chloro-2-(cyclopropylmethyl-propyl-amino)-pyridin-3-yl]-methanol prepared in the Example 21(2) as the starting compound is treated in a similar manner to Example 9(3) to give (3,5-bis-trifluoromethyl-benzyl)-(5-bromo-pyrimidin-2-yl)-[5-chloro-2-(cyclopropylmethyl-propyl-amino)-pyridin-3-ylmethyl]-amine. MS (m/z): 636/638 [M+H]⁺

### Example 23

The corresponding starting compound is treated in a similar manner to Example 4 to give the compound as listed in Table 6.

### Example 24

Thereto are added (3,5-bis-trifluoromethyl-benzyl)-(5-bromo-pyrimidin-2-yl)-[5-chloro-2-(cyclopropylmethyl-propyl-amino)-pyridin-3-ylmethyl]-amine (150mg) is dissolved in toluene (5ml) and thereto are added tris(dibenzylideneacetone)dipalladium (22mg), sodium tert-butoxide (34mg) and 2-(di-tert-butylphosphino)biphenyl (28mg) and morpholine (31µl),, and the mixture is stirred under nitrogen flow at room temperature overnight. The reaction solution is washed with a saturated brine, and the organic layer is dried over magnesium sulfate and concentrated under reduced pressure. The resulting residue is purified by NH-silica gel column chromatography (hexane : ethyl acetate = 49:1→87:13) to give (3,5-bis-trifluoromethyl-benzyl)-[5-chloro-2-(cyclopropylmethyl-propyl-amino)-pyridin-3-ylmethyl]-(5-morpholin-4-yl-pyrimidin-2-yl)-amine (88mg). MS (m/z): 643/645 [M+H]⁺

**Table 6**

| | | |
|---|---|---|
| 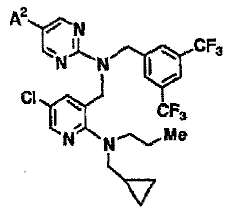 | | |

| Ex. No. | A²- | Physical properties, etc. |
|---|---|---|
| 21 | 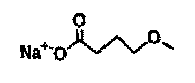 | MS (m/z) : 658/660 [M-Na]⁻ |
| 22 | 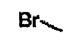 | MS (m/z) : 636/638 [M+H]⁺ |
| 23 | 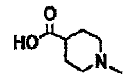 | MS (m/z) : 685/687 [M+H]⁺ |
| 24 | 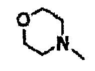 | MS (m/z) : 643/645 [M+H]⁺ |

### Example 25

(1) Ethyl 4-chloro-2-methylsulfanil-pyrimidine-5-carboxylate (10.00g) is dissolved in tetrahydrofuran (120ml) and thereto are added cyclopropylmethyl-propyl-amine (7.38ml) and triethylamine (7.19ml) in water bath, and the mixture is stirred at room temperature for 1 hour. To the reaction mixture is added a saturated brine, and the mixture is extracted with ethyl acetate and the organic layer is washed with a saturated brine, dried over magnesium sulfate, and concentrated under reduced pressure. The resulting residue is purified by silica gel column chromatography (hexane : ethyl acetate = 17:3) to give ethyl 4-(cyclopropylmethyl-propyl-amino)-2-methylsulfanil-pyrimidine-5-carboxyate (11.07g). MS (m/z): 310 [M+H]⁺
(2) Ethyl 4-(cyclopropylmethyl-propyl-amino)-2-methylsulfanil-pyrimidine-5-carboxylate (10.58g) is dissolved in tetrahydrofuran (50ml), and to a suspension of lithium aluminum hydride (1.30g) and tetrahydrofuran (50ml) is added dropwise the resulting solution under ice-cooling, and the mixture is stirred at the same temperature for 2 hours. To the reaction mixture are added a 1M aqueous sodium hydroxide solution (1.3ml) and water (1.3ml) under ice-cooling, and the mixture is stirred at the same temperature for 30 minutes, and the resulting precipitate is filtered and the resulting filtrate is dried over magnesium sulfate and concentrated under reduced pressure. The resulting residue is purified by NH-silica gel column chromatography (hexane : ethyl acetate= 2:1→1:4) to give 4-(cyclopropylmethyl-propyl-amino)-2-methylsulfanil-pyrimidin-5-yl]-methanol (2.24g). MS (m/z): 268 [M+H]⁺
(3) [4-(Cyclopropylmethyl-propyl-amino)-2-methylsulfanil-pyrimidin-5-yl]-methanol (1.49g) is dissolved in chloroform (25ml) and thereto is added manganese dioxide (5g), and the mixture is stirred at room temperature overnight. The manganese dioxide is removed by filtration and the resulting filtrate is concentrated under reduced pressure, and to the resulting residue are added hexane and isopropylether, and the mixture is filtered to give 4-(cyclopropylmethyl-propyl-aminol-2-methylsulfanil-pyrimidine-5-carbaldehyde (1.27g). MS (m/z): 266 [M+H]⁺
(4) 4-(Cyclopropylmethyl-propyl-amino)-2-methylsulfanil-pyrimidine-5-carbaldehyde (1.27g) is dissolved in 1,2-dichloroethane (15ml), and thereto are added 3,5-bis-(trifluoromethyl)benzylamine (1.51g), acetic acid (0.55ml) and triacetoxy-sodium borohydride (2.03g) and the mixture is stirred at room temperature for 1 hour. To reaction mixture is added a saturated aqueous sodium bicarbonate solution and the mixture is extracted with ethyl acetate. The organic layer is washed twice with a saturated brine, dried over magnesium sulfate, and concentrated under reduced pressure. The resulting residue is purified by silica gel column chromatography (hexane : ethyl acetate = 4:1) to give {5-[(3,5-bis-trifluoromethyl-benzylamino)-methyl]-2-methylsulfanil-pyrimidin-4-yl}-cyclopropylmethyl-propyl-amine (2.06g). MS (m/z): 493 [M+H]⁺
(5) {5-[(3,5-Bis-trifluoromethyl-benzylamino)-methyl]-2-methylsulfanil-pyrimidin-4-yl}-cyclopropylmethyl-propyl-amine (2.05g) is dissolved in toluene (15ml), and thereto are added 5-bromo-2-chloropyrimidine (3.22g) and N,N-diisopropylethylamine (4.34ml) and the mixtue is stirred at 110°C under nitrogen atmosphere for 3 days. To the reaction mixture is added a saturated brine, and the mixture is extracted with ethyl acetate and the organic layer is washed with a saturated brine, dried over magnesium sulfate, and concentrated under reduced pressure. To the resulting residue are added hexane and isopropylether, and the precipitated insoluble material is filtered and the filtrate is concentrated under reduced pressure, and the resulting residue is purified by silica gel column chromatography (hexane : ethyl acetate = 9:1→4:1) to give (5-{[(3,5-bis-trifluoromethyl-benzyl)-(5-bromo-pyrimidin-2-yl)-amino]-methyl}-2-methylsulfanil-pyrimidin-4-yl)-cyclopropylmethyl-propyl-amine (2.13g). MS (m/z): 651/649 [M+H]⁺
(6) (5-{[(3,5-Bis-trifluoromethyl-benzyl)-(5-bromo-pyrimidin-2-yl)-amino]-methyl}-2-methylsulfanil-pyrimidin-4-yl)-cyclopropylmethyl-propyl-amine (1.00g) is dissolved in chloroform (7ml) and thereto is added m-chloro perbenzoic acid (1.00g) and the mixture is stirred at room temperature for 3 hours. To the reaction solution is added a saturated aqueous sodium bicarbonate solution, and the mixture is extracted with chloroform and the organic layer is washed successively with a saturated aqueous sodium bicarbonate solution and brine, dried over magnesium sulfate, and concentrated under reduced pressure. The resulting residue is purified by silica gel column chromatography (hexane : ethyl acetate = 4:1→3:1) to give (5-{[(3,5-bis-trifluoromethyl-benzyl)-(5-bromo-pyrimidin-2-yl)-amino]-methyl}-2-methanesulfonyl-pyrimidin-4-yl)-cyclopropylmethyl-propyl-amine (858mg). MS (m/z): 683/681 [M+H]⁺
(7) (5-{[(3,5-Bis-trifluoromethyl-benzyl)-(5-bromo-pyrimidin-2-yl)-amino]-methyl}-2-methanesulfonyl-pyrimidin-4-yl)-cyclopropylmethyl-propyl-amine (500mg) is dissolved in methanol (5ml), and thereto is added a 28% sodium methoxide in methanol (231µl) and the mixture is stirred at room temperature overnight. The raction mixture solution is concentrated under reduced pressure and thereto is added a 10% aqueous citric acid solution, and the mixture is extracted with ethyl acetate and the organic layer is washed successively with a saturated aqueous sodium bicarbonate solution and a saturated brine, dried over magnesium sulfate, and concentrated under reduced pressure. The resulting residue is purified by silica gel column chromatography (hexane : ethyl acetate = 17:3→7:3) to give (5-{[(3,5-bis-trifluoromethyl-benzyl)-(5-bromo-pyrimidin-2-yl)-amino]-methyl}-2-methoxy-pyrimidin-4-yl)-cyclopropylmethyl-propyl-amine (425mg). MS (m/z): 635/633 [M+H]⁺
(8) (5-{[(3,5-Bis-trifluoromethyl-benzyl)-(5-bromo-pyrimidin-2-yl)-amino]-methyl}-2-methoxy-pyrimidin-4-yl)-cyclopropylmethyl-propyl-amine (422mg) is dissolved in a mixed solvent of dimethylsulfoxide (2ml) and methylene chloride (0.5ml), and the mixture is degassed, and thereto are added [1,1'-bis(diphenylphosphino)ferrocene]palladium(II) dichloride (27mg), potassium acetate (196mg) and bis(pinacolate)diboron (254mg), and the mixture is heated to 80°C under nitrogen flow and stirred for 45 minutes. The reaction solution is allowed cool to room temperature and thereto is added a saturated brine, and the mixture is extracted with ethyl acetate, and the organic layer is washed twice with a saturated brine, dried over magnesium sulfate, and concentrated under reduced pressure. The resulting residue is dissolved in tetrahydrofuran (2.5ml), and thereto is added dropwise a 30 % aqueous hydrogen peroxide solution (0.75ml) under ice-cooling and the mixture is stirred at the same temperature for 1 hour. To the reaction mixture is added dropwise a saturated aqueous sodium thiosulfate solution under ice-cooling and the mixture is extracted with ethyl acetate. The organic layer is washed with a saturated brine, dried over magnesium sulfate, and concentrated under reduced pressure. To the resulting residue is added isopropylether, and the precipitated solid is filtered to give 2-{(3,5-bis-trifluoromethyl-benzyl)-[4-(cyclopropylmethyl-propyl-amino)-2-methoxy-pyrimidin-5-ylmethyl]-amino}-pyrimidin-5-ol (328mg). MS (m/z): 571 [M+H]⁺
(9) 2-{(3,5-Bis-trifluoromethyl-benzyi)-[4-(cyclopropylmethyl-propylamino)-2-methoxy-pyrimidin-5-ylmethyl]-amino}-pyrimidin-5-ol (181mg) is dissolved in N,N-dimethylformamide (2ml), and thereto are added potassium carbonate (132mg) and ethyl 4-bromo-butyrate (160µl), and the the mixture is stirred at room temperature overnight. To the reaction mixture is added a saturated brine, and the mixture is extracted with ethyl acetate, and the organic layer is washed with a saturated brine, dried over magnesium sulfate, and concentrated under reduced pressure. The resulting residue is purified by silica gel column chromatography (hexane : ethyl acetate = 4:1→13:7) to give ethyl 4-(2-{(3,5-bis-trifluoromethylbenzyl)-[4-(cyclopropylmethyl-propyl-amino)-2-methoxy-pyrimidin-5-ylmethyl]-amino}-pyrimidin-5-yloxy)-butyrate (152mg). MS (m/z): 685 (M+H]⁺
(10) Ethyl 4-(2-{(3,5-bis-trifluoromethyl-benzyl)-[4-(cyclopropylmethyl-propyl-amino)-2-methoxy-pyrimidin-5-ylmethyl]-amino}-pyrimidin-5-yloxy)-butyrate (148mg) is dissolved in a mixed solvent of ethanol (1ml) and tetrahydrofuran (0.5ml), and thereto is added a 1M-aqueous sodium hydroxide solution (1ml), and the mixture is stirred at room temperature for 2 hours. To the reaction mixture is added a 10% aqueous citric acid solution and a saturated brine, and the mixture is extracted with ethyl acetate and the organic layer is washed with a saturated brine, dried over magnesium sulfate, and concentrated under reduced pressure. To the resulting residue is added ether and the precipitated solid is filtered to give 4-(2-{(3,5-bis-trifluoromethyl-benzyl)-[4-(cyclopropylmethyl-propyl-amino)-2-methoxy-pyrimidin-5-ylmethyl]-amino}-pyrimidin-5-yloxy)-butyric acid (122mg). MS (m/z): 657 [M+H]⁺

### Example 26

(1) (5-{[(3,5-Bis-trifluoromethyl-benzyl)-(5-bromo-pyrimidin-2-yl)-amino]-methyl}-2-methanesulfonyl-pyrimidin-4-yl)-cyclopropylmethyl-propyl-amine (450mg) is dissolved in ether (3ml) and thereto is added dropwise a 3M methylmagnesium chloride in tetrahydrofuran (286µl) under ice-cooling, and the mixture is stirred at the same temperature for 1 hour and a half. To the reaction mixture solution are added a saturated brine and ethyl acetate, and the insoluble materials are removed by filtration through Celite™, and the organic layer is washed with a saturated brine, dried over magnesium sulfate, and concentrated under reduced pressure. The resulting residue is purified by silica gel column chromatography (hexane : ethyl acetate = 4:1→3:1) to give (5-{[(3,5-bis-trifluoromethyl-benzyl)-(5-bromo-pyrimidin-2-yl)-amino]-methyl}-2-methyl-pyrimidin-4-yl)-cyclopropylmethyl-propyl-amine (346mg). MS (m/z): 619/617 [M+H]⁺
(2) (5-{[(3,5-Bis-trifluoromethyl-benzyl)-(5-bromo-pyrimidin-2-yl)-amino]-methyl}-2-methyl-pyrimidin-4-yl)-cyclopropylmethyl-propyl-amine is treated in similar manners to Examples 25(8)-(10) to give 4-(2-{(3,5-bis-trifluoromethyl-benzyl)-[4-(cyclopropylmethyl-propyl-amino)-2-methyl-pyrimidin-5-ylmethyl]-amino}-pyrimidin-5-yloxy)-butyric acid. MS (m/z): 641 [M+H]⁺

**Table 7**

| | | |
|---|---|---|
| 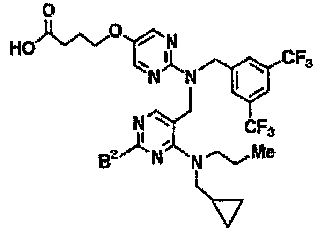 | | |

| Ex. No. | B²- | Physical properties, etc. |
|---|---|---|
| 25 | 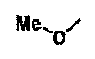 | MS (m/z): 657 [M+H]⁺ |
| 26 | 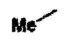 | MS (m/z): 641 [M+H]⁺ |

### Example 27

(6-Bromo-pyridin-2-yl)-methanol (1.0g) is dissolved in methylene chloride (10ml) and thereto is added thionyl chloride (427µl) under ice-cooling, and the mixture is stirred at the same temperature for 15 minutes. To the reaction solution is added a saturated aqueous sodium bicarbonate solution, and the mixture is separated, and the organic layer is washed with a saturated brine, dried over magnesium sulfate, and concentrated under reduced pressure. The resulting residue and (3,5-bis-trifluoromethyl-benzyl)-5-bromopyrimidin-2-yl)-amine (2.12g) is dissolved in N,N-dimethylformamide (20ml), and thereto is added sodium hydride (62%) (226mg) under ice-cooling, and mixture is stirred at room temperature overnight. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 1:0→19:1) to give (3,5-bis-trifluoromethyl-benzyl)-(5-bromo-pyrimidin-2-yl)-(6-bromo-pyridin-2-ylmethyl)-amine (2.7g). MS (m/z): 569/571 |M+H|⁺

**Table 8**

| Ex. No. | 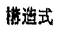 | Physical properties, etc. |
|---|---|---|
| 27 | 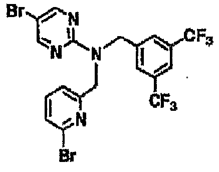 | MS (m/z): 569/571 [M+H]⁺ |

### Example 28

(1) Tert-butyl 7-formyl-2,3-dihydro-indol-1-carboxylate (2.0g) is dissolved in a mixed solvent of toluene (5ml) and ethanol (1ml), and thereto is added sodium borohydride (367mg), and the mixture is stirred at room temperature for 2 hours. To the reaction solution are added a saturated aqueous ammonium chloride solution and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 9:1→83:17) to give tert-butyl 7-hydroxymethyl-2,3-dihydro-indol-1-carboxylate (1.79g).
(2) Tert-butyl 7-hydroxymethyl-2,3-dihydro-indol-1-carboxylate (1.79g) is dissolved in toluene (30ml) and thereto is added thionyl chloride (630µl) under ice-cooling, and the mixture is stirred at room temperature for 1 hour. To the reaction solution are added water and ethyl acetate, and the mixture is separated, and the organic layer is washed with a saturated brine, dried over magnesium sulfate, and concentrated under reduced pressure. The resulting residue is dissolved in N,N-dimethylformamide (30ml) and thereto are added (3,5-bis-trifluoromethyl-benzyl)-(5-bromo-pyrimidin-2-yl)-amine (3.4g) and sodium tert-butoxide (830mg), and the mixture is stirred at room temperature for 3 days. To the reaction solution are added water and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 1:0→17:3) to give tert-butyl 7-{[(3,5-bis-trifluoromethyl-benzyl)-(5-bromo-pyrimidin-2-yl)-amino]-methyl}-2,3-dihydro-indol-1-carboxylate (3.5g). MS (m/z): 631/633 [M+H]⁺
(3) Tert-butyl 7-{[(3,5-bis-trifluoromethyl-benzyl)-(5-bromo-pyrimidin-2-yl)-amino]-methyl}-2,3-dihydro-indol-1-carboxylate (2.0g) is dissolved in toluene (30ml), and thereto are added tris(dibenzylideneacetone)-dipalladium (290mg), 2-(di-tert-butylphosphino)biphenyl (380mg), sodium tert-butoxide (457mg) and morpholine (415µl), and the mixture is stirred at room temperature under nitrogen atmosphere overnight, and to the reaction solution are added tris(dibenzylideneacetone)dipalladium (290mg), 2-(di-tert-butylphosphino)biphenyl (380mg) and sodium tert-butoxide (457mg), and the mixture is stirred for 3 hours. To the reaction solution are added a saturated aqueous sodium bicarbonate solution and ethyl acetate, and the mixture is separated, and the organic layer is washed with a saturated brine, dried over magnesium sulfate, and concentrated under reduced pressure. The resulting residue is purified by NH-silica gel column chromatography (hexane : ethyl acetate = 9:1→7:3) to give tert-butyl 7-{[(3,5-bis-trifluoromethyl-benzyl)-(5-morpholin-4-yl-pyrimidin-2-yl)-amino]-methyl}-2,3-dihydro-indol-1-carboxylate (1.53g). MS (m/z): 638 [M+H]⁺
(4) Tert-butyl 7-{[(3,5-bis-trifluoromethyl-benzyl)-(5-morpholin-4-yl-pyrimidin-2-yl)-amino]-methyl}-2,3-dihydro-indol-1-caboxylate (1.53g) is dissolved in a 4N-hydrochloric acid in dioxane (5ml), and the mixture is stirred at room temperature for 1 hour and 30 minutes. To the reaction solution are added a saturated aqueous sodium bicarbonate solution and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 4:1→3:2) to give (3,5-bis-trifluoromethyl-benzyl)-(2,3-dihydro-1H-indol-7-ylmethyl)-(5-morpholin-4-yl-pyrimidin-2-yl)-amine (1.15g). MS (m/z): 538 [M+H]⁺

### Example 29

(1) (3,5-Bis-trifluoromethyl-benzyl)-(2,3-dihydro-1H-indol-7-ylmethyl)-(5-morpholin-4-yl-pyrimidin-2-yl)-amine (450mg) is dissolved in N,N-dimethylformamide (10ml), and thereto is added sodium hydride (60%) (47mg) under ice-cooling and the mixture is stirred for 30 minutes, and thereto is added ethyl 7-bromo-heptanoate (275mg) and the mixture is stirred at room temperature overnight. To the reaction solution are added water and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 4:1→3:2) to give ethyl 7-(7-{[(3,5-bis-trifluoromethyl-benzyl)-(5-morpholin-4-yl-pyrimidin-2-yl)-amino]-methyl}-indol-1-yl)-heptanoate (95mg). MS (m/z): 692 [M+H]⁺
(2) Ethyl 7-(7-{[(3,5-bis-trifluoromethyl-benzyl)-(5-morpholin-4-yl-pyrimidin-2-yl)-amino]-methyl}-indol-1-yl)-heptanoate (90mg) is dissolved in ethanol (4ml), and thereto is added a 1N-aqueous sodium hydroxide solution (1ml) and the mixture is stirred at room temperature overnight. To the reaction solution are added a 1N-hydrochloric acid and ethyl acetate, and the mixture is separated, and the organic layer is washed with a saturated brine, dried over magnesium sulfate, and concentrated under reduced pressure. The resulting residue is purified by silica gel column chromatography (chloroform : methanol = 1:0→9:1) to give 7-(7-{[(3,5-bis-trifluoromethyl-benzyl)-(5-morpholin-4-yl-pyrimidin-2-yl)-amino]-methyl}-indol-1-yl)-heptanoic acid (76mg). MS (m/z): 664 [M+H]⁺

### Example 30

(1) (3,5-Bis-trifluoromethyl-benzyl)-(2,3-dihydro-1H-indol-7-ylmethyl)-(5-morpholin-4-yl-pyrimidin-2-yl)-amine (150mg) is dissolved in methylene chloride (5ml), and thereto are added ethyl 6-(chloroformyl)hexanoate (86mg) and triethylamine (34µl) under ice-cooling, and the mixture is stirred at room temperature for 2 hours. To the reaction solution are added a saturated aqueous ammonium chloride solution and chloroform, and the mixture is separated, and the organic layer is washed with a saturated brine, dried over magnesium sulfate, and concentrated under reduced pressure. The resulting residue is purified by silica gel column chromatography (hexane : ethyl acetate = 3:2→9:11) to give ethyl 7-(7-{[(3,5-bis-trifluoromethyl-benzyl)-(5-morpholin-4-yl-pyrimidin-2-yl)-amino]-methyl}-2,3-dihydro-indol-1-yl)-7-oxo-heptanoate (135mg). MS (m/z): 708 [M+H]⁺
(2) Ethyl 7-(7-{[(3,5-bis-trifluoromethyl-benzyl)-(5-morpholin-4-yl-pyrimidin-2-yl)-amino]-methyl}-2,3-dihydro-indol-1-yl)-7-oxo-heptanoate (130mg) is dissolved in ethanol (4ml), and thereto is added a 1N-aqueous sodium hydroxide solution (1ml), and the mixture is stirred at room temperature for 3 hours. To the reaction solution are added a 1N-hydrochloric acid and ethyl acetate, and the mixture is separated, and the organic layer is washed with a saturated brine, dried over magnesium sulfate, and concentrated under reduced pressure. The resulting residue is purified by silica gel column chromatography (chloroform : methanol - 1:0→9:1) to give 7-(7-{[(3,5-bis-trifluoromethyl-benzyl)-(5-morpholin-4-yl-pyrimidin-2-yl)-amino]-methyl}-2,3-dihydro-indol-1-yl)-7-oxo-heptanoic acid (116mg). MS (m/z): 680 [M+H]⁺

### Example 31

(1) (3,5-Bis-trifluoromethyl-benzyl)-(2,3-dihydro-1H-indol-7-ylmethyl)-(5-morpholin-4-yl-pyrimidin-2-yl)-amine (250mg) and triethylamine (77µl) are dissolved in methylene chloride (8ml), and thereto is added triphosgene (55mg) under ice-cooling, and the mixture is stirred at room temperature under nitrogen atmosphere for 1 hour. To the reaction solution are added a saturated aqueous sodium bicarbonate solution and chloroform, and the mixture is separated, and the organic layer is washed with a saturated brine, dried over magnesium sulfate, and concentrated under reduced pressure. The resulting residue is dissolved in tetrahydrofuran (5ml), and thereto are added methyl 3-hydroxy-2,2-dimethyl-propionate (360µl) and sodium hydride (60%) (108mg) and the mixture is stirred at room temperature overnight. To the reaction solution are added water and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate - 4:1→3:2) to give 7-{[(3,5-bis-trifluoromethyl-benzyl)-(5-morpholin-4-yl-pyrimidin-2-yl)-amino]-methyl}-2,3-dihydro-indole-1-carboxylic acid 2-methoxycarbonyl-2-methylpropylester (86mg). MS (m/z): 696 [M+H]⁺
(2) 7-{[(3,5-Bis-trifluoromethyl-benzyl)-(5-morpholin-4-yl-pyrimidin-2-yl)-amino]-methyl}-2,3-dihydro-indole-1-carboxylic acid 2-methoxycarbonyl-2-methyl-propylester (80mg) is dissolved in ethanol (4ml), and thereto is added a 1N-aqueous sodium, hydroxide solution (1ml), and the mxture is stirred at room temperature for 4 hours and 30 minutes. To the reaction solution are added a 1N-hydrochloric acid and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 3:2→2:3) to give 7-{[(3,5-bis-trifluoromethyl-benzyl)-(5-morpholin-4-yl-pyrimidin-2-yl)-amino]-methyl}-2,3-dihydro-indole-1-carboxylic acid 2-carboxy-2-methylpropylester (25mg). MS (m/z): 682 [M+H]⁺

### Example 32

The corresponding starting compound is treated in a similar manner to Example 5 to give the compound as listed in Table 9.

**Table 9**

| | | | |
|---|---|---|---|
| 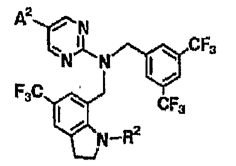 | | | |

| Ex. No. | A²- | -R² | Physical properties, etc. |
|---|---|---|---|
| 28 | 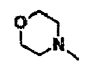 | 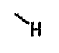 | MS (m/z): 538 [M+H]⁺ |
| 29 | 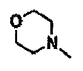 | 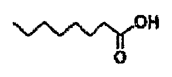 | MS (m/z): 664 [M+H]⁺ |
| 30 | 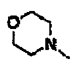 | 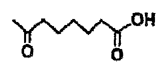 | MS (m/z): 680 [M+H]⁺ |
| 31 | 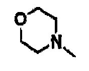 | 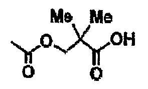 | MS (m/z): 682 [M+H]⁺ |
| 32 | 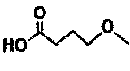 | 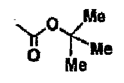 | MS (m/z): 655 [M+H]⁺ |

### Example 33

1-Benzyl-2-chloromethyl-1H-imidazole hydrochloride (1.0g) and (3,5-bis-trifluoromethyl-benzyl)-(5-bromopyrimidin-2-yl)-amine (1.65g) are dissolved in N,N-dimethylformamide (20ml), and thereto is added sodium hydride (60%) (318mg) under ice-cooling, and the mixture is stirred at room temperature for 4 hours. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 4:1→3:2) to give (1-benzyl-1H-imidazol-2-ylmethyl)-(3,5-bis-trifluoromethyl-benzyl)-(5-bromo-pyrimidin-2-yl)-amine (1.94g). MS (m/z): 570/572 [M+H]⁺

### Example 34

The corresponding starting compound is treated in a similar manner to Example 24 to give the compound as listed in Table 10.

**Table 10**

| | | |
|---|---|---|
| 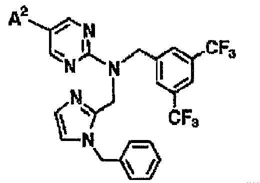 | | |

| Ex. No. | A²- | Physical properties, etc. |
|---|---|---|
| 33 | 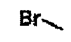 | MS (m/z): 570/572 [M+H]⁺ |
| 34 | 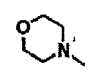 | MS (m/z): 577 [M+H]⁺ |

### Example 35

(1) 2-Chloro-3-nitro-pyridine (3.17g) and (aminomethyl)cyclopropane hydrochloride (2.15g) are dissolved in N,N-dimethylformamide (10ml), and thereto is added potassium carbonate (8.29g) and the mixture is stirred at room temperature for 6 hours. The reaction solution is cooled to room temperature, and thereto are added ethyl acetate and water, and the mixture is separated, and the organic layer is washed with a saturated brine, dried over magnesium sulfate, and concentrated under reduced pressure. The resulting residue is dissolved in a mixed solvent of tetrahydrofuran (50ml) and methanol (10ml), and thereto is added Raney nickel, and the mixture is stirred at room temperature under hydrogen atmosphere overnight. The catalyst is removed by filtration, and the filtrate is concentrated under reduced pressure. The resulting residue is dissolved in chloroform, dried over magnesium sulfate, and concentrated under reduced pressure. The resulting residue is dissolved in ethanol (100ml), and thereto is added 2-chloroacetimidic acid ethyl ester hydrochloride (31.6g) and the mixture is stirred at 60°C overnight. To the reaction solution are added ethyl acetate and a saturated aqueous sodium bicarbonate solution, and the mixture is separated, and the organic layer is washed with a saturated brine, dried over magnesium sulfate, and concentrated under reduced pressure. The resulting residue is purified by silica gel column chromatography (hexane : ethyl acetate = 2:1→1:1) to give 2-chloromethyl-3-cyclopropylmethyl-3H-imidazo[4,5-b]pyridine (2.92g).
(2) (3,5-Bis-trifluoromethyl-benzyl)-(5-bromo-pyrimidin-2-yl)-amine (361mg) is dissolved in N,N-dimethylformamide (2ml), and thereto is added sodium hydride (62%) (45mg) under ice-cooling, and the mixture is stirred at the same temperature for 30 minutes. Then, to the reaction solution are added 2-chloromethyl-3-cyclopropylmethyl-3H-imidazo[4,5-b]pyridine (200mg) and the mixture is stirred at room temperature for 15 minutes. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 9:1→19:6) to give (3,5-bis-trifluoromethyl-benzyl)-(5-bromo-pyrimidin-2-yl)-(1-cyclopropylmethyl-1H-imidazo[4,5-b]pyridin-2-ylmethyl)-amine (390mg). MS (m/z): 585/587 [M+H]⁺

### Example 36

The corresponding starting compound is treated in a similar manner to Example 4 to give the compound as listed in Table 11.

**Table 11**

| | | |
|---|---|---|
| 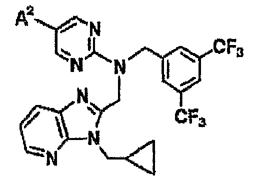 | | |

| Ex. No. | A²- | Physical properties, etc. |
|---|---|---|
| 35 | 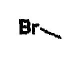 | MS (m/z): 585/587 [M+H]⁺ |
| 36 | 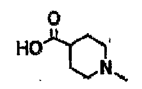 | MS (m/z): 634 [M+H]⁺ |

### Example 37

Ethyl 4-chloro-2-methylsulfanil-pyrimidine-5-carboxylate (200mg) is dissolved in N,N-dimethylformamide (3ml), and thereto are added 3,5-bis-(trifluoromethyl)benzylamine (313mg) and triethylamine (360µl), and the mixture is stirred at room temperature for 3 hours. To the reaction mixture is added water and the mixture is extracted with ethyl acetate, and the organic layer is washed with a saturated brine, dried over magnesium sulfate, and concentrated under reduced pressure. The resulting residue is purified by silica gel column chromatography (chloroform : hexane : ethyl acetate = 10:20:1) to give ethyl 4-(3,5-bis-trifluoromethyl-benzylamino)-2-methylsulfanil-pyrimidine-5-carboxylate (340mg). MS (m/z): 440 [M+H]⁺

**Table 12**

| Ex. No. | Structural formula | Physical properties, etc. |
|---|---|---|
| 37 | 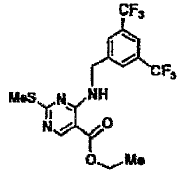 | as described above |

### Example 38

(1) To methanol (20ml) is added dropwise thionyl chloride (1.21ml) under ice-cooling, followed by an addition of 2-amino-3-(3,5-bis-trifluoromethyl-phenyl)-propionic acid (1.00g), and the mixture is stirred at room temperature for 2 days. The reaction mixture is concentrated under reduced pressure, and to the resulting residue are added ether and hexane, and the precipitated crystals are filtered to give 2-amino-3-(3,5-bis-trifluoromethyl-phenyl)-propionic acid methyl ester hydrochloride (1.11g). MS (m/z): 316 [M+H]⁺
(2) Ethyl 4-chloro-2-methylsulfanil-pyrimidine-5-carbaxylate (300mg) is dissolved in N,N-dimethylformamide (4ml), and thereto are added 2-amino-3-(3,5-bis-trifluoromethyl-phenyl)-propionic acid methyl ester hydrochloride (544mg) and triethylamine (540µl), and the mixture is stirred at room temperature for 3 hours. To the reaction mixture is added water and the mixture is extracted with ethyl acetate, and the organic layer is washed with water, dried over magnesium sulfate, and concentrated under reduced pressure. The resulting residue is purified by silica gel column chromatography (chloroform : hexane : ethyl acetate = 10:12:1) to give ethyl 4-[2-(3,5-bis-trifluoromethyl-phenyl)-1-methoxycarbonyl-ethylamino]-2-methylsulfanil-pyrimidine-5-carboxylate (542mg). MS (m/z): 512 [M+H]⁺

### Example 39

Ethyl 4-[2-(3,5-bis-trifluoromethyl-phenyl)-1-methoxycarbonyl-ethylamino]-2-methylsulfanil-pyrimidine-5-carboxylate (490mg) is dissolved in chloroform (6ml) and thereto is added m-chloroperbenzoic acid (248mg) under ice-cooling, and the mixture is stirred at the same temperatute for 10 minutes. The reaction mixture solution is purified by silica gel column chromatography (chloroform : acetone = 15:1→10:1) to give ethyl 4-[2-(3,5-bis-trifluoromethyl-phenyl)-1-methoxycarbonyl-ethylamino]-2-methanesulfinyl-pyrimidine-5-carboxylate (480mg). MS (m/z): 528 [M+H]⁺

### Example 40

Ethyl 4-[2-(3,5-bis-trifluoromethyl-phenyl)-1-methoxycarbonyl-ethylamino]-2-methanesulfinyl-pyrimidine-5-carboxylate (236mg) is dissolved in tetrahydrofuran (3ml), and thereto is added five drops of a 28% sodium methoxide in methanol by pasteur pipette, and the mixture is stirred at room temperature for 5 minutes. The reaction mixture solution is neutralized with a 10% aqueous citric acid solution, and thereto is added a saturated aqueous sodium bicarbonate solution and the mixture is extracted with ethyl acetate, and the organic layer is dried over magnesium sulfate, and concentrated under reduced pressure. The resulting residue is purified by thin-layer silica gel column chromatography (hexane : ethyl acetate = 4:1) to give ethyl 4-[2-(3,5-bis-trifluoromethyl-phenyl)-1-methoxycarbonyl-ethylamino]-2-methoxy-pyrimidine-5-carboxylate (55mg). MS (m/z): 496 [M+H]⁺

### Example 41

Ethyl 4-[2-(3,5-bis-trifluoromethyl-phenyl)-1-methoxycarbonyl-ethylamino]-2-methanesulfinyl-pyrimidine-5-carboxylate (80mg) is dissolved in tetrahydrofuran (2ml), and thereto is added a 50% aqueous dimethylamine solution (1ml), and the mixture is stirred at room temperature for 10 minutes. To the reaction mixture solution is added water, and the mixture is extracted with ethyl acetate, and the organic layer is washed with a saturated brine, dried over magnesium sulfate, and concentrated under reduced pressure. The resulting residue is purified by silica gel column chromatography (hexane : ethyl acetate = 4:1→13:7). The resulting residue is dissolved in chloroform and thereto is added a 4N-hydrochloric acid in dioxane and the reaction mixture is concentrated under reduced pressure. To the resulting residue is added ether and the precipitated crystals are filtered to give 4-[2-(3,5-bis-trifluoromethylphenyl)-1-methoxycarbonyl-ethylamino]-2-dimethylamino-pyrimidine-5-carboxylic acid ethyl ester hydrochloride (64mg). MS (m/z): 509 [M+H]⁺

### Example 42

The corresponding starting compound is treated in a similar manner to Example 41 to give the compound as listed in Table 13.

**Table 13**

| | | | |
|---|---|---|---|
| 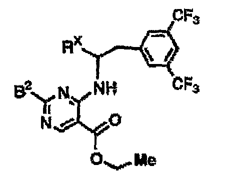 | | | |

| Ex. No. | B²- | R^{X}- | Physical properties, etc. |
|---|---|---|---|
| 38 | 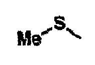 | 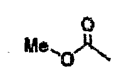 | MS (m/z): 512 [M+H]⁺ |
| 39 | 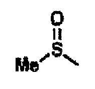 | 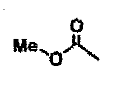 | MS (m/z): 528 [M+H]⁺ |
| 40 | 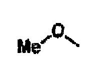 | 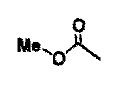 | MS (m/z) : 496 [M+H]⁺ |
| 41 | 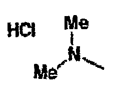 | 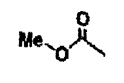 | MS (m/z): 509 [M+H]⁺ |
| 42 | 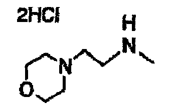 | 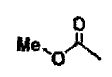 | MS (m/z): 594 [M+H]⁺ |

### Examples 43 to 49

The corresponding starting compounds are treated in similar manners to any of the above Examples to give the compounds as listed in Table 14.

**Table 14**

| Ex. No. | Structural formula | Physical properties, etc. |
|---|---|---|
| 43 | 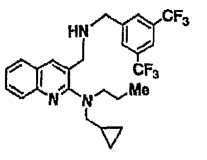 | MS (m/z): 496 [M+H]⁺ |
| 44 | 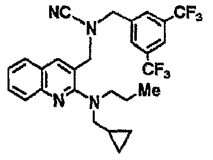 | MS (m/z): 521 [M+H]⁺ |
| 45 | 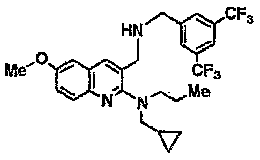 | MS (m/z): 526 [M+H]⁺ |
| 46 | 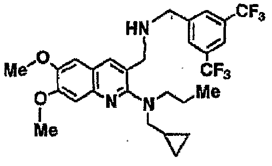 | MS (m/z): 556 [M+H]⁺ |
| 47 | 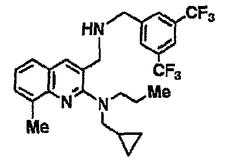 | MS (m/z): 510 [M+H]⁺ |
| 48 | 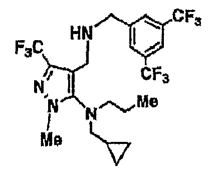 | MS (m/z): 517 [M+H]⁺ |
| 49 | 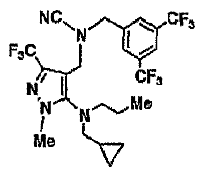 | MS (m/z) : 542 [M+H]⁺ |

### Example 50

(1) A mixture of 2-chloroquinoline-3-carboxaldehyde (1.0g), 2-benzyloxymethyl-5-tributylstannanyl-2H-tetrazol (5.0g) and dichlorobis(triphenylphosphine)palladium (II) (366mg) in N,N-dimethylformamide (5ml) is stirred at 100°C under nitrogen atmosphere overnight. The reaction solution is cooled to room temperature, and thereto is added a saturated aqueous potassium fluoride solution, and the mixture is stirred at room temperature for 30 minutes. The insoluble materials are removed by filtration through Celite™, and the filtrate is extracted with ethyl acetate. The organic layer is washed successively with water and a saturated brine, dried over magnesium sulfate, and concentrated under reduced pressure. The resulting residue is purified by silica gel column chromatography (hexane : ethyl acetate = 80:20→62:38) to give 3-(2-benzyloxymethyl-2H-tetrazol-5-yl)quinoline-3-carboxaldehyde (237mg). MS (m/z): 346 [M+H]⁺
(2) 3-(2-Benzyloxymethyl-2H-tetrazol-5-yl)quinoline-3-carboxaldehyde (237mg) is treated in similar manners to Examples 3(2)-(3) and 24 to give [2-(2-bezyloxymethyl-2H-tetrazol-5-yl)quinolin-3-ylmethyl]-(3,5-bis-trifluoromethylbenzyl)-(5-morpholin-4-yl-pyrimidin-2-yl)amine (59mg). MS (m/z): 736 [M+H]⁺

**Table 15**

| | | | |
|---|---|---|---|
| 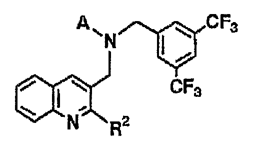 | | | |

| Ex. No. | A- | -R² | Physical properties, etc. |
|---|---|---|---|
| 50 | 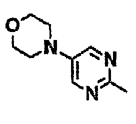 | 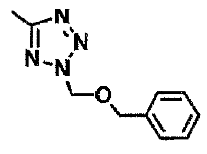 | MS (m/z): 736 [M+H]⁺ |

### Example 51

(1) 2-Hydroxymethyl-6-methylpyridin-3-ol (3.0g) is dissolved in N,N-dimethylformamide (40ml) and thereto are added benzyl bromide (3ml) and potassium carbonate (3.58g), and the mixture is stirred at room temperature overnight. To the reaction solution are added ethyl acetate and water, and the mixture is separated, and the organic layer is washed with a saturated brine, dried over magnesium sulfate, and concentrated under reduced pressure. The residue is purified by silica gel column chromatography (hexane : ethyl acetate = 19:1→4:1) to give (3-benzyloxy-6-methyl-pyridin-2-yl)-methanol (4.45g). MS (m/z): 230 [M+H]⁺
(2) (3-Benzyloxy-6-methyl-pyridin-2-yl)-methanol (4.45g) is dissolved in methylene chloride (15ml), and thereto are added carbon tetrabromide (7.08g) and triphenylphosphine (5.34g) under ice-cooling, and the mixture is stirred at 0°C for 1 hour. The reaction solution is concentrated under reduced pressure and the resulting residue is purified by silica gel column chromatography (hexane : ethyl acetate = 19:1→4:1) to give 3-benzyloxy-2-bromomethyl-6-methyl-pyridine (4.85g). MS (m/z): 292/294 [M+H]⁺
(3) (3,5-Bis-trifluoromethyl-benzyl)-(5-bromo-pyrimidin-2-yl)-amine (6mg) is dissolved in N,N-dimethylformamide (50ml), and thereto is added sodium hydride (60%) (720mg) under ice-cooling, and the mixture is stirred at 0°C for 15 minutes. To the reaction solution is added 3-benzyloxy-2-bromomethyl-6-methyl-pyridine (4.85g) and the mixture is stirred at room temperature for 1.5 hours. To the reaction solution are added water and ethyl acetate, and the mixture is separated, and the organic layer is washed with a saturated brine, dried over magnesium sulfate, and concentrated under reduced pressure. The resulting residue is purified by NH-silica gel column chromatography (hexane : ethyl acetate = 19:1→17:3) to give (3-benzyloxy-6-methyl-pyridin-2-ylmethyl)-(3,5-bis-trifluoromethylbenzyl)-(5-bromo-pyrimidin-2-yl)-amine (8.92g). 611/613 [M+H]⁺
(4) (3-Benzyloxy-6-methyl-pyridin-2-ylmethyl)-(3,5-bis-trifluoromethyl-benzyl)-(5-bromo-pyrimidin-2-yl)-amine (1g) is dissolved in toluene (15ml), and thereto are added tris(dibenzylideneacetone)dipalladium (150mg), sodium tert-butoxide (236mg), 2-(di-tert-butylphosphino)biphenyl (196mg), and ethyl piperidine-4-carboxylate (380µl), and the mixture is stirred at room temperature under nitrogen flow for 3 days. To the reaction solution are added a saturated sodium bicarbonate and ethyl acetate, and the mixture is separated, and the organic layer is washed with a saturated brine, dried over magnesium sulfate, and concentrated under reduced pressure. The residue is purified by silica gel column chromatography (hexane : ethyl acetate = 19:1→4:1→1:1) to give ethyl 1-{2-[(3-benzyloxy-6-methyl-pyridin-2-ylmethyl)-(3,5-bis-trifluoromethyl-benzyl)-amino]-pyrimidin-5-yl}-piperidine-4-carboxylate (234mg). 688 [M+H]⁺
(5) Ethyl 1-{2-[(3-benzyloxy-6-methyl-pyridin-2-ylmethyl)-(3,5-bis-trifluoromethyl-benzyl)-amino]-pyrimidin-5-yl}-piperidine-4-carboxylate (230mg) is dissolved in ethanol (5ml), and thereto is added 10% palladium-carbon (50mg) and the mixture is stirred at room temperature under hydrogen atmosphere for 5 hours. The catalyst is removed by filtration, and the filtrate is concentrated under reduced pressure. The resulting residue is purified by silica gel column chromatography (hexane : ethyl acetate = 19:1→4:1) to give ethyl 1-{2-[(3,5-bis-trifluoromethyl-benzyl)-(3-hydroxy-6-methyl-pyridin-2-ylmethyl)-amino]-pyrimidin-5-yl}-piperidine-4-carboxylate (152mg). MS (m/z): 598 [M+H]⁺
(6) Ethyl 1-{2-[(3,5-bis-trifluoromethyl-benzyl)-(3-hydroxy-6-methyl-pyridin-2-ylmethyl)-amino]-pyrimidin-5-yl}-piperidine-4-carboxylate (152mg) and pyridine (31µl) are dissolved in methylene chloride (4ml), and thereto is added trifluoromethanesulfonic acid anhydride (65µl) under ice-cooling, and the mixture is stirred at 0°C under nitrogen atmosphere for 1 hour, and to the reaction solution is added trifluoromethanesulfonic acid anhydride (65µl) and the mixture is cooled to the room temperature and stirred for 1 hour. To the reaction solution are added a saturated aqueous sodium bicarbonate solution and chloroform, and the mixture is separated, and the organic layer is washed with a saturated brine, dried over magnesium sulfate, and concentrated under reduced pressure. The residue is purified by silica gel column chromatography (hexane : ethyl acetate = 19:1→4:1→3:2) to give ethyl 1{2-[(3,5-bis-trifluoromethylbenzyl)-(6-methyl-3-trifluoromethanesulfonyloxy-pyridin-2-ylmethyl)-amino]-pyrimidin-5-yl}-piperidine-4-carboxylate (147mg). MS (m/z): 730 [M+H]⁺
(7) Ethyl 1-{2-[(3,5-bis-trifluoromethyl-benzyl)-(6-methyl-3-trifluoromethanesulfonyloxy-pyridin-2-ylmethyl)-amino]-pyrimidin-5-yl}-piperidine-4-carboxylate (143mg) is dissolved in 1,4-dioxane (4ml), and thereto are added 5-isopropyl-2-methoxy-phenyl boric acid (57mg), [1,1'-bis(diphenylphosphino)ferrocene]dichloropalladium dichloromethane complex (16mg) and cesium carbonate (96mg), and the mixture is stirred at 80°C under nitrogen atmosphere for 2.5 hours. The reaction solution is cooled to room temperature, and thereto are added water and ethyl acetate, and the mixture is separated. The organic layer is washed with a saturated brine, dried over magnesium sulfate, and concentrated under reduced pressure. The resulting residue is purified by silica gel column chromatography (hexane : ethyl acetate = 19:1→4:1) to give ethyl 1-(2-{(3,5-bis-trifluoromethyl-benzyl)-[3-(5-isopropyl-2-methoxy-phenyl)-6-methyl-pyridin-2-ylmethyl]-amino}-pyrimidin-5-yl)-piperidine-4-carboxylate (97mg). MS (m/z): 730 [M+H]⁺
(8) Ethyl 1-(2-{(3,5-bis-trifluoromethyl-benzyl)-[3-(5-isopropyl-2-methoxy-phenyl)-6-methyl-pyridin-2-ylmethyl]-amino}-pyrimidin-5-yl)-piperidine-4-carboxylate (92mg) is dissolved in ethanol (4ml), and thereto is added a 1N-aqueous sodium hydroxide solution (1ml) and the mixture is stirred at room temperature for 1 day. To the reaction solution are added a 1N-hydrochloric acid and ethyl acetate, and the mixture is separated, and the organic layer is washed with a saturated brine, dried over magnesium sulfate, and concentrated under reduced pressure. The resulting residue is purified by silica gel column chromatography (chloroform : methanol = 1:0→9:1) to give 1-(2-{(3,5-bis-trifluoromethyl-benzyl)-[3-(5-isopropyl-2-methoxy-phenyl)-6-methyl-pyridin-2-ylmethyl]-amino}-pyrimidin-5-yl)-piperidine-4-carboxylic acid (73mg). MS (m/z): 702 [M+H]⁺

### Example 52

(1) (3-Benzyloxy-6-methylpyridin-2-ylmethyl)-(3,5-bis-trifluoromethylbenzyl)-(5-bromo-pyrimidin-2 yl)-amine (5g) is treated in similar manners to Examples 5(1)-(2) to give ethyl 4-{2-[(3-benzyloxy-6-methyl-pyridin-2-ylmethyl)-(3,5-bis-trifluoromethyl-benzyl)-amino]-pyrimidin-5-yloxy}-butyrate (1.69g). MS (m/z): 663 [M+H]⁺
(2) Ethyl 4-{2-[(3-benzyloxy-6-methyl-pyridin-2-ylmethyl)-(3,5-bis-trifluoromethyl-benzyl)-amino]-pyrimidin-5-yloxy}-butyrate (1.67g) is treated in similar manners to Examples 51(5)-(6) to give ethyl 4-{2-[(3,5-bis-trifluoromethyl-benzyl)-(6-methyl-3-trifluoromethanesulfonyloxy-pyridin-2-ylmethyl)-amino]-pyrimidin-5-yloxy}-butyrate (1.4g). MS (m/z): 705 [M+H]⁺
(3) Ethyl 4-{2-[(3,5-bis--trifluoromethyl-benzyl)-(6-methyl-3-trifluoromethanesulfonyloxy-pyridin-2-ylmethyl)-amino]-pyrimidin-5-yloxy)-butyrate (313mg) is treated in similar manners to Examples 51(7)-(8) to give 4-(2-{(3,5-bis-trifluoromethyl-benzyl)-[3-(5-isopropyl-2-methoxyphenyl)-6-methyl-pyridin-2-ylmethyl]-amino}-pyrimidin-5-yloxy)-butyrate (74mg). MS (m/z): 677 [M+H]⁺

**Table 16**

| | | |
|---|---|---|
| 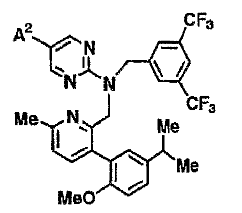 | | |

| Ex. No. | A²- | Physical properties, etc. |
|---|---|---|
| 51 | 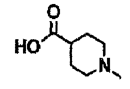 | MS (m/z): 702 [M+H]⁺ |
| 52 | 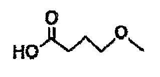 | MS (m/z): 677 [M+H]⁺ |

### Example 53

(1) (3,5-Bis-trifluoromethyl-benzyl)-(5-bromopyrimidin-2-yl)-[2-(5-isopropyl-2-methoxy-phenyl)-8-methyl-quinolin-3-ylmethyl]amine (200mg) is dissolved in dioxane (1.5ml), and thereto are added tris(dibenzylideneacetone)dipalladium (39mg), tetrafluoroboric acid tri-tert-butylphosphonium (25mg), methyl acrylate (51µl) and N,N-diisopropylethylamine (74µl), and the mixture is degassed under reduced pressure and stirred at 50°C under nitrogen flow for 5 hours. The reaction solution is allowed cool to room temperature, and thereto is added a saturated brine, and the mixture is extracted with ethyl acetate and the organic layer is washed with a saturated brine, dried over magnesium sulfate, and concentrated under reduced pressure. The resulting residue is purified by silica gel column chromatography (hexane : ethyl acetate = 91:9-4:1) to give methyl 3-(2-{(3,5-bis-trifluoromethyl-benzyl)-[2-(5-isopropyl-2-methoxy-phenyl)-8-methyl-quinolin-3-ylmethyl]amino}-pyrimidin-5-yl)-acrylate (152mg). MS (m/z): 709 [M+H]⁺
(2) Methyl 3-(2-{(3,5-bis-trifluoromethyl-benzyl)-[2-(5-isopropyl-2-methoxy-phenyl)-8-methyl-quinolin-3-ylmethyl]amino}-pyrimidin-5-yl)-acrylate (149mg) is dissolved in a mixed solvent of methanol (0.5ml) and tetrahydrofuran (2ml), and thereto is added a 1M-aqueous sodium hydroxide solution (0.5ml) and the mixture is stirred at room temperature for 4 hours. The reaction mixture is made acidic with a 10% aqueous citric acid solution and the mixture is extracted with ethyl acetate. The organic layer is washed with a saturated brine, dried over magnesium sulfate, and concentrated under reduced pressure to give 3-(2-{(3,5-bis-trifluoromethyl- , benzyl)-[2-(5-isopropyl-2-methoxy-phenyl)-8-methyl-quinolin-3-ylmethyl]amino}-pyrimidin-5-yl)-acrylic acid (151mg). MS (m/z): 695 [M+H]⁺

### Examples 54 to 56

The corresponding starting compounds are treated in a similar manner to Example 4 to give the desired compounds.

### Example 57

(1) (3-{[(3,5-Bis-trifluoromethyl-benzyl)-(5-bromo-pyrimidin-2-yl)-amino]-methyl}-8-methyl-quinolin-2-yl)-cyclopropylmethyl-propyl-amine (300mg) is dissolved in toluene (5ml), and thereto are added tris(dibenzylideneacetone)dipalladium (41mg), sodium tert-butoxide (65mg), 2-(di-tert-butylphosphino)biphenyl (54mg) and tert-butyl 3-methylamino-propionate (107mg) and the mixture is stirred at room temperature under nitrogen flow overnight. To the reaction solution are added water and diethyl ether, and the mixture is separated, and the organic layer is dried over magnesium sulfate, and concentrated under reduced pressure. The resulting residue is purified by NH-silica gel column chromatography (hexane : ethyl acetate = 1:0→85:15) to give tert-butyl 3-[(2-{(3,5-bis-trifluoromethyl-benzyl)-[2-(cyclopropylmethyl-propyl-amino)-8-methyl-quinolin-3-ylmethyl]-amino}-pyrimidin-5-yl)-methyl-amino]-propionate (121mg). MS (m/z): 745 [M+H]⁺
(2) Tert-butyl 3-[(2-{(3,5-bis-trifluoromethyl-benzyl)-[2-(cyclopropylmethyl-propyl-amino)-8-methyl-quinolin-3-ylmethyl]-amino}-pyrimidin-5-yl)-methyl-amino]-propionate (115mg) is dissolved in a 4N-hydrochloric acid in ethyl acetate (3ml) and the mixture is stirred at room temperature for 3 hours. To the reaction solution are added water and a saturated aqueous sodium bicarbonate solution to make the pH of aqueous layer to be about 4, and the mixture is extracted with diethyl ether. The organic layer is washed with a saturated brine, dried over magnesium sulfate, and concentrated under reduced pressure. The resulting residue is purified by silica gel column chromatography (chloroform : methanol = 49:1→93:7) to give 3-[(2-{(3,5-bis-trifluoromethyl-benzyl)-[2-(cyclopropylmethyl-propyl-amino)-B-methyl-quinolin-3-ylmethyl]-amino}-pyrimidin-5-yl)-methyl-amino]-propionic acid (86mg). MS (m/z): 689 [M+H]⁺

### Example 58

(1) 2-{(3,5-Bis-trifluommethyl-benzyl)-[2-(5-isopropyl-2-methoxyphenyl)-8-methyl-quinolin-3-ylmethyl]amino}-pyrimidin-5-ol (88mg) is dissolved in tetrahydrofuran (1ml), and thereto are added 2-(methylthio)ethanol (25mg), and triphenylphosphine (72mg), followed by an addition of a 40% diethyl azodicarboxylate/toluene (119µl) under water-cooling, and the the mixture is stirred at room temperature for 2 hours. To the reaction mixture is added a saturated brine, and the mixture is extracted with ethyl acetate. The organic layer is washed with a saturated brine, dried over magnesium sulfate, and concentrated under reduced pressure. The resulting residue is purified by silica gel column chromatography (hexane : ethyl acetate = 1:0→4:1) to give (3,5-bis-trifluoromethyl-benzyl)-[2-(5-isopropyl-2-methoxy-phenyl)-8-methyl-quinolin-3-ylmethyl]-[5-(2-methylsulfanil-ethoxy)-pyrimidin-2-yl]-amine (117mg). MS (m/z): 715 [M+H]⁺
(2) (3,5-Bis-trifluoromethyl-benzyl)-[2-(5-isopropyl-2-methoxy-phenyl)-8-methyl-quinolin-3-ylmethyl]-[5-(2-methylsulfanil-ethoxy)-pyrimidin-2-yl]-amine (114mg) is dissolved in chloroform (2ml), and thereto is added m-chloroperbenzoic acid (44mg) and the mixture is stirred at room temperature for 2 hours. To the reaction solution are added a saturated aqueous sodium bicarbonate solution and the mixture is extracted with ethyl acetate and the organic layer is washed successively with a saturated aqueous sodium bicarbonate solution and a saturated brine, dried over magnesium sulfate, and concentrated under reduced pressure. The resulting residue is purified by silica gel column chromatography (hexane : ethyl acetate= 1:1, and chloroform : methanol = 19:1) to give (3,5-bis-trifluoromethyl-benzyl)-[2-(5-isopropyl-2-methoxy-phenyl)-8-methyl-quinolin-3-ylmethyl]-[5-(2-methanesulfonyl-ethoxy)-pyrimidin-2-yl]-amine (63mg). MS (m/z): 747 [M+H]⁺

**Table 17**

| | | | |
|---|---|---|---|
| 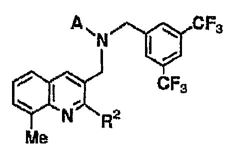 | | | |

| Ex. No. | A- | -R² | Physical properties, etc. |
|---|---|---|---|
| 53 | 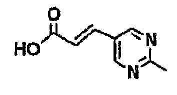 | 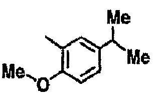 | MS (m/z): 695 [M+H]⁺ |
| 54 | 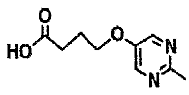 | 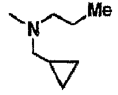 | MS (m/z): 690 [M+H]⁺ |
| 55 | 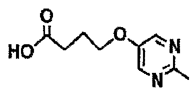 | 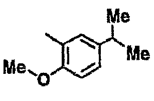 | MS (m/z): 727 [M+H]⁺ |
| 56 | 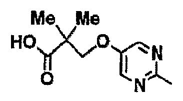 | 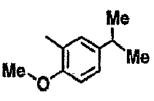 | MS (m/z): 741 [M+H]⁺ |
| 57 | 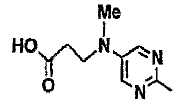 | 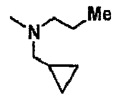 | MS (m/z): 689 [M+H]⁺ |
| 58 | 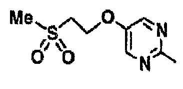 | 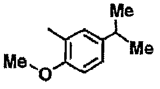 | MS (m/z): 747 [M+H]⁺ |

### Example 59

(1) Benzyl alcohol (22.7ml) is dissolved in tetrahydrofuran (180ml), and thereto is added stepwise sodium hydride (60%) (8.76g) under ice-cooling, and the mixture is stirred at room temperature for 20 minutes. To this reaction mixture is added a solution of 2-chloro-8-methyl-quinoline-3-carbaldehyde (15.00g) dissolved in tetrahydrofuran (180ml), and the mixture is stirred at 50°C for 5 hours. To the reaction mixture is added a 10% aqueous citric acid solution and a saturated brine and the mixture is extracted with ethyl acetate. The organic layer is washed with a saturated brine, dried over magnesium sulfate, and concentrated under reduced pressure. To the resulting residue is added ether, and the insoluble materials are removed by filteration and the filtrate is concentrated under reduced pressure. The resulting residue is purified by silica gel column chromatography (hexane : ethyl acetate = 19:1→3:1). To the resulting residue is added isopropylether and the precipitated crystals are filtered to give (2-benzyloxy-8-methyl-quinolin-3-yl)-methanol (2.62g). MS (m/z): 280 [M+H]⁺
(2) (2-Benzyloxy-8-methyl-quinolin-3-yl)-methanol (3.59g) is treated in similar manners to Example 51(2)-(3) to give (2-benzyloxy-8-methyl-quinolin-3-ylmethyl)-(3,5-bis-trifluoromethyl-benzyl)-(5-bromo-pyrimidin-2-yl)-amine (5.39g). MS (m/z): 663/661 [M+H]⁺
(3) (2-Benzoyloxy-8-methyl-quinolin-3-ylmethyl)-(3,5-bis-trifluoromethylbenzyl)-(5-bromo-pyrimidin-2-yl)-amine (4.73g) is treated with the corresponding starting compound in a similar manner to Example 52 to give 4-(2-{(3,5-bis-trifluoromethyl-benzyl)-[2-(2,5-dimethoxy-phenyl)-8-methyl-quinolin-3-ylmethyl]-amino}-pyrimidin-5-yloxy)-butyric acid (66mg). MS (m/z): 713 [M-Na]⁻

### Example 60

The corresponding starting compound is treated in a similar manner to Example 59 to give the desired compound.

### Example 61

(1) 2-[(2-Benzyloxy-8-methyl-quinolin-3-ylmethyl)-(3,5-bis-trifluoromethyl-benzyl)-amino]-pyrimidin-5-ol (700mg) is treated in a similar manner to Example 58 to give (2-benzyloxy-8-methyl-quinolin-3-ylmethyl)-(3,5-bis-trifluoromethyl-benzyl)-[5-(2-methanesulfonyl-ethoxy)-pyrimidin-2-yl]-amine (607mg). MS (m/z): 705 [M+H]⁺
(2) (2-Benzyloxy-8-methyl-quinolin-3-ylmethyl)-(3,5-bis-trifluoromethylbenzyl)-[5-(2-methanesulfonyl-ethoxy)-pyrimidin-2-yl]-amine (546mg) is dissolved in methylene chloride (5ml), and thereto is added a 4N-hydrochloric acid in dioxane (5ml), and the mixture is stirred at room temperature for 1 hour. The reaction solution is concentrated under reduced pressure, and the residue is neutralized with a saturated aqueous sodium bicarbonate solution, and the mixture is made weakly acidic with a 10% aqueous citric acid solution and extracted with a mixed solution of ethyl acetate and a small amount of methylene chloride. The organic layer is washed with a saturated brine, dried over magnesium sulfate, and concentrated under reduced pressure to give 3-({(3,5-bis-trifluoromethylbenzyl)-[5-(2-methanesulfonyl-ethoxy)-pyrimidin-2-yl]-amino}-methyl)-8-methyl-quinolin-2-ol (530mg). MS (m/z): 615 [M+H]⁺
(3) 3-({(3,5-Bis-trifluoromethyl-benzyl)-[5-(2-methanesulfonyl-ethoxy)-pyrimidin-2-yl]-amino}-methyl)-8-methyl-quinolin-2-ol (462mg) is dissolved in methylene chloride (8ml), and thereto is added triethylamine (630µl), followed by an addition stepwise of trifluoromethanesulfonic acid anhydride (380µl) under water-cooling, and the mixture is stirred at room temperature for 2 hours. To the reaction mixture is added a saturated brine, and the mixture is extracted with ethyl acetate and the organic layer is washed with a saturated brine, dried over magnesium sulfate, and concentrated under reduced pressure. The resulting residue is purified by silica gel column chromatography (hexane : ethyl acetate = 19:1→13:7) to give trifluoromethanesulfonic acid 3-({(3,5-bis-trifluoromethyl-benzyl)-[5-(2-methanesulfonyl-ethoxy)-pyrimidin-2-yl]-amino}-methyl)-8-methyl-quinolin-2-ylester (427mg). MS (m/z): 747 [M+H]⁺
(4) Trifluoromethanesulfonic acid 3-({(3,5-bis-trifluoromethyl-benzyl)-[5-(2-methanesulfonyl-ethoxy)-pyrimidin-2-yl]-amino}-methyl)-8-methyl-quinolin-2-ylester (100mg) is treated with the corresponding starting compound in a similar manner to Example 51(7) to give (3,5-bis-trifluoromethyl-benzyl)-[2-(2,4-dimethoxy-pyrimidin-5-yl)-8-methyl-quinolin-3-ylmethyl]-[5-(2-methanesulfonyl-ethoxy)-pyrimidin-2-yl]-amine
(45mg). MS (m/z): 737 [M+H]⁺

### Example 62

(1) (3,5-Bis-trifluoromethyl-benzyl)-[2-(5-isopropyl-2-methoxy-phenyl)-8-methyl-quinolin-3-ylmethyl]-(1H-tetrazol-5-yl)-amine (which is obtained by treating the corresponding starting compound in a similar manner to Example 6) (100mg) is dissolved in N,N-dimethylformamide (10ml), and thereto are added potassium carbonate (67mg) and ethyl 4-bromo-butyrate (70µl) and the mixture is stirred at 60°C for 3 hours. The reaction solution is allowed cool to room temperature, and thereto are added a 10% aqueous citric acid solution and a saturated brine, and the mixture is extracted with ethyl acetate. The organic layer is washed with a saturated brine, dried over magnesium sulfate, and concentrated under reduced pressure. The resulting residue is purified by silica gel column chromatography (hexane : ethyl acetate = 99:1→17:3) to give ethyl 4-(5-{(3,5-bis-trifluoromethyl-benzyl)-[2-(5-isopropyl-2-methoxyphenyl)-8-methyl-quinolin-3-ylmethyl]-amino}-tetrazol-2-yl)-butyrate (111mg). MS (m/z): 729 [M+H]⁺
(2) Ethyl 4-(5-{(3,5-bis-trifluoromethyl-benzyl)-[2-(5-isopropyl-2-methoxy-phenyl)-8-methyl-quinolin-3-ylmethyl]-amino}-tetrazol-2-yl)-butyrate (96mg) is treated in a similar manner to Example 1(3) to give 4-(5-{(3,5-bis-trifluoromethyl-benzyl)-[2-(5-isopropyl-2-methoxy-phenyl)-8-methyl-quinolin-3-ylmethyl]-amino}-tetrazol-2-yl)-butyric acid (99mg). MS (m/z): 701 [M+H]⁺

**Table 17**

| | | | |
|---|---|---|---|
| 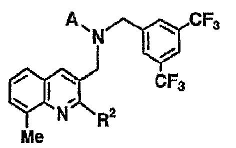 | | | |

| Ex. No. | A- | -R² | Physical properties, etc. |
|---|---|---|---|
| 59 | 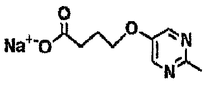 | 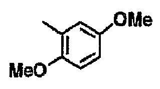 | MS (m/z): 713 [M-Na]⁻ |
| 60 | 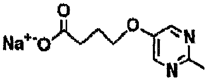 | 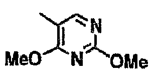 | MS (m/z): 715 [M-Na]⁻ |
| 61 | 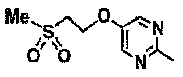 | 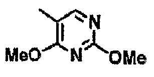 | MS (m/z): 735 [M+H]⁺ |
| 62 | 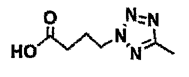 | 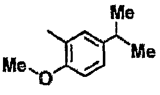 | MS (m/z): 701 [M+H]⁺ |

### Example 63

(1) To tetrahydrofuran (30ml) is added a 1.6M n-butyl lithium/hexane (10.9ml), followed by an addition dropwise of diisopropylamine (2.45ml) under nitrogen atmosphere at -78°C, and the mixture is stirred at 0°C for 1 hour. To the raction solution is added dropwise a solution of 2,8-dichloroquinoline (3.15g) dissolved in tetrahydrofuran (10ml) at -78°C, and the mixture is stirred at the same temperature for 2 hours. To the reaction solution are added dropwise ethyl formate (5.1ml) at -78°C, and the mixture is stirred for 1 hour, and thereto are added an aqueous ammonium chloride solution and the mixture is extracted with ethyl acetate. The organic layer is washed with a saturated brine, dried over magnesium sulfate, and concentrated under reduced pressure, and to the resulting residue are added ethyl acetate and ether and the precipitated crystals are filtered to give 2,8-dichloro-quinoline-3-carbaldehyde (1.21g). MS (m/z): 228/226 [M+H]⁺
(2) 2,8-Dichloro-quinoline-3-carbaldehyde (1.18g) is treated in similar manners to Examples 9 and 4 to give 1-(2-{(3,5-bis-trifluoromethyl-benzy)-[8-chloro-2-(cyclopropylmethyl-propyl-amino)-quinolin-3-ylmethyl]-amino}-pyrimidin-5-yl)-piperazine-4-carboxylic acid (87mg). MS (m/z): 737/735 [M+H]⁺

**Table 18**

| | | | |
|---|---|---|---|
| 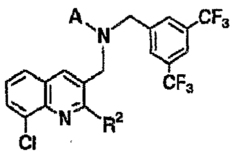 | | | |

| Ex. No. | A- | -R² | Physical properties, etc. |
|---|---|---|---|
| 63 | 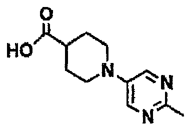 | 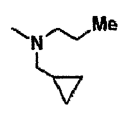 | MS (m/z): 735/737 [M+H]⁺ |

### Example 64

(3,5-Bis-rtrifluoromethyl-benzyl)-(5-bromo-pyrimidin-2-yl)-quinolin-8-ylmethyl-amine (300mg) (which is obtained by treating the corresponding starting compound in similar manners to Examples 25(4)-(5)) is treated in a similar manner to Example 4 to give 1-{2-[(3,5-bis-trifluoromethylbenzyl)-quinolin-8-ylmethyl-amino]-pyrimidin-5-yl}-piperidine-4-carboxylic acid (16mg). MS (m/z): 590 [M+H]⁺

**Table 19**

| Ex. No. | Structural formula | Physical properties, etc. |
|---|---|---|
| 64 | 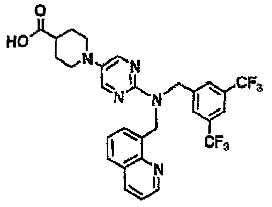 | MS (m/z): 590 [M+H]⁺ |

### Example 65

(1) [2-(5-Isopropyl-2-methoxy-phenyl)-8-methyl-quinolin-3-yl]-methanol (which is obtained by treating the corresponding starting compound in similar manners to Examples 3(1)-(2)) (2.06g) is dissolved in methylene chloride (10ml), and thereto is added dropwise thionyl chloride (545µl) at 0°C. The mixture is stirred at room temperature for 15 minutes and concentrated under reduced pressure, and the resulting residue is dissolved in methylene chloride and concentrated under reduced pressure again to give 3-chloromethyl-2-(5-isopropyl-2-methoxy-phenyl)-8-methylquinoline (2.39g). MS (m/z): 342/340 [M+H]⁺
(2) To 3-chloromethyl-2-(5-isopropyl-2-methoxy-phenyl)-8-methylquinoline (2.39g) are added N,N-dimethylformamide (12ml), phthalimide potassium salt (2.54g) and potassium carbonate (2.58g), and the mixtrue is stirred at 80°C for 4 hours. The reaction solution is allowed cool to room temperature, and thereto are added a saturated brine and a 10% aqueous citric acid solution, and the mixture is extracted with ethyl acetate. The organic layer is washed twice with a saturated brine, dried over magnesium sulfate, and concentrated under reduced pressure. To the resulting residue is added ether and the mixture is filtered and the filtrate is concentrated under reduced pressure. The resulting residue is purified by silica gel column chromatography (hexane : ethyl acetate = 19:1→3:1) to give 2-[2-(5-isopropyl-2-methoxy-phenyl)-8-methyl-quinolin-3-ylmethyl]-isoindol-1,3-dione (2.35g). MS (m/z): 451 [M+H]⁺
(3) 2-[2-(5-isopropyl-2-methoxy-phenyl)-8-methyl-quinolin-3-ylmethyl]-isoindol-1,3-dione (2.34g) is dissolved in ethanol (25ml) and thereto is added hydrazine anhydrous (200mg), and the mixture is stirred at 80°C for 3 hours and a half. The reaction solution is allowed cool to room temperature, and thereto is added brine and the mixture is extracted with ethyl acetate. The organic layer is washed with a saturated brine, dried over magnesium sulfate, and concentrated under reduced pressure. The resulting residue is purified by silica gel column chromatography (chloroform : methanol = 1:0→19:1) to give C-[2-(5-isopropyl-2-methoxyphenyl)-8-methyl-quinolin-3-yl]-methylamine (1.54g). MS (m/z): 321 (M+H]⁺
(4) C-[2-(5-isopropyl-2-methoxy-phenyl)-8-methyl-quinolin-3-yl]-methylamine (760mg) is dissolved in toluene (12ml), and thereto are added tert-butyl 4-(2-chloro-pyrimidin-5-yloxy)-butyrate (838mg), palladium acetate(II) (53mg), (±)-2,2-bis(diphenylphosphino)-1,1'-binaphthalene (148mg), tetra-n-butylammonium iodide (175mg) and sodium tert-butoxide (274mg), and the mixture is stirred at 85°C under nitrogen flow overnight The reaction solution is allowed cool to room temperature, and thereto is added water and the mixture is extrated with chloroform, and the organic layer is dried over magnesium sulfate and concentrated under reduced pressure. The resulting residue is purified by silica gel column chromatography (hexane : ethyl acetate = 4:1) to give tert-butyl 4-(2-{[2-(5-isopropyl-2-methoxy-phenyl)-8-methyl-quinolin-3-ylmethyl]-amino}-pyrimidin-5-yloxy)-butyrate (415mg). MS (m/z): 557 [M+H]⁺
(5) Tert-butyl 4-(2-{[2-(5-isopropyl-2-methoxy-phenyl)-8-methyl-quinolin-3-ylmethyl]-amino}-pyrimidin-5-yloxy)-butyrate (187mg) is dissolved in N,N-dimethylformamide (1.5ml), and thereto are added sodium hydride (60%) (20mg) and 3-bromomethyl-5-trifluoromethyl-benzonitrile (266mg) under water-cooling, and the mixture is stirred at room temperature for 30 minutes, and thereto are added sodium hydride (60%) (60mg) and 3-bromomethyl-5-trifluoromethyl-benzonitrile (100mg) again, and the mixture is stirred at room temperature for 2 hours and a half. To the reaction solution is added water and the mixture is extracted with chloroform and the organic layer is dried over magnesium sulfate and concentrated under reduced pressure. The resulting residue is purified by silica gel column chromatography (hexane : ethyl acetate = 4:1) to give tert-butyl 4-(2-{(3-cyano-5-trifluoromethyl-benzyl)-[2-(5-isopropyl-2-methoxyphenyl)-8-methyl-quinolin-3-ylmethyl]-amino}-pyrimidin-5-yloxy)-butyrate (144mg). MS (m/z): 740 [M+H]⁺
(6) To tert-butyl 4-(2-{(3-cyano-5-trifluoromethyl-benzyl)-[2-(5-isopropyl-2-methoxy-phenyl)-8-methyl-quinolin-3-ylmethyl]-amino}-pyrimidin-5-yloxy)-butyrate (135mg) is added a 4N-hydrochloric acid in dioxane (2ml), and the mixture is stirred at room temperature for 2 hours. To the reaction mixture is added a saturated brine, and the mixture is extracted with ethyl acetate and the organic layer is dried over magnesium sulfate and concentrated under reduced pressure. The resulting residue is purified by silica gel column chromatography (chloroform : methanol = 1:0→9:1) to give 4-(2-{(3-cyano-5-trifluoromethyl-benzyl)-[2-(5-isopropyl-2-methoxy-phenyl)-8-methyl-quinolin-3-ylmethyl]-amino}-pyrimidin-5-yloxy)-butyrate (115mg). MS (m/z): 684 [M+H]⁺

**Table 20**

| Ex. No. | Structural formula | Physical properties, etc. |
|---|---|---|
| 65 | 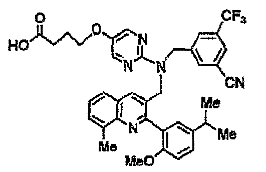 | MS (m/z): 684 [M+H]⁺ |

### Example 66

(1) Phthalaldehydic acid (2.5g) is dissolved in N,N-dimethylformamide (300ml) and thereto are added potassium carbonate (50.8g) and di-tert-butyl bromomalonate (54g), and the mixture is stirred at room temperature overnight. To the reaction solution are added water and diethyl ether, and the mixture is separated, and the organic layer is dried over magnesium sulfate and concentrated under reduced pressure. The resulting residue is dissolved in a 4N-hydrochloric acid/ethyl acetate (200ml) and the mixture is stirred at room temperature overnight. The solvent is evaporated under reduced pressure and the residue is dissolved in acetic acid (100ml) and 1,4-dioxane (500ml), and the mixture is heated under reflux overnight. The reaction solution is concentrated under reduced pressure and the precipitated crystals are filtered with diisopropylether to give 1-oxo-1H-isochromene-3-carboxylic acid (17.5g). MS (m/z): 191 [M+H]⁺
(2) 1-Oxo-1H-isochromene-3-carboxylic acid (17.5g) is dissolved in 7N-ammonia/methanol solution (350ml) and the mixture is stirred at room temperature overnight. The solvent is evaporated under reduced pressure and the residue is dissolved in a 4N-hydrochloric acid/dioxane (300ml) and the mixture is stirred at room temperature overnight. The reaction solution is concentrated under reduced pressure and The precipitated crystals are filtered with diisopropylether to give 1-oxo-1,2-dihydroisoquinoline-3-carboxylic acid (19.0g). MS (m/z): 190 [M+H]⁺
(3) The mixture of 1-oxo-1,2-dihydroisoquinoline-3-carboxylic acid (19.0g), a concentrated sulfuric acid (100ml) and methanol (500ml) is heated under reflux overnight. The reaction solution is poured into ice-water and the mixture is extracted with chloroform. The organic layer is washed with a saturated brine, dried over magnesium sulfate, and concentrated under reduced pressure. The precipitated crystals are filtered with diisopropylether to give methyl 1-oxo-1,2-dihydroisoquinoline-3-carboxylate (15.4g). MS (m/z): 204 [M+H]⁺
(4) The mixture of methyl 1-oxo-1,2-dihydroisoquinoline-3-carboxylate (15.3g), benzyl bromide (10.75ml), silver carbonate (31g) and toluene (300ml) is stirred at 100°C for 2 hours. The insoluble materials are removed by filtration through Celite™, and the filtrate is concentrated under reduced pressure. Crystallization of the residue from diisopropylether gives methyl 1-benzyloxy-isoquinoline-3-carboxylate (21.3g). MS (m/z): 294 [M+H]⁺
(5) The mixture of methyl 1-benzyloxy-isoquinoline-3-carboxylate (20.6g), sodium borohydride (15.9g) and tetrahydrofuran (500ml) is stirred at 70°C for 15 minutes. Then to the reaction solution are added dropwise methanol (100ml) over 1 hour, and the mixture is stirred for additional 1 hour. The reaction solution is cooled to room temperature, and thereto is added a saturated aqueous ammonium chloride solution to consume the excess reagents and the mixture is extracted with chloroform. The organic layer is washed with a saturated brine, dried over magnesium sulfate, and concentrated under reduced pressure. Crystallization of the residue from diisopropylether-hexane gives (1-benzoxy-isoquinolin-3-yl)-methanol (17.3g). MS (m/z): 266 [M+H]⁺
(6) (1-Benzyloxy-isoquinolin-3-yl)-methanol (500mg) is dissolved in methylene chloride (10ml), and thereto is added thionyl chloride (151µl) under ice-cooling and the mixture is stirred at the same temperature for 15 minutes and concentrated under reduced pressure. The resulting residue and (3,5-bis-trifluoromethyl-benzyl)-(5-bromo-pyrimidin-2-yl)-amine (752mg) is dissolved in N,N-dimethylformamide (5ml), and thereto is added sodium hydride (60%) (165mg) under ice-cooling, and the mixture is stirred at room temperature overnight. To the reaction solution are added diethyl ether and water, and the mixture is separated, and the organic layer is washed with a saturated brine, dried over magnesium sulfate, and concentrated under reduced pressure. The resulting residue is purified by silica gel column chromatography (hexane : ethyl acetate = 1:0→19:5) and the resulting crude product is recrystallized from diisopropylether to give (1-benzyloxy-isoquinolin-3-ylmethyl)-(3,5-bis-trifluoromethyl-benzyl)-(5-bromo-pyrimidin-2-yl)-amine (317mg). MS (m/z): 647/649 [M+H]⁺
(7) (1-Benzyloxy-isoquinolin-3-ylmethyl)-(3,5-bis-trifluoromethylbenzyl)-(5-bromo-pyrimidin-2-yl)-amine (6.35g) is dissolved in toluene (100ml), and thereto are added tris(dibenzylideneacetone)dipalladium (898mg), sodium tert-butoxide (1.41g), 2-(di-tert-butylphosphino)biphenyl (1.17g) and ethyl isonipecotate (2.2ml), and the mixture is stirred at room temperature under nitrogen atmosphere overnight. To the reaction solution are added water and ethyl acetate, and the mixture is separated, and the organic layer is dried over magnesium sulfate and concentrated under reduced pressure. The resulting residue is purified by NH-silica gel column chromatography (hexane : ethyl acetate = 1:0→9:1) to give ethyl 1-{2-[(1-benzyloxy-isoquinolin-3-ylmethyl)-(3,5-bis-trifluoromethyl-benzyl)-amino]-pyrimidin-5-yl}-piperidine-4-carboxylate (3.04g). MS (m/z): 724 [M+H]⁺
(8) Ethyl 1-{2-[(1-benzyloxy-isoquinolin-3-ylmethyl)-(3,5-bis-trifluoromethyl-benzyl)-amino]-pyrimidin-5-yl}-piperidine-4-carboxylate (2.88g) is dissolved in a mixed solvent of ethanol (10ml) and tetrahydrofuran (30ml), and thereto is added 10% palladium-carbon (500mg) and the mixture is stirred under hydrogen atmosphere at room temperature overnight. The catalyst is removed by filtration, and the filtrate is concentrated under reduced pressure and the resulting residue is purified by silica gel column chromatography (hexane : ethyl acetate = 4:1→1:4) to give ethyl 1-{2-[(3,5-bis-trifluoromethyl-benzyl)-(1-oxo-1,2-dihydroisoquinolin-3-ylmethyl)-amino]-pyrimidin-5-yl}-piperidine-4-carboxylate (1.80g). MS (m/z): 634 [M+H]⁺
(9) Ethyl 1-{2-[(3,5-bis-trifluommethyl-benzyl)-(1-oxo-1,2-dihydroisoquinolin-3-ylmethyl)-amino]-pyrimidin-5-yl}-piperidine-4-carboxylate (100mg) is dissolved in ethanol (1ml) and thereto is added a 2N-aqueous sodium hydroxide solution (215µl) and the mixture is stirred at room temperature overnight. To the reaction solution are added water and a 1N-hydrochloric acid to adjust the mixture to be pH 4, and the mixture is extracted with chloroform. The organic layer is dried over magnesium sulfate and the mixture is concentrated under reduced pressure to give 1-{2-[(3,5-bis-trifluoromethyl-benzyl)-(1-oxo-1,2-dihydroisoquinolin-3-ylmethyl)-amino]-pyrimidin-5-yl}-piperidine-4-carboxylic acid (85mg). MS (m/z): 606 [M+H]⁺

**Table 21**

| Ex. No. | Structural formula | Physical properties, etc. |
|---|---|---|
| 66 | 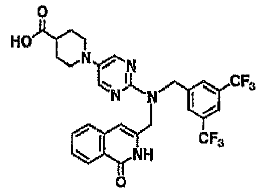 | MS (m/z): 606 [M+H]⁺ |

### Example 67

Ethyl 1-{2-[(1-benzyloxy-isoquinolin-3-ylmethyl)-(3,5-bis-trifluoromethyl-benzyl)-amino]-pyrimidin-5-yl}-piperidine-4-carboxylate (150mg) is treated in a similar manner to Example 4(2) to give 1-{2-[(1-benzyloxy-isoquinolin-3-ylmethyl)-(3,5-bis-trifluoromethyl-benzyl)-amino]-pyrimidin-5-yl}-piperidine-4-carboxylic acid (127mg). MS (m/z): 696 [M+H]⁺

### Example 68

(1) Ethyl 1-{2-[(3,5-bis-trifluoromethyl-benzyl)-(1-oxo-1,2-dihydroisoquinolin-3-ylmethyl)-amino]-pyrimidin-5-yl}-piperidine-4-carboxylate (300mg), isopropanol (49µl) and triphenylphosphine (170mg) are dissolved in tetrahydrofuran (5ml), and thereto is added dropwise a 40% diethyl azodicarboxylate/toluene (255µl) and the mixture is stirred at room temperature for 1 hour. The reaction solution is concentrated under reduced pressure and the resulting residue is purified by NH-silica gel column chromatography (hexane : ethyl acetate = 1:0→95:5) to give ethyl 1-{2-[(3,5-bis-trifluoromethyl-benzyl)-(1-isopropoxy-isoquinolin-3-ylmethyl)-amino]-pyrimidin-5-yl}-piperidine-4-carboxylate (281mg). MS (m/z): 676 [M+H]⁺
(2) Ethyl 1-{2-[(3,5-bis-trifluoromethyl-benzyl)-(1-isopropoxy-isoquinolin-3-ylmethyl)-amino]-pyrimidin-5-yl}-piperidine-4-carboxylate (275mg) is dissolved in ethanol (5ml), and thereto is added a 2N-aqueous sodium hydroxide solution (610µl) and the mixture is stirred at room temperature overnight. To the reaction solution are added water and a 1N-hydrochloric acid to adjust the mixture to be pH 4 and the mixture is extracted with chloroform. The organic layer is dried over magnesium sulfate and concentrated under reduced pressure. The resulting residue is purified by silica gel column chromatography (chloroform : methanol - 1:0→94:6) to give 1-{2-[(3,5-bis-trifluoromethyl-benzyl)-(1-isopropoxy-isoquinolin-3-ylmethyl)-amino]-pyrimidin-5-yl}-piperidine-4-carboxylic acid (215mg). MS (m/z): 648 [M+H]⁺

### Example 69

(1) Ethyl 1-{2-[(3,5-bis-trifluoromethyl-benzyl)-(1-oxo-1,2-dihydroisoquinolin-3-ylmethyl)-amino]-pyrimidin-5-yl}-piperidine-4-carboxylate (300mg), 2-methoxyethanol (51µl) and triphenylphosphine (170mg) are dissolved in tetrahydrofuran (5ml), and thereto is added dropwise a 40% diethyl azodicarboxylate/toluene (255µl), and the mixture is stirred at room temperature for 1 hour. The reaction solution is concentrated under reduced pressure and the resulting residue is purified by NH-silica gel column chromatography (hexane : ethyl acetate = 93:7→67:33) to give ethyl 1-(2-{(3,5-bis-trifluoromethyl-benzyl)-[1-(2-methoxy-ethoxy)-isoquinolin-3-ylmethyl]-amino}-pyrimidin-5-yl)-piperidine-4-carboxylate (187mg; MS (m/z): 692 [M+H]⁺) and ethyl 1-(2-{(3,5-bis-trifluoromethyl-benzyl)-[2-(2-methoxy-ethyl)-1-oxo-1,2-dihydroisoquinolin-3-ylmethyl]-amino}-pyrimidin-5-yl)-piperidine-4-carboxylate (94mg; MS (m/z): 692 [M+H]⁺).
(2) Ethyl 1-(2-{(3,5-bis-trifluoromethyl-benzyl)-[1-(2-methoxy-ethoxy)-isoquinolin-3-ylmethyl]-amino}-pyrimidin-5-yl)-piperldine-4-carboxylate obtained in the above (1) (180mg) is dissolved in ethanol (3ml), and thereto is added a 2N-aqueous sodium hydroxide solution (390µl) and the mixture is stirred at room temperature overnight. To the reaction solution are added water and a 1N-hydrochloric acid to adjust the mixture to be pH 4 and the mixture is extracted with chloroform. The organic layer is dried over magnesium sulfate and concentrated under reduced pressure. The resulting residue is purified by silica gel column chromatography (chloroform : methanol = 1:0→93:7) to give 1-(2-{(3,5-bis-trifluoromethylbenzyl)-[1-(2-methoxy-ethoxy)-isoquinolin-3-ylmethyl]-amino}-pyrimidin-5-yl)-piperidine 4-carboxylic acid (147mg). MS (m/z): 664 [M+H]⁺

### Examples 70 to 72

The corresponding starting compounds are treated in a similar manner to Example 69 to give the compounds as listed in Table 22.

**Table 22**

| | | |
|---|---|---|
| 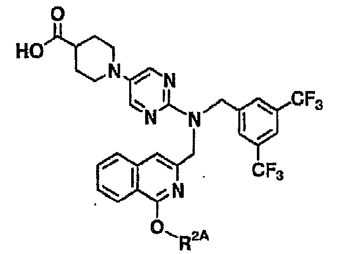 | | |

| Ex. No. | -R^{2A} | Physical properties, etc. |
|---|---|---|
| 67 | 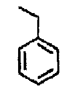 | MS (m/z): 696 [M+H]⁺ |
| 68 | 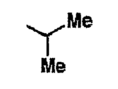 | MS (m/z): 648 [M+H]⁺ |
| 69 | 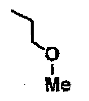 | MS (m/z): 664 [M+H]⁺ |
| 70 | 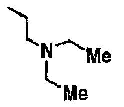 | MS (m/z): 705 [M+H]⁺ |
| 71 | 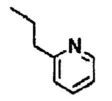 | MS (m/z): 711 [M+H]⁺ |
| 72 | 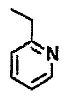 | MS (m/z): 697 [M+H]⁺ |

### Example 73

Ethyl 1-(2-{(3,5-bis-trifluoromethyl-benzyl)-[2-(2-methoxy-ethyl)-1-oxo-1,2-dihydroisoquinolin-3-ylmethyl]-amino}-pyrimidin-5-yl)-piperidine-4-carboxylate obtained in Example 69(1) (90mg) is dissolved in ethanol (2ml), and thereto is added a 2N-aqueous sodium hydroxide solution (195µl) and the mixture is stirred at room temperature overnight. To the reaction solution are added water and a 1N-hydrochloric acid to adjust the mixture to be about pH 4, and the mixture is extracted with chloroform. The organic layer is dried over magnesium sulfate and concentrated under reduced pressure. The resulting residue is purified by silica gel column chromatography (chloroform: methanol = 99:1→92:8) to give 1-(2-{(3,5-bis-trifluoromethyl-benzyl)-[2-(2-methoxy-ethyl)-1-oxo-1,2-dihydroisoquinolin-3-ylmethyl]-amino}-pyrimidin-5-yl)-piperidine-4-carboxylic acid (22mg). MS (m/z): 664 [M+H]⁺

### Examples 74 to 76

The corresponding starting compounds are treated in a similar manner to Example 73 to give the compounds as listed in Table 23.

**Table 23**

| | | |
|---|---|---|
| 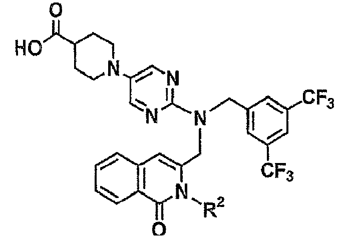 | | |

| Ex. No. | -R² | Physical properties, etc. |
|---|---|---|
| 73 | 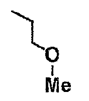 | MS (m/z): 664 [M+H]⁺ |
| 74 | 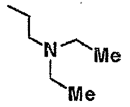 | MS (m/z) : 705 [M+H]⁺ |
| 75 | 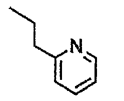 | MS (m/z) : 711 [M+H]⁺ |
| 76 | 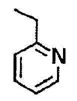 | MS (m/z) : 697 [M+H]⁺ |

### Examples 77 to 78

The corresponding starting compounds are treated in similar manners to any of the above Examples to give the compounds as listed in Table 24.

**Table 24**

| | | | |
|---|---|---|---|
| 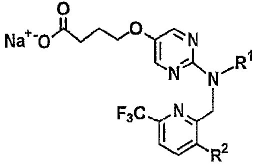 | | | |

| Ex. No. | -R¹ | -R² | Physical properties, etc. |
|---|---|---|---|
| 77 | 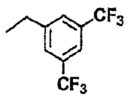 | 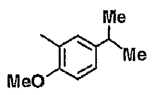 | |
| 78 | 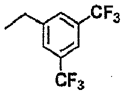 | 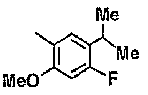 | |
| 79 | 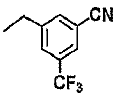 | 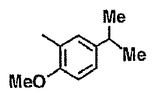 | |
| 80 | 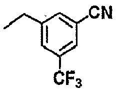 | 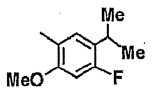 | |

### Examples 81 to 84

The corresponding starting compounds are treated in similar manners to any of the above Examples to give the compounds as listed in Table 25.

**Table 25**

| | | | |
|---|---|---|---|
| 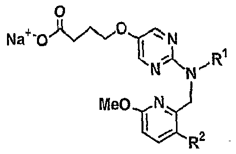 | | | |

| Ex. No. | -R¹ | -R² | Physical properties, etc. |
|---|---|---|---|
| 81 | 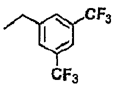 | 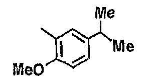 | |
| 82 | 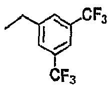 | 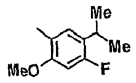 | |
| 83 | 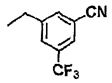 | 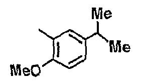 | |
| 84 | 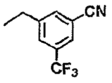 | 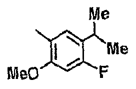 | |

### Reference Example 1

Cyclohexanecarboxaldehyde (38g), diethylamine hydrochloride (55g) and acetic acid (29ml) are dissolved in methylene chloride (500ml) and thereto is added triacetoxy sodium borohydride (71.8g) at room temperature and the mixture is stirred at room temperature overnight. To the reaction solution are added a 2N-aqueous sodium hydroxide solution and methylene chloride, and the mixture is separated, and the organic layer is washed with a saturated brine, dried over magnesium sulfate, and the mixture is concentrated under reduced pressure to give cyclohexylmethy-ethyl-amine (40.1g) as a crude product. MS (m/z): 142 [M+H]⁺

### Reference Example 2

3,5-Bis-trifluoromethyl-benzylamine (10g) and 5-bromo-2-chloropyrimidine (12g) is dissolved in 1,4-dioxane (50ml) and thereto is added N,N-diisopropylethylamine (10.7ml) and the mixture is heated under reflux overnight. The reaction solution is cooled to room temperature and concentrated under reduced pressure. The resulting residue is purified by silica gel column chromatography (hexane : ethyl acetate = 9:1→7:3) to give (3,5-bis-trifluoromethyl-benzyl)-(5-bromo-pyrimidin-2-yl)-amine (10.1g). MS (m/z): 713 [M+H]⁺

### Reference Example 3

(1) Ethylamine hydrochloride (2g) is dissolved in methylene chloride (20ml) and thereto are added pyridine (6ml) and ethyl 6-(chloroformyl)hexanoate (7.6g) and the mixture is stirred at room temperature overnight. To the reaction solution are added a saturated aqueous sodium bicarbonate solution and the mixture is separated, and the organic layer is washed successively with a 1 N-hydrochloric acid, water and a saturated brine, dried over magnesium sulfate, and concentrated under reduced pressure to give ethyl 6-ethylcarbamoyl hexanoate (9.29g) as a crude product. MS (m/z): 216 [M+H]⁺
(2) Crude ethyl 6-ethylcarbamoyl hexanoate (9.29g) is dissolved in tetrahydrofuran (50ml) and thereto is added sodium borohydride (7.35g). The reaction solution is heated under reflux and thereto is added dropwise acetic acid (11ml) and the mixture is heated under reflux for 1 hour and 30 minutes. To the reaction solution is added water under ice-cooling and the mixture is extracted with ethyl acetate. The organic layer is washed with a saturated brine, dried over magnesium sulfate, and concentrated under reduced pressure. The resulting residue is dissolved in ethanol (30ml), and thereto is added a 4N-hydrochloric acid in ethyl acetate (7.6ml), and the mixture is stirred at room temperature overnight. To the reaction solution are added a 2N-aqueous sodium hydroxide solution and ethyl acetate and the mixture is separated, and the organic layer is washed with a saturated brine, dried over magnesium sulfate, and concentrated under reduced pressure to give ethyl 7-ethylamino-heptanaate (3.98g) as a crude product. MS (m/z): 216 [M+H]⁺

### Reference Example 4

(1) 6-Aminohexanoic acid methyl ester hydrochloride (5g) is dissolved in methylene chloride (20ml) and thereto are added pyridine (4.5ml) and acetyl chloride (2ml), and the mixture is stirred at room temperature for 1 hour and 45 minutes. To the reaction solution are added a 1N-hydrochloric acid and chloroform, and the mixture is separated, and the organic layer is washed with a saturated brine, dried over magnesium sulfate, and concentrated under reduced pressure to give methyl 6-acetylamino-hexanoate (5.19g) as a crude product. MS (m/z): 188 [M+H]⁺
(2) Crude methyl 6-acetylamino-hexanoate (5.19g) is dissolved in tetrahydrofuran (50ml) and thereto is added sodium borohydride (5.03g). The reaction solution is heated under reflux and thereto is added dropwise acetic acid (7.6ml) and the mixture is heated under reflux for 1 hour and 30 minutes. To the reaction solution are added water under ice-cooling and the mixture is extracted with ethyl acetate. The organic layer is washed with a saturated brine, dried over magnesium sulfate, and concentrated under reduced pressure. The resulting residue is dissolved in ethanol (30ml) and thereto is added a 4N-hydrochloric acid in ethyl acetate (7.6ml) and the mixture is stirred at room temperature overnight. To the reaction solution are added a 2N-aqueous sodium hydroxide solution and ethyl acetate, and the mixture is separated, and the organic layer is washed with a saturated brine, dried over magnesium sulfate, and concentrated under reduced pressure to give ethyl 6-ethylamino-hexanoate (1.74g) as a crude product. MS (m/z): 188 [M+H]⁺

### Reference Example 5

(1) (3,5-Bis-trifluoromethyl-benzyl)-(5-bromopyrmidin-2-yl)-amine (10g) and triethylamine (4.18ml) are dissolved in methylene chloride (100mL) and thereto is added triphosgene (2.97g) under ice-cooling. The reaction solution is stirred at the same temperature for 30 minutes and concentrated under reduced pressure. The resulting residue is dissolved in tetrahydrofuran (100mL) and thereto are added benzyl alcohol (3.88ml) and triethylamine (10.45ml) at room temperature, and the mixture is stirred overnight. The reaction solution is diluted with ethyl acetate and washed with a saturated aqueous sodium bicarbonate solution and a 1N-hydrochloric acid. The organic layer is washed with a saturated brine, dried over magnesium sulfate, and concentrated under reduced pressure. The resulting residue is purified by silica gel column chromatography (hexane : ethyl acetate = 97:3→9:1) to give benzyl (3,5-bis-trifluoromethylbenzyl)-(5-bromo-pyrimidin-2-yl)-carbamate (11.58g). MS (m/z): 534/536 [M+H]⁺
(2) Benzyl (3,5-bis-trifluoromethyl-benzyl)-(5-bromo-pyrimidin-2-yl)-carbamate (11.5g), [1,1'-bis(diphenylphosphino)ferrocene]-dichloropalladium dichloromethane complex (3.51g), potassium acetate (6.33g) and bis(pinacolate)diboron (10.9g) are dissolved in dimethylsulfoxide (75ml), and the mixture is heated to 80°C under nitrogen atmosphere and stirred for 30 minutes. The reaction solution is cooled to room temperature and thereto are added water and ethyl acetate, and the insoluble materials are removed by filtration through Celite™, and the mixture is separated and the organic layer is washed with a saturated brine, dried over magnesium sulfate, and concentrated under reduced pressure. The resulting residue is dissolved in tetrahydrofuran (100ml) and thereto is added dropwise a 30 % aqueous hydrogen peroxide solution (50ml) under ice-cooling and the mixture is stirred for 1 hour. Thereto is added a saturated aqueous sodium thiosulfate solution under ice-cooling to consume the excess hydrogen peroxide, followed by an addition of water and ethyl acetate, and the mixture is separated. The organic layer is washed with a saturated brine, dried over magnesium sulfate, and concentrated under reduced pressure. The resulting residue is purified by silica gel column chromatography (chloroform : methanol = 1:0→9:1) to give benzyl (3,5-bis-trifluoromethyl-benzyl)-(5-hydroxy-pyrimidin-2-yl)-carbamate (9.70g). MS (m/z): 472 [M+H]⁺
(3) Benzyl (3,5-bis-trifluoromethyl-benzyl)-(5-hydroxy-pyrimidm-2-yl)-carbamate (9.70g) and ethyl 4-bromobutyrate (3.53g) are dissolved in N,N-dimethylformamide (50mL) and thereto is added potassium carbonate (3.41g) and the mixture is stirred at 50°C for 1 hour. Thereto are added ethyl acetate and a saturated brine, and the mixture is separated, and the organic layer is washed with a saturated brine, dried over magnesium sulfate, and concentrated under reduced pressure. The resulting residue is purified by silica gel column chromatography (hexane : ethyl acetate = 4:1→1:1) to give ethyl 4-(2-[benzyloxycarbonyl-(3,5-bis-trifluoromethylbenzyl)-amino]-pyrimidin-5-yloxy}-butyrate (8.29g). MS (m/z): 586 [M+H]⁺
(4) Ethyl 4-{2-[benzyloxycarbonyl-(3,5-bis-trifluoromethyl-benzyl)-amino]-pyrimidin-5-yloxy)-butyrate (3.0g) is dissolved in tetrahydrofuran (20ml) and thereto is added 10% palladium-carbon (500mg) and the mixture is stirred under hydrogen atmosphere at room temperature for 2 hours and 30 minutes. The catalyst is removed by filtration, and the filtrate is concentrated under reduced pressure. The resulting residue is purified by silica gel column chromatography (hexane : ethyl acetate = 9:1→1:1) to give ethyl 4-[2-(3,5-bis-trifluoromethyl-benzylamino)-pyrimidin-5-yloxy]-butyrate (2.22g). MS (m/z): 452 [M+H]⁺

### Reference Examples 6

(1) 6-Aminohexanoic acid methyl ester hydrochloride (2.50g) is dissolved in tetrahydrofuran (50ml) and thereto are added water (50ml) and sodium bicarbonate (3.44g), followed by an addition dropwise of benzyl chloroformate (2.17ml) under ice-cooling, and the mixture is stirred at the same temperature for 2 hours and a half. To the reaction mixture is added a saturated brine, and the mixture is extracted with ethyl acetate. The organic layer is washed twice with a saturated brine, dried over magnesium sulfate, and concentrated under reduced pressure to give methyl 6-benzyloxycarbonylamino hexanoate (4.16g). MS (m/z): 280 [M+H]⁺
(2) Methyl 6-benzyloxycarbonylamino-hexanoate (4.15g) is dissolved in N,N-dimethylformamide (25mL) ans thereto is added sodium hydride (63%) (552mg) under ice-cooling and the mixture is stirred at the same temperature for 1 hour and thereto is added methyl iodide (1.72ml), and the mixture is stirred at the same temperature for an additional 2 hours. To the reaction mixture is added a saturated brine, and the mixture is extracted with ethyl acetate, and the organic layer is washed with a saturated brine, dried over magnesium sulfate, and concentrated under reduced pressure. The resulting residue is purified by silica gel column chromatography (hexane : ethyl acetate = 4:1) to give methyl 6-(benzyloxycarbonyl-methyl-amino)-hexanoate (2.25g). MS (m/z): 294 [M+H]⁺
(3) Methyl 6-(benzyloxycarbonyl-methyl-amino)-hexanoate (2.24g) is dissolved in methanol (35ml) and thereto is added 10% palladium-carbon (500mg) and the mixture is stirred at room temperature under hydrogen atmosphere for 2 hours. The reaction mixture is filtered and the filtrate is concentrated under reduced pressure to give methyl 6-methylamino hexanoate (1.11g). MS (m/z): 160 [M+H]⁺

### Reference Example 7

Propylamine (0.65g) is dissolved in tetrahydrofuran (5ml) and thereto is added pyridine (0.89ml), followed by an addition dropwise of methyl adipoyl chloride (1.96g) under ice-cooling and the mixture is stirred at room temperature for 1 hour. To the reaction mixture is added a saturated brine, and the mixture is extracted with ethyl acetate. The organic layer is washed with a saturated brine, dried over magnesium sulfate, and concentrated under reduced pressure. The resulting residue is dissolved in tetrahydrofuran (30ml) and thereto is added sodium borohydride (1.93g) at room temperature and the mixture is heated to 65°C and thereto is added dropwise acetic acid (2.92ml) over 1 hour and the mixture is stirred at the same temperature for 9 hours. To the reaction mixture is added an ice-cooled dilute hydrochloric acid, and the mixture is stirred for 30 minutes and extracted with ethyl acetate, and the organic layer is washed with a mixed solution of a saturated aqueous sodium bicarbonate solution and a saturated brine, dried over magnesium sulfate, and concentrated under reduced pressure. The resulting residue is dissolved in methanol (15ml) and thereto is added a 4N-hydrochloric acid in dioxane (7.5mL) and the mixture is stirred at room temperature overnight To reaction mixture is added a saturated aqueous sodium bicarbonate solution and a saturated brine, and the mixture is extracted six times with ethyl acetate and the collected organic layer is dried over magnesium sulfate, and concentrated under reduced pressure to give methyl 6-propylamino-hexanoate (914mg). MS (m/z): 188 [M+H]⁺

### Reference Example 8

(1) Tert-butyl piperidine-4-ylmethyl-carbamate (2.00g) is dissolved in tetrahydrofuran (10ml), and thereto is added triethylamine (1.69ml), followed by an addition dropwise of ethyl bromoacetate (1.24mL) in water bath and the mixture is stirred at room temperature for 2 hours. To the reaction mixture is added a saturated brine, and the mixture is extracted with ethyl acetate. The organic layer is washed twice with a saturated brine, dried over magnesium sulfate, and concentrated under reduced pressure, and to the resulting crystalline residue is added isopropylether and the mixture is filtered to give ethyl [4-(tert-butoxy-carbonylaminomethyl)-piperidin-1-yl]-acetate (1.87g). MS (m/z): 301 [M+H]⁺
(2) Ethyl [4-(tert-butoxycarbonylamino-methyl)-piperidin-1-yl]-acetate (1.86g) is dissolved in N,N-dimethylformamide (10ml), and thereto are added sodium hydride (63%) (1.19g) and ethyl iodide (6.0ml), and the mixture is stirred at room temperature for 2 hours. The reaction mixture is made weak basic with a 10% aqueous citric acid solution and a saturated aqueous sodium bicarbonate solution, and extracted with ethyl acetate, and the organic layer is washed with a saturated brine, dried over magnesium sulfate, and concentrated under reduced pressure. The resulting residue is purified by silica gel column chromatography (hexene : ethyl acetate = 2:1) to give ethyl {4-[(tert-butoxycarbonyl-ethyl-amino)-methyl]-piperidine-1-yl}-acetate (905mg). MS (m/z): 329 [M+H]⁺
(3) Ethyl (4-[(tert-butoxycarbonyl-ethyl-amino)-methyl]-piperidine-1-yl-acetate (235mg) is dissolved in methylene chloride (1ml) and thereto is added trifluoroacetic acid (1ml) and the mixture is stirred at room temperature overnight. The reaction mixture is concentrated under reduced pressure to give (4-ethylaminomethyl-piperidin-1-yl)-acetic acid ethyl ester bistrifluoroacetic acid salt (482mg). MS (m/z): 229 [M+H]⁺

### Reference Example 9

(1) 2-Tert-butoxy-ethylamine (2g) is dissolved in methylene chloride (10ml) and thereto are added pyridine (940µl) and ethyl 6-(chloroformyl)hexanoate (1.13g) under ice-cooling and the mixture is stirred at room temperature overnight. To the reaction solution are added a 1N-hydrochloric acid and chloroform, and the mixture is separated, and the organic layer is washed with a saturated brine, dried over magnesium sulfate, and concentrated under reduced pressure to give ethyl 6-(2-tert-ethylcarbamoyl)-hexanoate as a crude product (2.96g). MS (m/z): 288 [M+H]⁺
(2) Crude ethyl 6-(2-tert-ethylcarbamoyl)-hexanoate (2.96g) is dissolved in tetrahydrofuran (15ml) and thereto is added sodium borohydride (1.60g). The reaction solution is heated under reflux and thereto is added dropwise acetic acid (2.4mL) and the mixture is heated under reflux for 1 hour. To the reaction solution is added water under ice-cooling and the mixture is extracted with ethyl acetate. The organic layer is washed with a saturated brine, dried over magnesium sultate, and concentrated under reduced pressure. The resulting residue is dissolved in ethanol (6ml) and thereto is added a 4N-hydrochloric acid in ethyl acetate (1.5ml) and the mixture is stirred at room temperature for 3 days. To the reaction solution are added a saturated aqueous sodium bicarbonate solution and ethyl acetate, and the mixture is separated, and the organic layer is washed with a saturated brine, dried over magnesium sulfate, and concentrated under reduced pressure to give ethyl 7-(2-tert-butoxy-ethylamino)-heptanoate as a crude product (2.6g). MS (m/z): 274 [M+H]⁺

### Reference Example 10

(1) 3-Nitro-5-(trifluoromethyl)benzoic acid (50g) is dissolved in tetrahydrofuran (300ml) and thereto is added dropwise a 1.0M-borane tetrahydrofuran complex/tetrahydrofuran (300ml) at 0°C under nitrogen atmosphere over 2 hours and the mixture is stirred at 75°C for 1 hour and a half. The reaction solution is allowed cool to room temperature and concentrated under reduced pressure, and thereto is added a 1N-hydrochloric acid and the mixture is extracted with ethyl acetate. The organic layer is washed successively with water and a saturated brine, dried over magnesium sulfate, and concentrated under reduced pressure to give crude (3-nitro-5-trifluoromethyl-phenyl)-methanol. The obtained crude product is dissolved in methanol (500mL) and thereto is added 10% palladium-carbon (5g) and the mixture is stirred under hydrogen atmosphere at room temperature overnight. The catalyst is removed by filtration, and the filtrate is concentrated under reduced pressure to give crude (3-amino-5-trifluoromethyl-phenyl)-methanol. To copper (II) bromide (53.6g) is added acetonitrile (500ml), followed by an addition dropwise of tert-butyl nitrite (35.7ml) under ice-cooling and the mixture is stirred under nitrogen atmosphere for 5 minutes. To reaction mixture is added dropwise a solution of the above crude (3-amino-5-trifluoromethyl-phenyl)-methanol in acetonitrile (200ml) under ice-cooling over 1 hour and 15 minutes and the mixture is stirred at room temperature under nitrogen atmosphere overnight. To reaction mixture is added a 1N-hydrochloric acid and the mixture is extracted with ethyl acetate. The organic layer is washed successively with a 1N-hydrochloric acid, water and a saturated brine, dried over magnesium sulfate, and concentrated under reduced pressure. The resulting residue is purified by silica gel column chromatography (hexane : ethyl acetate = 7:1→4:1) to give (3-bromo-5-trifluoromethyl-phenyl)-methanol (40.7g). NMR (CDCl₃): 1.90 (1H,t), 4.76 (2H,d), 7.56 (1H, s), 7.68 (1H, s), 7.72 (1H,s)
(2) (3-Bromo-5-trifluoromethyl-phenyl)-methanol (33.9g) is dissolved in N,N-dimethylformamide (400mL) and thereto are added zinc(II) cyanide (16.39g) and tetrakis(triphenylphosphine)palladium (7.68g) and the mixture is heated under nitrogen atmosphere at 120°C for 2 hours. The reaction solution is allowed cool to room temperature, and filtered through Celite™, and the filtrate is concentrated under reduced pressure. Thereto is added water and the mixture is extracted with ethyl acetate. The organic layer is washed with a saturated brine, dried over sodium sulfate, and concentrated under reduced pressure. The resulting residue is purified by silica gel column chromatography (hexane : ethyl acetate = 2:1) to give 3-hydroxymethyl-5-trifluoromethyl-benzonitrile (23.4g). NMR (CDCl₃): 2.09 (1H,t), 4.85 (2H,d), 7.83 (1H,s), 7.87 (2H,s)
(3) 3-Hydroxymethyl-5-trifluoromethyl-benzonitrile (23.4g) is dissolved in methylene chloride (230mL) and thereto is added carbon tetrabromide (42.4g), followed by an addition of triphenylphosphine (32.0g) under ice-cooling and the mixture is stirred at the same temperature for 30 minutes. The reaction solution is concentrated under reduced pressure. The resulting residue is purified by silica gel column chromatography (hexane : ethyl acetate = 10:1) to give 3-bromo-methyl5-trifluoromethyl-benzonitrile (25.5g). NMR (CDCl₃): 4.51 (2H,s), 7.86 (1H,s), 7.88 (2H,s)

### Reference Example 11

(1) 2-Bromo-pyridine-3-ol (5g) is dissolved in water (150ml) and thereto are added sodium carbonate (6.15g) and iodine (7.65g) and the mixture is stirred at room temperature for 2 hours. Thereto are added a 1N-hydrochloric acid and ethyl acetate, and the mixture is separated, and the organic layer is washed with a saturated brine, dried over magnesium sulfate, and concentrated under reduced pressure. The residue is purified by silica gel column chromatography (hexane : ethyl acetate = 9:1→3:1) to give 2-bromo-6-iodo-pyridin-3-ol (4.52g). MS (m/z): 300/302 [M+H]⁺
(2) 2-Bromo-6-iodo-pyridin-3-ol (2.98g) is dissolved in N,N-dimethylformamide (140ml) and thereto are added cesium carbonate (16.3g) and methyl iodide (1.25mL) and the mixture is stirred at room temperature overnight. To the reaction solution are added water and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 19:1→4:1) to give 2-bromo-6-iodo-3-methoxy-pyridine (2.45g). MS (m/z): 314/316 [M+H]⁺
(3) To 0.5M-isopropenyl magnesium bromide/tetrahydrofuran is added trimethyl borate (3.3ml) and the mixture is stirred under nitrogen atmosphere at room temperature for 30 minutes. To the reaction solution are added 6N-hydrochloric acid and diethylether, and the mixture is separated. The organic layer is washed with a saturated brine, dried over magnesium sulfate, and concentrated under reduced pressure to give isopropenylboric acid as a crude product (818mg). The above crude isopropenyl boric acid (287mg) and 2-bromo-6-iodo-3-methoxy-pyridine (800mg) are dissolved in a mixed solvent of 1,2-dimethoxy-ethane (8ml) and ethanol (3.2ml) and thereto are added a 1M-aqueous sodium carbonate solution (6.4ml) and tetrakis(triphenylphosphine)palladium (240mg) and the mixture is stirred under nitrogen atmosphere at 80°C for 5 hours. To the reaction solution are added water and ethyl acetate, and the mixture is separated, and the organic layer is washed with a saturated brine, dried over magnesium sulfate, and concentrated under reduced pressure. The residue is purified by silica gel column chromatography (hexane : ethyl acetate = 49:1→9:1) and followed by NH-silica gel column chromatography (hexane : ethyl acetate = 49:1→9:1) to give 2-bromo-6-isopropenyl-3-methoxy-pyridine (138mg). MS (m/z): 228/230 [M+H]⁺

### Reference Example 12

2-Bromo-6-iodo-3-methoxy-pyridine (500mg) is dissolved in dry toluene (5ml) and thereto is added dropwise 1.6M n-butyllithium in hexanes (1ml) at -78°C under nitrogen atmosphere, and the mixture is stirred for 1 hour and thereto is added acetone (0.23ml), and the mixture is stirred overnight To the reaction solution are added water and ethyl acetate, and the mixture is separated, and the organic layer is washed with a saturated brine, dried over magnesium sulfate, and concentrated under reduced pressure. The resulting residue is purified by silica gel column chromatography (hexane : ethyl acetate = 4:1→3:2) to give 2-(6-bromo-5-methoxy-pyridin-2-yl)-propan-2-ol (197mg). MS (m/z): 246/248 [M+H]⁺

### Reference Example 13

5-Methoxy-2-methylsulfanil-pyrimidin-4-ol (250mg) is dissolved in acetonitrile (7ml) and thereto are added phoshporus oxychloride (0.7ml) and diethyl aniline (460µl) and the mixture is heated under reflux for 5.5 hours. The reaction solution is evaporated azeotropically with toluene three times, and to the residue are added aqueous citric acid solution and chloroform, and the mixture is separated. The organic layer is washed with a saturated brine, dried over magnesium sulfate, and concentrated under reduced pressure. The resulting residue is purified by silica gel column chromatography (hexane : ethyl acetate = 19:1→17:3) to give 4-chloro-5-methoxy-2-methylsulfanil-pyrimidine (260mg). MS (m/z): 191/193 [M+H]⁺

### Reference Example 14

2-Chloropyrimidin-5-ol (3.89g) is dissolved in N,N-dimethylformamide (50ml) and thereto are added potassium carbonate (4.98g) and tert-butyl 4-bromo-butyrate (7.36g) and the mixture is stirred at room temperature overnight To the reaction solution are added ethyl acetate and water, and the mixture is separated, and the organic layer is washed with a saturated brine, dried over magnesium sulfate, and concentrated under reduced pressure. The resulting residue is purified by silica gel column chromatography (hexane : ethyl acetate = 24:1→4:1) to give tert-butyl 4-(2-chloropyrimidin-5-yloxy)bromobutyrate (6.22g). MS (m/z): 273 [M+H]⁺

### Reference Example 15

2,5-Dibromopyridine (4.74g) is dissolved in toluene (100ml) and thereto are added 3,5-bis-trifluoromethyl-benzylamine (5.84g) and palladium acetate (449.0mg), 2,2'-bis(diphenylphosphino)-1,1'-binaphthyl (1.25g) and sodium tert-butoxide (4.23g) and the mixture is stirred under nitrogen atmosphere at 80°C for 12 hours. The reaction solution is cooled to room temperature, and thereto is added a saturated aqueous sodium bicarbonate solution and the mixture is extracted with ethyl acetate twice, and the organic layer is washed successively with water and a saturated brine, dried over magnesium sulfate, and concentrated under reduced pressure. The resulting residue is purified by silica gel column chromatography (hexane : ethyl acetate = 19:1→4:1) to give (3,5-bis-trifluoromethyl-benzyl)-(5-bromopyridin-2-yl)-amine (2.08g). MS (m/z): 399/401 [M+H]⁺

### Reference Example 16

3-Bromomethyl-5-trifluorometyl-benzonitrile (which is prepared in Referrence Example 12) (15.9g) is dissolved in 7M-ammonia/methanol (550ml), and the mixture is stirred at 50-60°C for 30 minuites. The reaction solution is concentrated under reduced pressure. To the resulting residue are added a saturated aqueous sodium bicarbonate solution and chloroform, and the mixture is separated, and the organic layer is dried over sodium sulfate, and concentrated under reduced pressure. The resulting residue is purified by silica gel column chromatography (chloroform : methanol = 1:0→19:1→chloroform : methanol : ammonium hydroxyide solution = 19:1:0.1) to give 3-aminomethyl-5-trifluorometyl-benzonitrile (10.4g). MS (m/z): 201 [M+H]⁺

### Reference Example 17

Tert-butyl 4-(2-chloropyrimidin-5-yloxy)-butyrate (5.0g) is dissolved in toluene (100ml) and thereto are added palladium acetate (412mg) and 2,2'-bis(diphenylphosphino)-1,1'-binaphthyl (1.26g), and the mixture is stirred under nitrogen atmosphere at 50 °C for 1 hour. The reaction solution is cooled to room temperature, and thereto are added 3,5-bis-trifluoromethyl-benzylamine (5.35g) and sodium tert-butoxide (3.88g) and the mixture is stirred at 35°C for 2 hours. Thereto are added ethyl acetate and water, and the mixture is separated, and the organic layer is washed with a saturated brine, dried over magnesium sulfate, and concentrated under reduced pressure. The residue is purified by silica gel column chromatography (hexane : ethyl acetate = 9:1→2:1) and NH-silica gel column chromatography (hexane : ethyl acetate = 4:1→2:1) to give tert-butyl 4-[2-(3,5- bis-trifluoromethyl-benzylamino)-pyrimidin-5-yloxy]-butyrate (5.59g). MS (m/z): 480 [M+H]⁺

### Reference Example 18

The corresponding starting compound is treated in a similar manner to Reference Example 17 to give the compound.

### Reference Example 19

(1) 3-Bromo-4-methoxybenzoic acid (2.00g) is dissolved in tetrahydrofuran (50ml), and the mixture is cooled to -78°C, and thereto is added dropwise a 1.1M methyllithium in diethyl ether (7.7ml). The mixture is stirred at -78°C for 5 minutes, and thereto is added dropwise a 1.6M tert-butyllithium in n-pentane (13.2ml), and the mixture is stirred at -78°C for 15 minutes, and the mixture is allowed to warm to -45°C, and the mixture is stirred for 45 minutes, and then again cooled to -78°C. Thereto is added dropwise triisopropylborate, and the mixture is stirred at -78°C for 15 minutes, and the mixture is allowed to warm to room temperature. The mixture is stirred at room temperature for 1.5 hours, and the mixture is concentrated under reduced pressure, and thereto are added water and hexane. The aqueous layer is adjusted pH to 4 by addition of a 6N-hydrochloric acid and a saturated aqueous sodium bicarbonate solution, and the mixture is extracted with ethyl acetate and methanol twice. The organic layer is washed with a saturated brine, dried over magnesium sulfate and concentrated under reduced pressure. To the resulting residue is added diethyl ether, and the resulting solids are collected by filtration to give 2-methoxy-5-carboxyphenylboronic acid (1.37g) as a crude product.
(2) The crude 2-methoxy-5-carboxyphenylboronic acid (370mg) is dissolved in N,N-dimethylformamide (10ml), and thereto are added 2.0M-dimethylamine/tetrahydrofuran solution (1.9ml), 1-hydroxybenzotriazole dihydrate (725mg), 1-ethy-3-(3-dimethylaminopropyl)carbodiimide hydrochloride (579mg). The mixture is stirred at room temperature for 3 hours, and thereto is added a saturated aqueous sodium bicarbonate solution, and the mixture is extracted with ethyl acetate twice. The organic layer is dried over magnesium sulfate and concentrated under reduced pressure. The resulting residue is purified by silica gel column chromatography (chloroform : methanol = 19:1) to give 2-methoxy-5-dimethylcarbamoylphenylboronic acid (210mg). MS (m/z): 224 [M+H]⁺

### Reference Example 20

The corresponding starting compound is treated in a similar manner to Reference Example 19 to give the compound.

### Reference Example 21

(1) 4-sec-Butyl-phenol (3.0g) is dissolved in chloroform and thereto is added bromine (1.02ml) and the mixture is stirred at room temperature for 30 minutes. Thereto are added a saturated aqueous sodium thiosulfate solution, a saturated aqueous sodium bicarbonate solution and ethyl acetate, and the mixture is separated. The organic layer is washed with a saturated brine, dried over magnesium sulfate, and conscentrated under reduced pressure to give 2-bromo-4-sec-butyl-phenol (4.57g). NMR (CDCl₃): 0.81 (3H,t), 1.19 (3H,d), 1.56 (2H,m), 2.51 (1H,m), 5.33 (1H,s), 6.93 (1H,d), 7.02 (1H,d), 7.26 (1H,s).
(2) 2-Bromo-4-sec-butyl-phenol (1.50g) is dissolved in N,N-dimethylformamide (10ml) and thereto is added potassium carbonate (1.18g) and iodomethane (1.12g) and the mixture is stirred at room temperature overnight. Thereto are added ethyl acetate and water, and the mixture is separated, and the organic layer is washed with a saturated brine, dried over magnesium sulfate and concentrated under reduced pressure. The resulting residue is purified by silica gel column chromatography (hexane : ethyl acetate = 1:0→20:1) to give 2-bromo-4-sec-butyl-1-methoxy-benzene (1.58g). NMR (CDCl₃): 0.80 (3H,t), 1.20 (3H,d), 1.55 (2H,m), 2.51 (1H,m), 3.87 (3H,s), 6.82 (1H,d), 7.07 (1H,d), 7.35 (1H,s).
(3) 2-Bromo-4-sec-butyl-1-methoxy-benzene (1.15g) is dissolved in tetrahydrofuran (17ml) and the mixture is cooled to -78°C, and thereto is added dropwise 1.6M n-butyllithium in hexanes, and the mixture is stirred at -78°C for 15 minutes. To the reaction solution was added trimethyl borate (1.47g), and the reaction mixture is stirred at -78°C for 30 minutes, and thereto are added a saturated aqueous ammonium chloride solution and ethyl acetate. The mixture is separated, and the organic layer is washed with a saturated brine, dried over magnesium sulfate, and concentrated under reduced pressure to give crude 5-sec-butyl-1-methoxybenzene boronic acid (950mg). NMR (CDCl₃): 0.81 (3H,t), 1.22 (3H,d), 1.58 (2H,m), 2.58 (1H,m), 3.90 (3H,s), 6.25 (2H,s), 6.85 (2H,d), 7.25 (1H,d), 7.65 (1H,s).

### Reference Examples 22 to 25

The corresponding starting compounds are treated in a similar manner to Reference Example 21 to give the compounds.

| Ex. No. | Structural formula |
|---|---|
| 1 | 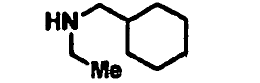 |
| 2 | 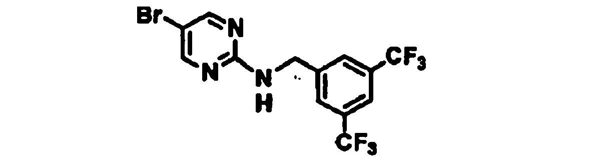 |
| 3 | 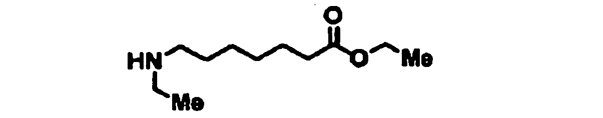 |
| 4 | 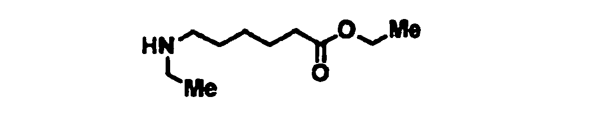 |
| 5 | 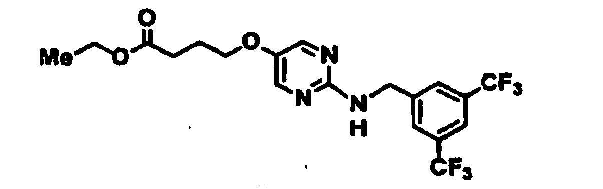 |
| 6 | 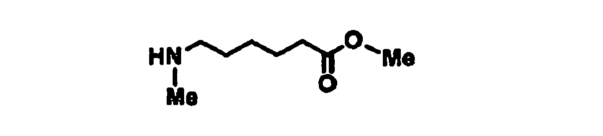 |
| 7 | 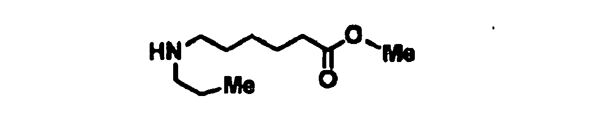 |
| 8 | 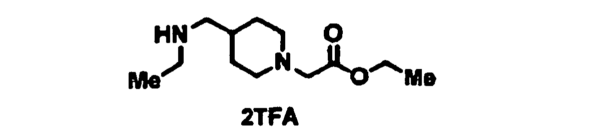 |
| 9 | 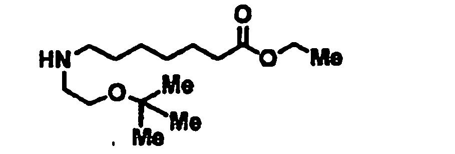 |
| 10 | 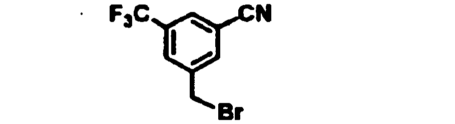 |
| 11 | 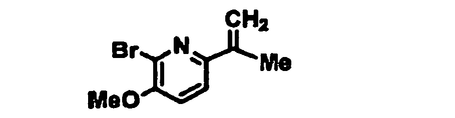 |
| 12 | 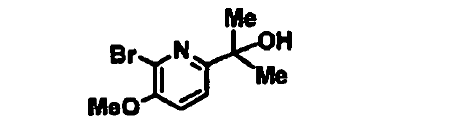 |
| 13 | 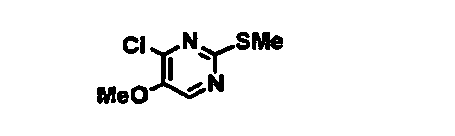 |
| 14 | 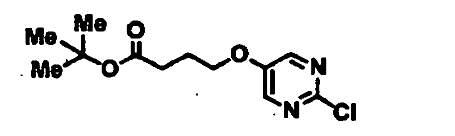 |
| 15 | 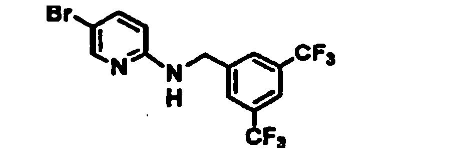 |
| 16 | 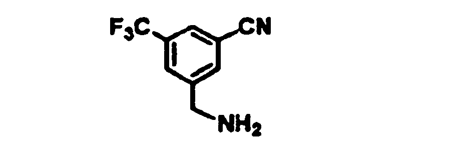 |
| 17 | 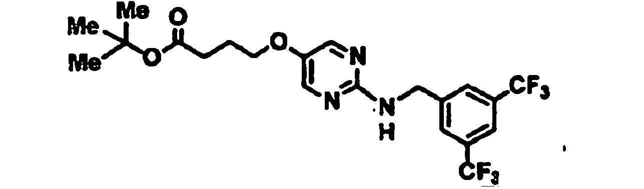 |
| 18 | 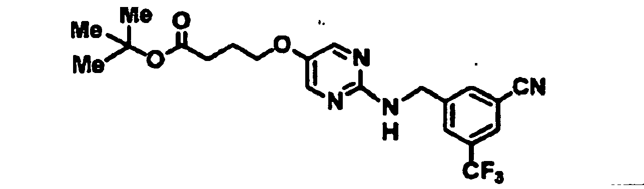 |
| 19 | 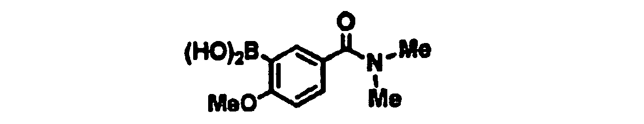 |
| 20 | 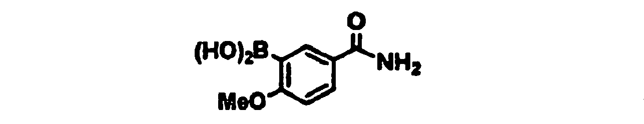 |
| 21 | 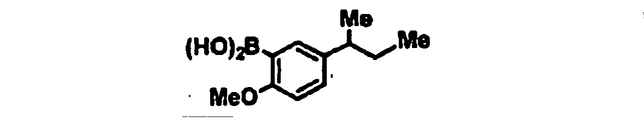 |
| 22 | 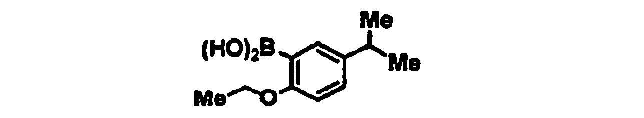 |
| 23 | 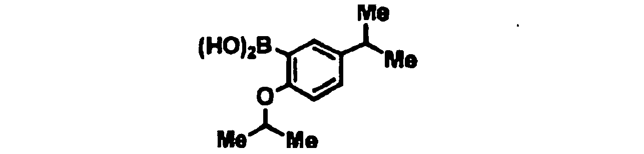 |
| 24 | 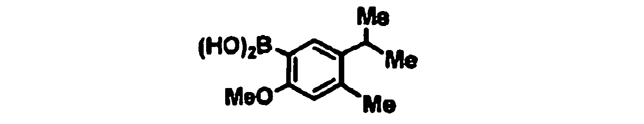 |
| 25 | 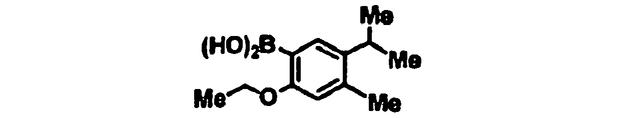 |

### INDUSTRIAL APPLICABILITY

The present compound of formula (1) or a pharmaceutically acceptable derivative thereof has an inhibitory activity against CETP and also shows an activity of increasing HDL cholesterol level and an activity of decreasing LDL cholesterol level. Thus, the compounds of the present invention are useful for prophylaxis and/or treatment of arteriosclerotic diseases, hyperlipemia or dyslipidemia, and the like.

## Claims

1. A compound of the general formula (1): 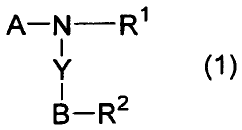 wherein Y is a methylene group;
A is a group of a formula: -A¹-A²,
A¹ is a pyrimidinyl group;
A² is an alkoxy group optionally substituted by carboxyl group, a piperidyl group optionally substituted by carboxyl group, an amino group optionally substituted by 1 to 2 groups selected independently from carboxyalkyl group and akyl group; or an alkyl group substituted by carboxyl group;
B is a pyrimidinyl, pyridyl, quinolyl, dihydroindolyl, pyrazolyl, isoquinolyl, imidazolyl or imidazopyridyl group; wherein said heterocyclic group may optionally be substituted by 1 to 5 substituents selected independently from the following groups: an oxo group; an alkoxy group; an alkyl group optionally substituted by 1 to 3 halogen atoms; a halogen atom; an alkylsulfanyl group; an alkylsulfinyl group; and an amino group optionally substituted by 1 to 2 substituents selected independently from an alkyl group or an alkyl group substituted by morpholinyl;
R¹ is 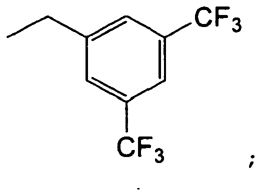 R² is
(a) an amino group optionally substituted by 1 to 2 groups selected independently from a carboxyalkyl group, an alkoxyalkyl group, a hydroxyalkyl group, mono- or dialkylaminoalkyl group, an alkyl group, a cycloalkylalkyl group and an alkoxycarbonyl group;
(b) an alkoxy group;
(c) a phenyl group optionally substituted by 1 to 2 substituents selected independently from an alkyl group and an alkoxy group;
(d) a pyrimidinyl group optionally substituted by 1 to 2 substituents selected from an alkoxy group and an alkyl group;
(e) a pyridyl group optionally substituted by 1 to 2 substituents selected from an alkoxy group and an alkyl group; or a pharmaceutically acceptable salt, solvate or ester thereof.

2. A compound selected from the following group: 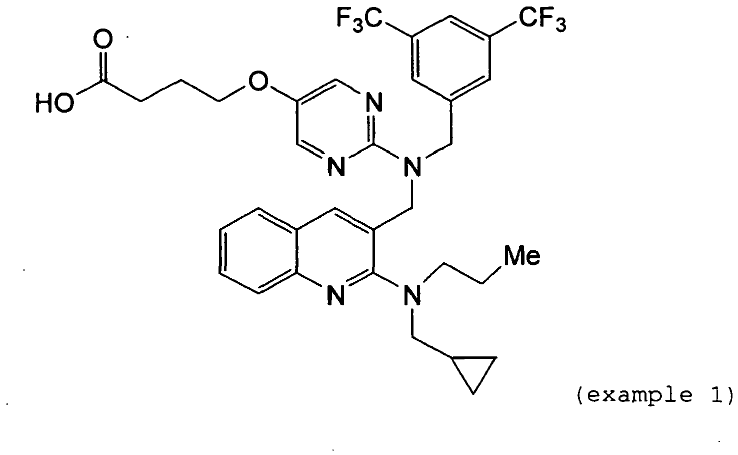 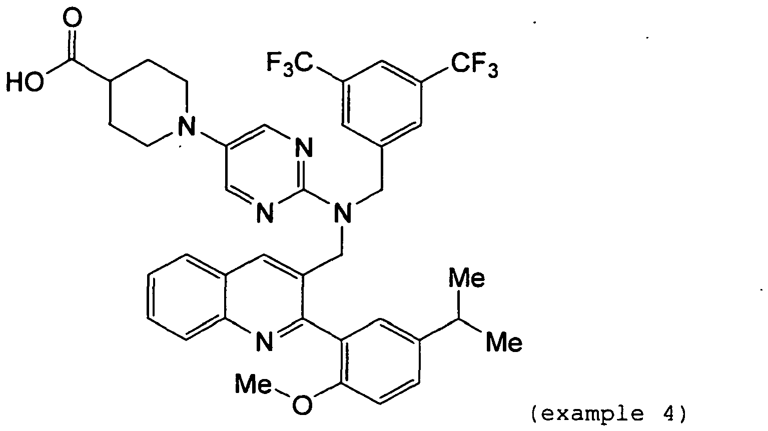 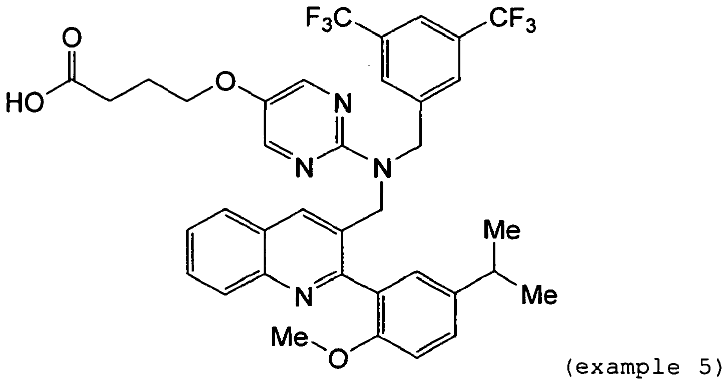 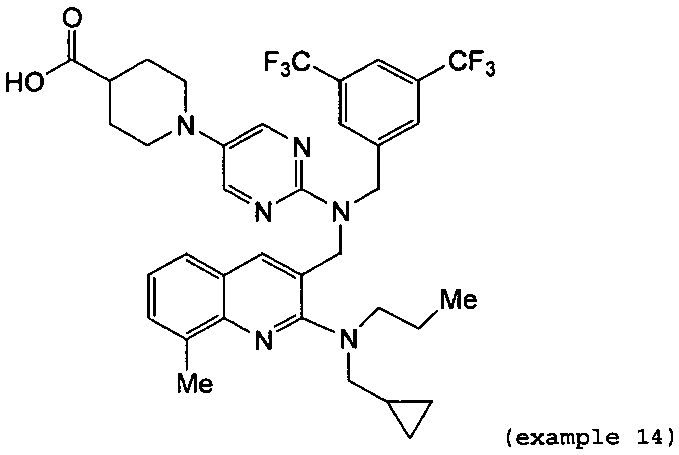 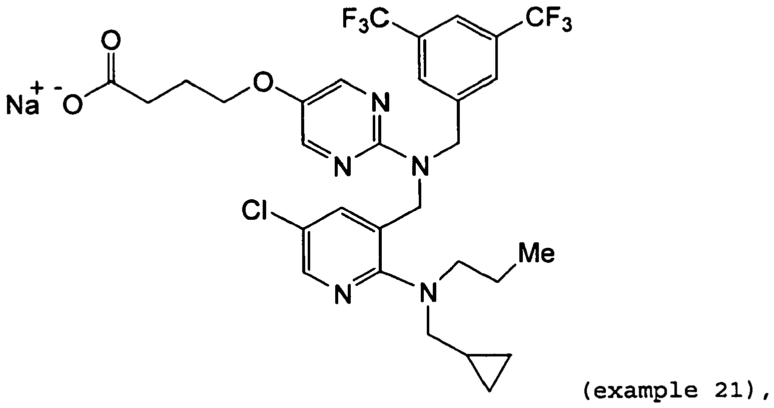 and 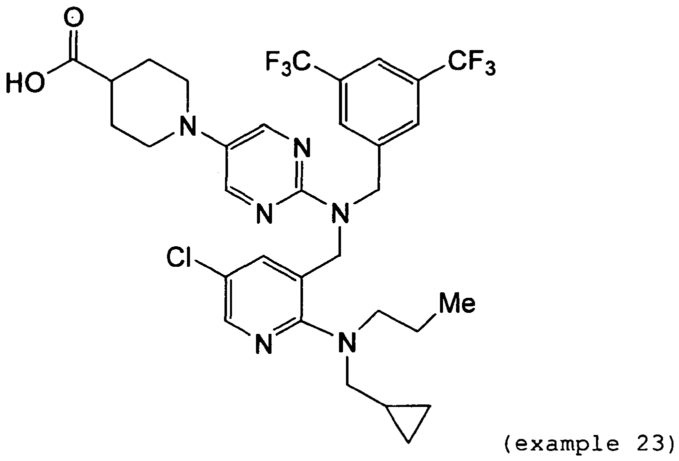 , or a pharmaceutically acceptable salt, solvate or ester thereof.

3. A pharmaceutical composition, which comprises as an active ingredient a compound according claim 1 or 2 or a pharmaceutically acceptable salt, solvate or ester thereof.

4. A compound according to claim 1 or 2, or a pharmaceutically acceptable salt, solvate or ester thereof for use in the prophylaxis or treatment of arteriosclerosis such as atherosclerosis, peripheral vascular disease, dyslipidemia, hyperbetalipoproteinemia, hypoalphalipoproteinemia,
hypercholesterolemia, hypertriglyceridemia, familial-hypercholesterolemia, cardiovascular diseases, angina, ischemia, cardiac ischemia, stroke, myocardial infarction, reperfusion, injury, restenosis after angioplasty, hypertension, cerebral infarction, cerebral stroke, diabetes, vascular complication of diabetes, thrombotic diseases, obesity, endotoxemia, metabolic syndrome, cerebrovascular disease, coronary artery disease, ventricular dysfunction, cardiac arrhythmia, pulmonary vascular disease, reno-vascular disease, renal disease, splanchnic vascular disease, vascular hemostatic disease, fatty liver disease, steatohepatitis, inflammatory disease, autoimmune disorders and other systemic disease indications, immune function modulation, pulmonary disease, anti-oxidant disease, sexual dysfunction, cognitive dysfunction, schistosomiasis, cancer, regression of xanthoma or Alzheimer's disease, which comprises administering an effective amount of a compound according to claim 1 or 2, or a pharmaceutically acceptable salt, solvate or ester thereof, to a patient in need thereof.

5. Use of a compound according to claim 1 or 2, or a pharmaceutically acceptable salt, solvate or ester thereof, for the manufacture of a medicament in treatment of patients suffering from arteriosclerosis such as atherosclerosis, peripheral vascular disease, dyslipidemia, hyperbetalipoproteinemia, hypoalphalipoproteinemia, hypercholesterolemia, hypertrigylceridemia, familial-hypercholesterolemia, cardiovascular diseases, angina, ischemia, cardiac ischemia, stroke, myocardial infarction, reperfusion injury, restenosis after angioplasty, hypertension, cerebral infarction, cerebral stroke, diabetes, vascular complication of diabetes, thrombotic diseases, obesity, endotoxemia, metabolic syndrome, cerebrovascular disease coronary artery disease, ventricular dysfunction, cardiac arrhythmia, pulmonary vascular disease, reno-vascular disease, renal disease, splanchnic vascular disease, vascular hemostatic disease, fatty liver disease, steatohepatitis, inflammatory disease, autoimmune disorders and other systemic disease indications, immune function modulation, pulmonary disease, anti-oxidant disease, sexual dysfunction, cognitive dysfunction, schistosomiasis, cancer, regression of xanthoma or Alzheimer's disease.

## Patentansprüche

1. Verbindung der allgemeinen Formel (1): 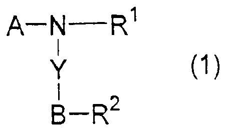 worin gilt:
Y ist eine Methylengruppe;
A ist eine Gruppe der Formel: -A¹-A²,
A¹ ist eine Pyrimidinylgruppe;
A² ist eine Alkoxygruppe, optional substituiert mit einer Carboxylgruppe, eine Pyridylgruppe, optional substituiert durch eine Carboxylgruppe, eine Aminogruppe, optional substituiert durch 1 bis 2 Gruppen, die unabhängig voneinander ausgewählt sind aus einer Carboxyalkylgruppe und einer Alkylgruppe; oder eine Alkylgruppe, die durch eine Carboxylgruppe substituiert ist;
B ist eine Pyrimidinyl-, Pyridyl-, Chinolyl-, Dihydroindolyl-, Pyrazolyl-, Isochinolyl-, Imidazolyl-oder Imidazopyridylgruppe;
worin die heterocyclische Gruppe optional substituiert sein kann mit 1 bis 5 Substituenten, die unabhängig voneinander ausgewählt sind aus den folgenden Gruppen:
eine Oxogruppe; eine Alkoxygruppe; eine Alkylgruppe, die optional mit 1 bis 3 Halogenatomen substituiert ist; ein Halogenatom; eine Alkylsulfanylgruppe; eine Alkylsulfinylgruppe; und eine Aminogruppe, die optional substituiert ist mit 1 bis 2 Substituenten, die unabhängig voneinander ausgewählt sind aus einer Alkylgruppe oder einer Alkylgruppe, die mit Morpholinyl substituiert ist;
R¹ ist 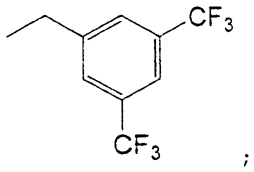 R² ist
(a) eine Aminogruppe, die optional substituiert ist mit 1 bis 2 Gruppen, die unabhängig voneinander ausgewählt sind aus einer Carboxyalkylgruppe, einer Alkoxyalkylgruppe, einer Hydroxyalkylgruppe, Mono-oder Di-Alkylaminoalkylgruppe, einer Alkylgrupe, einer Cycloalkylalkylgruppe und einer Alkoxycarbonylgruppe;
(b) eine Alkoxygruppe;
(c) eine Phenylgruppe, die optional substituiert ist mit 1 bis 2 Substituenten, die unabhängig voneinander ausgewählt sind aus einer Alkylgruppe und einer Alkoxygruppe;
(d) eine Pyrimidinylgruppe, die optional substituiert ist mit 1 bis 2 Substituenten, die ausgewählt sind aus einer Alkoxygruppe und einer Alkylgruppe;
(e) eine Pyridylgruppe, die optional substituiert ist mit 1 bis 2 Substituenten, die aus einer Alkoxygruppe und einer Alkylgruppe ausgewählt sind; oder ein pharmazeutisch annehmbare(s/r) Salz, Solvat oder Ester davon.

2. Verbindung, die aus der folgenden Gruppe ausgewählt ist: 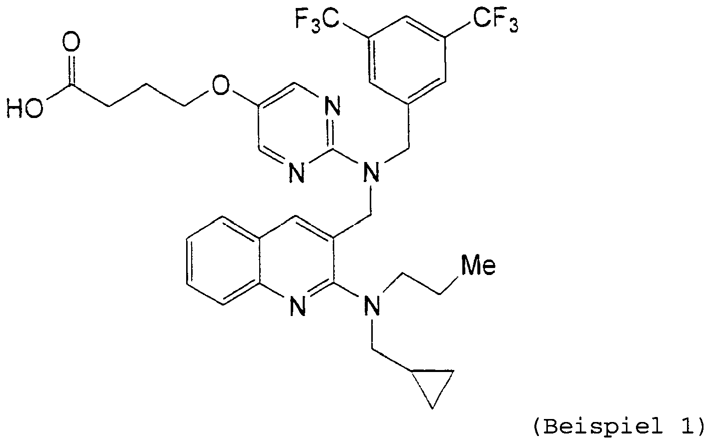 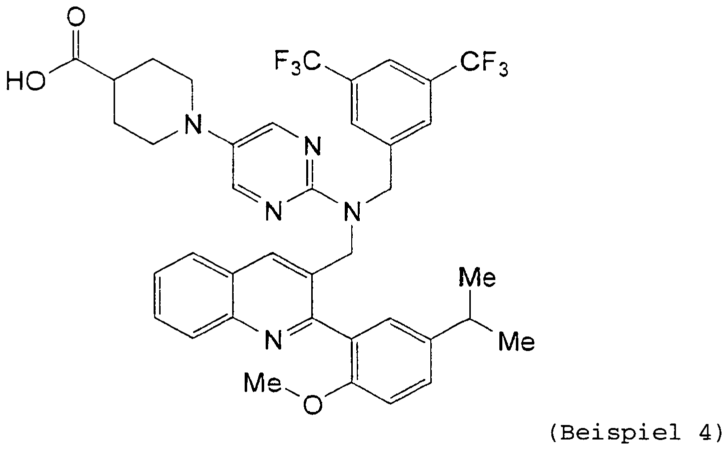 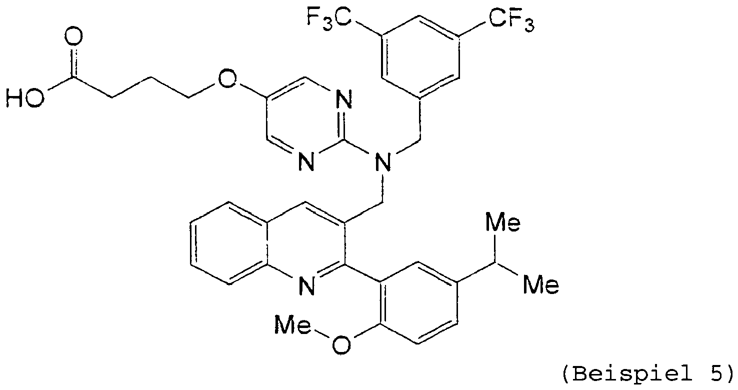 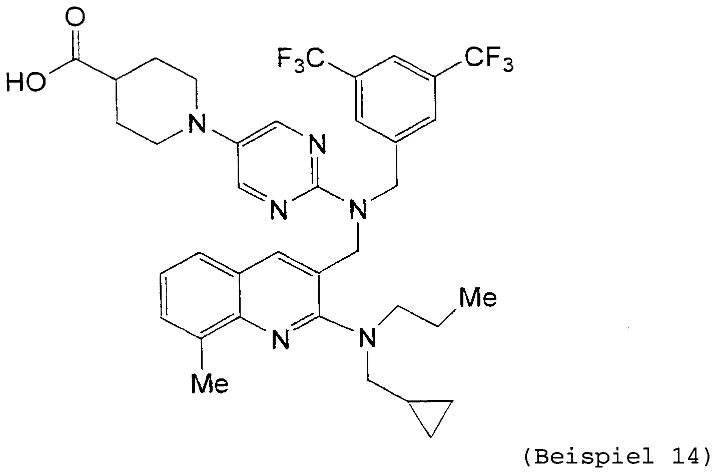 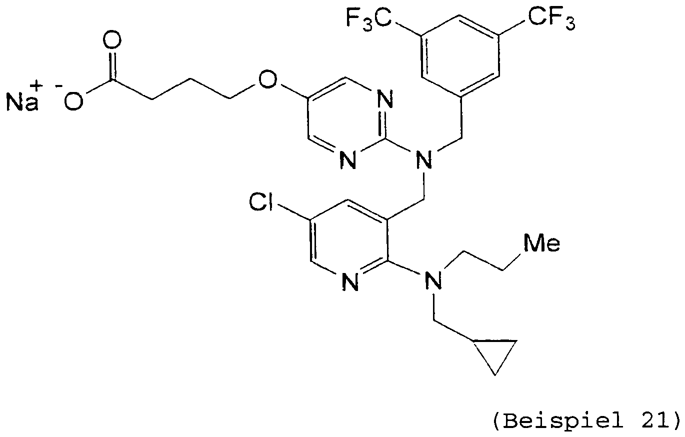 und 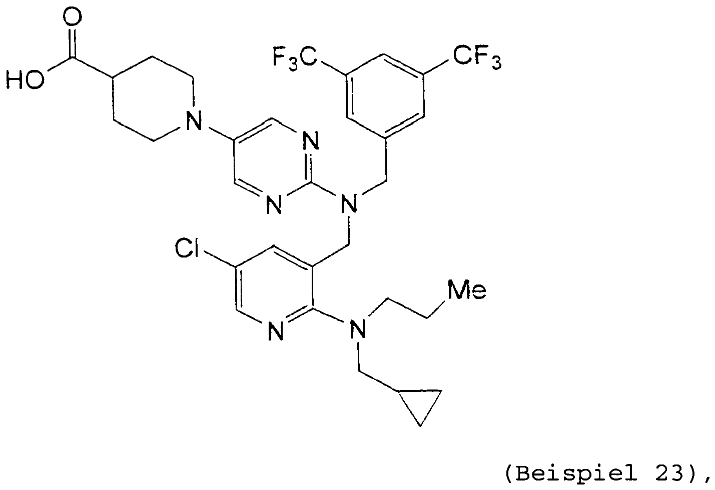 oder ein pharmazeutisch annehmbare(s/r) Salz, Solvat oder Ester davon.

3. Pharmazeutische Zusammensetzung, welche als Wirkstoff eine Verbindung gemäß Anspruch 1 oder 2 oder ein(en) pharmazeutisch annehmbare(s/n) Salz, Solvat oder Ester davon umfasst.

4. Verbindung gemäß Anspruch 1 oder 2, oder ein pharmazeutisch annehmbare(s/r) Salz, Solvat oder Ester davon für die Verwendung in der Prophylaxe oder Behandlung von Arteriosklerose, wie z.B. Atherosklerose, periphere Gefäßerkrankung, Dyslipidämie, Hyperbetalipoproteinämie, Hypoalphalipoproteinämie, Hypercholesterolämie, Hypertriglyceridämie, familiäre Hypercholesterolämie, kardiovaskuläre Erkrankungen, Angina, Ischämie, kardiale Ischämie, Schlaganfall, Myokardinfarkt, Reperfusion, Verletzung, Restenose nach Angioplastie, Bluthochdruck, Hirninfarkt, Gehirnschlag Diabetes, vaskuläre Komplikation der Diabetes, thrombotische Erkrankungen, Adipositas, Endotoxämie, metabolisches Syndrom, zerebrovaskuläre Erkrankung, Erkrankung der Koronararterie, ventrikuläre Dysfunktion, Herzrhythmusstörung, Lungengefäßerkrankung, Nierengefäßerkrankung, Nierenerkrankung, Splanchnikus-Gefäßerkrankung, vaskuläre haemostatische Erkrankung, Fettleberkrankheit, Fettleberentzündung, Entzündungskrankheit, Autoimmunerkrankungen und andere Systemerkrankungen, Immunfunktionsmodulation, Lungenerkrankung, Antioxidantienerkrankung, sexuelle Dysfunktion, kognitive Dysfunktion, Schistosomiasis, Krebs, Regression von Xanthoma oder Alzheimer-Krankheit, welche die Verabreichung einer wirksamen Menge einer Verbindung gemäß Anspruch 1 oder 2 oder eines pharmazeutisch annehmbaren Salzes, Solvats oder Esters davon an einen bedürftigen Patienten umfaßt.

5. Verwendung einer Verbindung gemäß Anspruch 1 oder 2 oder eines pharmazeutisch annehmbaren Salzes, Solvats oder Esters davon für die Herstellung eines Medikaments für die Behandlung von Patienten, die an Atherosklerose, periphere Gefäßerkrankung, Dyslipidämie, Hyperbetalipoproteinämie, Hypoalphalipoproteinämie, Hypercholesterolämie, Hypertriglyceridämie, familiäre Hypercholesterolämie, kardiovaskuläre Erkrankungen, Angina, Ischämie, kardiale Ischämie, Schlaganfall, Myokardinfarkt, Reperfusion, Verletzung, Restenose nach Angioplastie, Bluthochdruck, Hirninfarkt, Gehirnschlag, Diabetes, vaskuläre Komplikationen der Diabetes, thrombotische Erkrankungen, Adipositas, Endotoxämie, metabolisches Syndrom, zerebrovaskuläre Erkrankung, Erkrankung der Koronararterie, ventrikuläre Dysfunktion, Herzrhythmusstörung, Lungengefäßerkrankung, Nierengefäßerkrankung, Nierenerkrankung, Splanchnikus-Gefäßerkrankung, vaskuläre haemostatische Erkrankung, Fettleberkrankheit, Fettleberentzündung, Entzündungskrankheit, Autoimmunerkrankungen und andere Systemerkrankungen, Immunfunktionsmodulation, Lungenerkrankung, Antioxidantienerkrankung, sexuelle Dysfunktion, kognitive Dysfunktion, Schistosomiasis, Krebs, Regression von Xanthoma oder Alzheimer-Krankheit leiden.

## Revendications

1. Composé de la formule générale (1) : 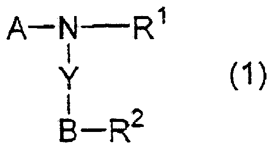 dans laquelle Y est un groupe méthylène ;
A est un groupe d'une formule : -A¹-A²,
A¹ est un groupe pyrimidinyle ;
A² est un groupe alcoxy éventuellement substitué par un groupe carboxyle, un groupe pipéridyle éventuellement substitué par un groupe carboxyle, un groupe amino éventuellement substitué par 1 ou 2 groupes choisis indépendamment parmi un groupe carboxyalkyle et un groupe alkyle ; ou un groupe alkyle substitué par un groupe carboxyle ;
B est un groupe pyrimidinyle, pyridyle, quinolyle, dihydroindolyle, pyrazolyle, isoquinolyle, imidazolyle ou imidazopyridyle ; dans lequel ledit groupe hétérocyclique peut éventuellement être substitué par de 1 à 5 substituants choisis indépendamment parmi les groupes suivants : un groupe oxo ; un groupe alcoxy ; un groupe alkyle éventuellement substitué par de 1 à 3 atomes d'halogène ; un atome d'halogène ; un groupe alkylsulfanyle ; un groupe alkylsulfinyle ; et un groupe amino éventuellement substitué par de 1 à 2 substituants choisis indépendamment parmi un groupe alkyle ou un groupe alkyle substitué par un groupe morpholinyle ;
R¹ est 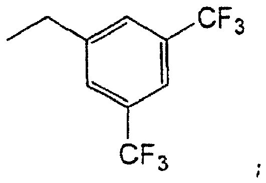 R² est
(a) un groupe amino éventuellement substitué par de 1 à 2 groupes choisis indépendamment parmi un groupe carboxyalkyle, un groupe alcoxyalkyle, un groupe hydroxyalkyle, un groupe mono- ou di-alkylaminoalkyle, un groupe alkyle, un groupe cycloalkylalkyle et un groupe alcoxycarbonyle ;
(b) un groupe alcoxy ;
(c) un groupe phényle éventuellement substitué par de 1 à 2 substituants choisis indépendamment parmi un groupe alkyle et un groupe alcoxy ;
(d) un groupe pyrimidinyle éventuellement substitué par de 1 à 2 substituants choisis parmi un groupe alcoxy et un groupe alkyle ;
(e) un groupe pyridyle éventuellement substitué par de 1 à 2 substituants choisis parmi un groupe alkoxy et un groupe alkyle ; ou sel, solvate ou ester pharmaceutiquement acceptable de celui-ci.

2. Composé choisi dans le groupe suivant : 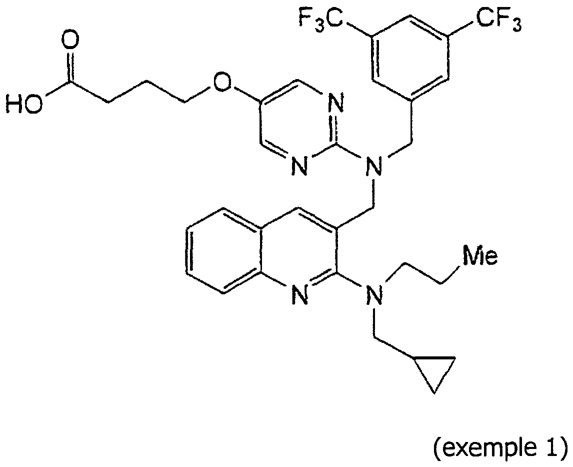 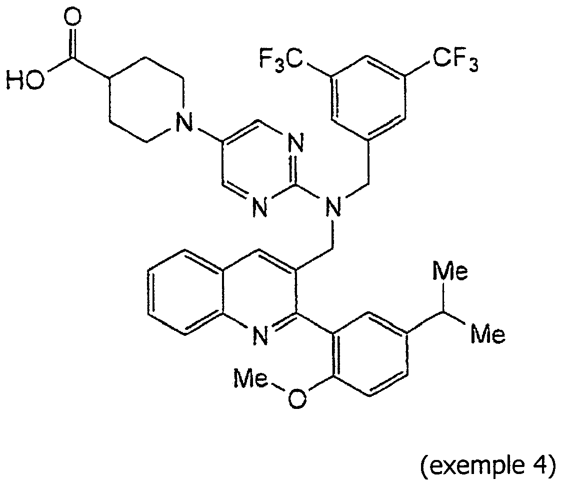 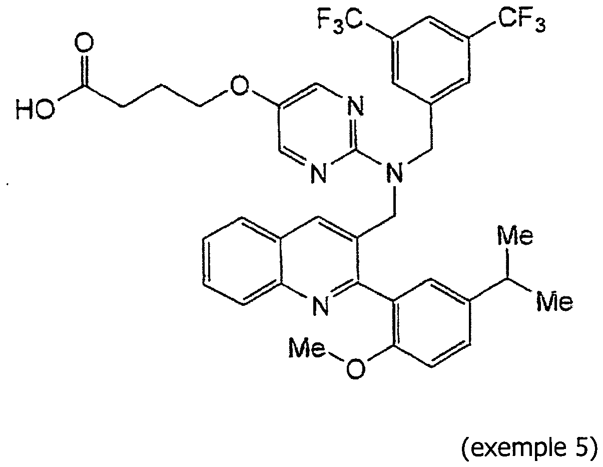 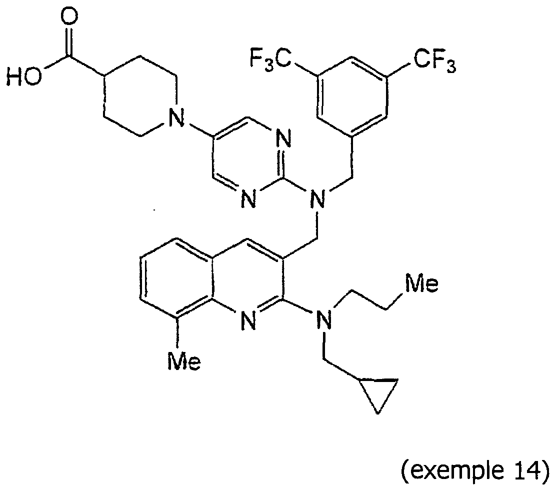 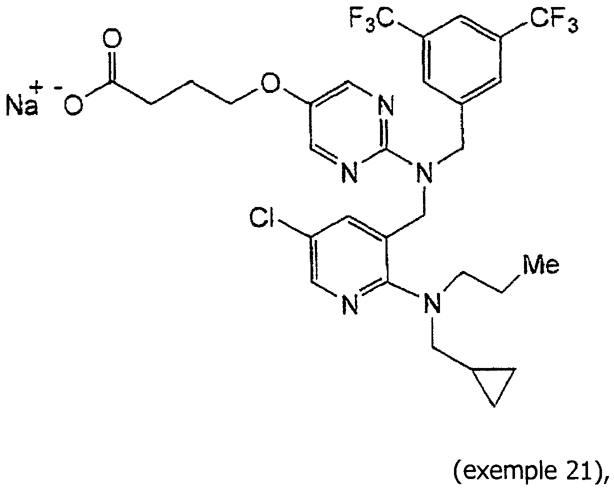 et 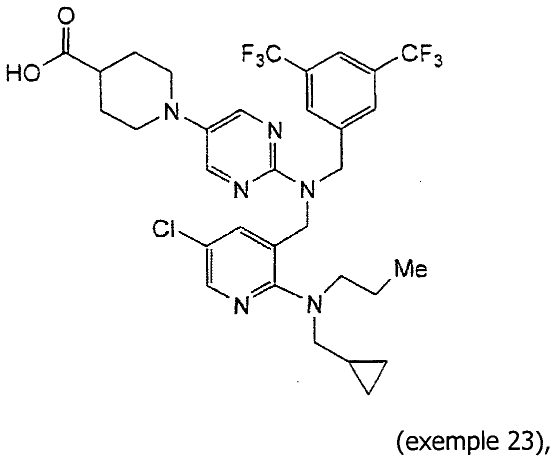 ou sel, solvate ou ester pharmaceutiquement acceptable de celui-ci.

3. Composition pharmaceutique, laquelle comprend comme ingrédient actif un composé selon la revendication 1 ou 2 ou un sel, solvate ou ester pharmaceutiquement acceptable de celui-ci.

4. Composé selon la revendication 1 ou 2, ou sel, solvate ou ester pharmaceutiquement acceptable de celui-ci pour une utilisation dans la prophylaxie ou le traitement de l'artériosclérose, tel que l'athérosclérose, d'une maladie vasculaire périphérique, de la dyslipidémie, de l'hyperbétalipoprotéinémie, de l'hypoalphalipoprotéinémie, de l'hyperchlolestérolémie, de l'hypertriglycéridémie, de l'hypercholestérolémie familiale, de maladies cardiovasculaires, de l'angine, de l'ischémie, de l'ischémie cardiaque, de l'attaque, de l'infarctus du myocarde, de la reperfusion, d'une lésion, d'une resténose après une angioplastie, de l'hypertension, de l'infarctus cérébral, de l'attaque cérébrale, de diabètes, d'une complication vasculaire des diabètes, de maladies thrombotiques, de l'obésité, de l'endotoxémie, d'un syndrome métabolique, d'une maladie cérébrovasculaire, d'une maladie des artères coronaires, d'un dysfonctionnement ventriculaire, d'une arythmie cardiaque, d'une maladie vasculaire pulmonaire, d'une maladie réno-vasculaire, d'une maladie rénale, d'une maladie vasculaire splanchnique, d'une maladie hémostatique vasculaire, d'une maladie du foie gras, de la stéatohépatite, d'une maladie inflammatoire, de troubles autoimmuns et d'autres indications de maladies systémiques, d'une modulation de la fonction immune, d'une maladie pulmonaire, d'une maladie anti-oxydante, d'un dysfonctionnement sexuel, d'un dysfonctionnement cognitif, de la schistosomiase, du cancer, de la régression de xanthome ou de la maladie d'Alzheimer, qui comprend l'administration d'une quantité efficace d'un composé selon la revendication 1 ou 2, ou d'un sel, solvate ou ester pharmaceutiquement acceptable de celui-ci, à un patient en besoin de celui-ci.

5. Utilisation d'un composé selon la revendication 1 ou 2, ou d'un sel, solvate ou ester pharmaceutiquement acceptable de celui-ci, pour la fabrication d'un médicament dans le traitement de patients souffrant de l'artériosclérose, tel que l'athérosclérose, d'une maladie vasculaire périphérique, de la dyslipidémie, de l'hyperbétalipoprotéinémie, de l'hypoalphalipoprotéinémie, de l'hyperchlolestérolémie, de l'hypertriglycéridémie, de l'hypercholestérolémie familiale, de maladies cardiovasculaires, de l'angine, de l'ischémie, de l'ischémie cardiaque, de l'attaque, de l'infarctus du myocarde, de la reperfusion, d'une lésion, d'une resténose après une angioplastie, de l'hypertension, de l'infarctus cérébral, de l'attaque cérébrale, de diabètes, d'une complication vasculaire des diabètes, de maladies thrombotiques, de l'obésité, de l'endotoxémie, d'un syndrome métabolique, d'une maladie cérébrovasculaire, d'une maladie des artères coronaires, d'un dysfonctionnement ventriculaire, d'une arythmie cardiaque, d'une maladie vasculaire pulmonaire, d'une maladie réno-vasculaire, d'une maladie rénale, d'une maladie vasculaire splanchnique, d'une maladie hémostatique vasculaire, d'une maladie du foie gras, de la stéatohépatite, d'une maladie inflammatoire, de troubles autoimmuns et d'autres indications de maladies systémiques, d'une modulation de la fonction immune, d'une maladie pulmonaire, d'une maladie anti-oxydante, d'un dysfonctionnement sexuel, d'un dysfonctionnement cognitif, de la schistosomiase, du cancer, de la régression de xanthome ou de la maladie d'Alzheimer.
